# EUROPEAN PATENT APPLICATION

(11) **EP 4 589 383 A2**
(43) Date of publication of application: **23.07.2025**
(21) Application number: 24220463.4
(22) Date of filing: 17.12.2024
(51) Int. Cl.: G03F 7/09, G03F 7/40

(54) **COMPOUND FOR FORMING METAL-CONTAINING FILM, COMPOSITION FOR FORMING METAL-CONTAINING FILM, AND PATTERNING PROCESS**

(30) Priority: 17.01.2024 JP 2024005610
(71) Applicant: SHIN-ETSU CHEMICAL CO., LTD., Tokyo 1000005 (JP)
(72) Inventor: Nagamachi, Nobuhiro, Niigata (JP); Kobayashi, Naoki, Niigata (JP); Kori, Daisuke, Niigata (JP); Ishiwata, Kenta, Niigata (JP); Iwamori, Shohei, Niigata (JP)
(74) Representative: Sonnenhauser, Thomas Martin

(57) **Abstract**

The present invention is a compound for forming a metal-containing film to be contained in a composition for forming a metal-containing film, including: at least one metal atom selected from the group consisting of Ti, Zr, and Hf; and a ligand coordinated to the metal atom, wherein the ligand contains a ligand represented by the following general formula (1). The present invention can provide: a compound for forming a metal-containing film having better dry etching resistance than conventional resist underlayer film materials and also having high filling and planarizing properties at the same time; a composition for forming a metal-containing film using the compound; and a patterning process using the composition.

HO-X-R^{A1} (1)

## Description

### TECHNICAL FIELD

The present invention relates to a compound for forming a metal-containing film, a composition for forming a metal-containing film, and a patterning process using the composition.

### BACKGROUND ART

Along with high integration and high processing speed of LSI, miniaturization of pattern size is rapidly advancing. Along with the miniaturization, lithography technology has achieved a fine patterning by shortening wavelength of a light source and selecting an appropriate resist composition accordingly. The main of those compositions is a positive photoresist composition used for monolayer. The monolayer positive photoresist composition not only allows a resist resin to have a skeleton having etching resistance with respect to dry etching with chlorine- or fluorine-based gas plasma, but also provides a switching mechanism that makes an exposed part soluble, thereby dissolving the exposed part to form a pattern and processing a substrate to be processed by dry etching while using the remaining resist pattern as an etching mask.

However, when the pattern becomes finer without changing the thickness of the photoresist film to be used, that is, when the pattern width is reduced, resolution performance of the photoresist film is lowered. In addition, in trying to subject the photoresist film to pattern development with a developer, a developer excessively increased a so-called aspect ratio of the pattern, causing a problem of pattern collapse. Therefore, the photoresist film has been thinned along with the miniaturization of the pattern.

On the other hand, a substrate to be processed has been generally processed by dry etching while using a pattern-formed photoresist film as an etching mask. In practice, however, there is no dry etching method capable of providing a complete etching selectivity between the photoresist film and the substrate to be processed. The photoresist film is thus also damaged and collapses during processing of the substrate, resulting in a problem of becoming incapable of accurate transferring the resist pattern to the substrate to be processed. Accordingly, higher dry etching resistance has been required in a resist composition along with the miniaturization of the pattern. However, on the other hand, in order to improve the resolution, a resin used for the photoresist composition has been required to have low light absorption at exposure wavelength. For this reason, the resin has shifted to a novolak resin, polyhydroxystyrene, and a resin having an aliphatic polycyclic skeleton as the exposure light became shorter in a wavelength from i-line to KrF and Ar. However, an actual situation has increased an etching rate under dry etching conditions in processing the substrate, and recent photoresist compositions having high resolution rather tend to have low etching resistance.

As a result, the substrate to be processed has to be dry etched with a thinner photoresist film having lower etching resistance. Therefore, securing materials and processes for such processing has become an urgent matter.

A multilayer resist method is one of the solutions for the above problems. This method is as follows: a resist underlayer film having a different etching selectivity from a photoresist film (i.e., a resist upper layer film) is placed between the resist upper layer film and a substrate to be processed; a pattern is formed in the resist upper layer film; the pattern is transferred to the resist underlayer film by dry etching while using the resist upper layer film pattern as a dry etching mask; and the pattern is further transferred to the substrate to be processed by dry etching while using the resist underlayer film as a dry etching mask.

One of the multilayer resist methods is a three-layer resist method, which can be performed with a typical resist composition used in the monolayer resist method. For example, this three-layer resist method includes the following steps: an organic film containing a novolak resin or the like is formed as a resist underlayer film on a substrate to be processed; a silicon-containing resist middle layer film is formed thereon as a resist middle layer film; and a usual organic photoresist film is formed thereon as a resist upper layer film. Since the organic resist upper layer film ensures a better etching selectivity ratio than the silicon-containing resist middle layer film when dry etching is performed with fluorine-based gas plasma, the resist upper layer film pattern can be transferred to the silicon-containing resist middle layer film by dry etching with fluorine-based gas plasma. This method allows the pattern to be transferred to the silicon-containing resist middle layer film (resist middle layer film) even by using a resist composition with which it is difficult to form a pattern having a sufficient film thickness for directly processing the substrate to be processed or a resist composition that has insufficient dry etching resistance for processing the substrate. Then, further performing dry etching with oxygen-based gas plasma or hydrogen-based gas plasma allows the pattern to be transferred to the organic film (resist underlayer film) containing a novolak resin or the like, which has a sufficient dry etching resistance for processing the substrate. As to the resist underlayer film, many materials are already publicly known as disclosed in Patent Document 1, for example.

On the other hand, in recent years, the miniaturization of DRAM has accelerated, and there has been an increasing need for a resist underlayer film having further improved dry etching resistance and excellent filling and planarizing properties. As a coating-type resist underlayer film material with the excellent filling and planarizing properties, for example, materials such as disclosed in Patent Document 2 have been reported, but when considering application in advanced generation, they have concerns about dry etching resistance and the application limits of conventional coating-type resist underlayer film materials are approaching.

In order to solve the above problems, development using a material containing a metal element in the resist underlayer film is being studied. Patent Document 3 discloses that a material containing a Ti compound exhibits excellent dry etching resistance with respect to CHF₃/CF₄-based gas and CO₂/N₂-based gas.

On the other hand, problems include a filling property when using a metal compound for a resist underlayer film. Although Patent Document 3 does not refer to filling property, metal compounds generally have large thermal shrinkage during baking and cause significant deterioration of filling properties between patterns after high-temperature baking. Therefore, there is a concern that it may be insufficient, when used as a resist underlayer film material that requires high planarizing and filling properties and heat resistance property. Patent Documents 4 and 5 disclose that a metal compound modified with specific a ligand has an excellent filling property, but the baking temperature in the filling property evaluation conducted is as low as 150°C. Therefore, there is a concern that the resist underlayer film material may be insufficient when used as a resist underlayer film material that requires heat resistance (for example, properties against heat treatment that may be performed after forming the resist underlayer film).

### CITATION LIST

### PATENT LITERATURE

Patent Document 1: JP2004-205685A
Patent Document 2: JP6714493B2
Patent Document 3: JP6189758B2
Patent Document 4: JP6786391B2
Patent Document 5: JP7050137B2

### SUMMARY OF INVENTION

### TECHNICAL PROBLEM

The present invention has been made in view of the above-described problem. An object of the present invention is to provide: a compound for forming a metal-containing film having better dry etching resistance than conventional resist underlayer film materials and also having high filling and planarizing properties at the same time; a composition for forming a metal-containing film using the compound; and a patterning process using the composition.

### SOLUTION TO PROBLEM

To solve the problems above, the present invention provides a compound for forming a metal-containing film to be contained in a composition for forming a metal-containing film, comprising: at least one metal atom selected from the group consisting of Ti, Zr, and Hf; and a ligand coordinated to the metal atom, wherein the ligand contains a ligand represented by the following general formula (1),

HO-X-R^{A1} (1)

wherein R^{A1} represents a monovalent organic group having 2 to 30 carbon atoms; X represents a single bond or a linear or branched divalent organic group having 2 to 10 carbon atoms; and either R^{A1} and X contains one or more structures represented by the following general formulae (a-1) to (a-3), 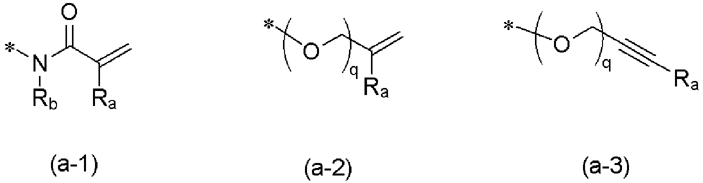
wherein Rₐ represents a hydrogen atom or a monovalent organic group having 1 to 10 carbon atoms; R_{b} represents a hydrogen atom or a monovalent organic group having 1 to 10 carbon atoms; "q" represents 0 or 1; and "*" represents an attachment point.

Such a compound for forming a metal-containing film contains at least one of the crosslinking groups represented by the above general formulae (a-1) to (a-3). When this compound is used for a composition for forming a metal-containing film, so it is possible to provide a composition for forming a metal-containing film that has low volume shrinkage at the time of baking, excellent planarizing and filling properties, and sufficient dry etching resistance even after high-temperature baking.

Further, in the present invention, R^{A1} in the general formula (1) preferably includes any one or more of aromatic rings, alicyclic hydrocarbons, and heterocycles.

In the case of such a compound for forming a metal-containing film, the compound contains a ligand having an excellent heat resistance derived from a organic compound of the above formula (1) and one or more of the crosslinking groups represented by the above general formulae (a-1) to (a-3). Thus, when the compound is used for a composition for forming a metal-containing film, it is possible to provide a composition for forming a metal-containing film that has low volume shrinkage at the time of baking, excellent planarizing and filling properties even after high-temperature baking.

Further, in the present invention, the ligand preferably contains a ligand represented by the following general formula (2), 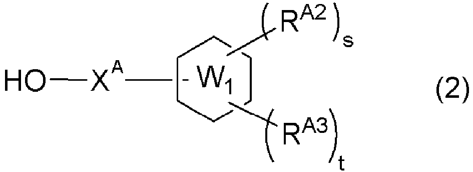 wherein W₁ is a divalent or trivalent organic group having 3 to 20 carbon atoms and including any one or more of aromatic rings, alicyclic hydrocarbons, and heterocycles; R^{A2} represents a hydrogen atom, a halogen atom, or a monovalent organic group having 1 to 10 carbon atoms; R^{A3} represents a monovalent organic group having 1 to 10 carbon atoms; X^{A} represents a single bond or a linear or branched divalent organic group having 1 to 10 carbon atoms; either of R^{A2} and X^{A} has one or more structures represented by the above general formulae (a-1) to (a-3); and "s" represents an integer of 1 to 6, "t" represents an integer from 0 to 5, and t+s is an integer from 1 to 6.

When the ligand is a ligand derived from a compound represented by the above general formula (2), the compound for forming a metal-containing film can achieve both high thermal flowability and high thermosetting property. Thus, when the compound is contained in a composition for forming a metal-containing film, it is possible to provide a composition for forming a metal-containing film that exhibits more excellent planarizing and filling properties.

Further, in the present invention, W₁ in the general formula (2) represents any one of the following structures.

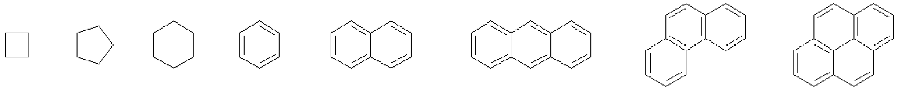

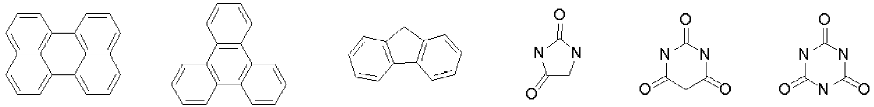

In the above general formula (2), when W₁ has any of the above structures, it becomes possible to improve the heat resistance of the compound for forming a metal-containing film. Thus, when the compound is contained in a composition for forming a metal-containing film, it is possible to provide a composition for forming a metal-containing film exhibiting more excellent planarizing and filling properties.

Further, in the present invention, the compound for forming a metal-containing film is preferably a reaction product of a reaction between: a metal compound represented by the following general formula (3) or a metal-containing compound containing a hydrolysate, condensate, or hydrolysis condensate of the metal compound represented by the following general formula (3); and a compound represented by the above general formula (1) or (2),

LₐMX^{B}_{b} (3)

wherein, M represents any of Ti, Zr, and Hf; L represents a monodentate ligand having 0 to 30 carbon atoms or a polydentate ligand having 0 to 30 carbon atoms; X^{B} represents a hydrolyzable group selected from the group consisting of a halogen atom, an alkoxy group, a carboxylate group, an acyloxy group, and -NR^{a'}R^{b'}; R^{a'} and R^{b'} each independently represent a hydrogen atom or a monovalent organic group having 1 to 20 carbon atoms; and "a" and "b" represent integers from 0 to 4, satisfying a+b=4.

In the case of such a compound for forming a metal-containing film, the compound for forming a metal-containing film is derived from a metal compound represented by the above general formula (3) or a metal-containing compound obtained through hydrolysis, condensation, or hydrolysis-condensation of a metal compound represented by the above general formula (3). Accordingly, when the compound is contained in composition for forming a metal-containing film a metal-containing film, it becomes possible to form a dense metal-containing film, and it is possible to provide a metal-containing film with extremely excellent dry etching resistance to the substrate to be processed.

Further, in the present invention, the general formula (3) preferably has a structure represented by the following general formula (4),

M(OR^{1A})₄ (4)

wherein M is any of Ti, Zr, and Hf and R^{1A} represents a monovalent organic group having 1 to 20 carbon atoms.

A metal compound having such a structure is preferable from the viewpoint of productivity and material availability.

Further, in the present invention, the compound for forming a metal-containing film preferably comprises further a ligand represented by the following general formula (5), 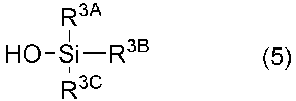
wherein R^{3A}, R^{3B} and R^{3C} represent any organic group selected from the group consisting of: an organic group having 1 to 30 carbon atoms including a crosslinking group of any structure represented by the following general formulae (b-1) to (b-3); a substituted or unsubstituted alkyl group having 1 to 20 carbon atoms; and an aryl group having 6 to 20 carbon atoms, 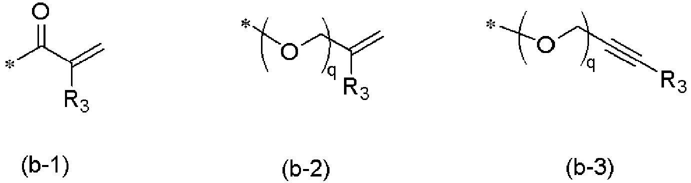
wherein R₃ represents a hydrogen atom or a monovalent organic group having 1 to 10 carbon atoms; "q" represents 0 or 1; and "*" represents an attachment point.

By including such a ligand, the stability of the compound for forming a metal-containing film can be further improved.

Further, the present invention provides a composition for forming a metal-containing film used for manufacturing a semiconductor, comprising: (A) the compound for forming a metal-containing film according to the above; and (B) an organic solvent.

Such a composition for forming a metal-containing film contains a metal-containing compound having excellent heat resistance and thermal flowability, so it is possible to provide a composition for forming a metal-containing film that has better dry etching resistance than conventional resist underlayer film and more advanced filling and planarizing properties than conventional metal-containing film materials.

Further, in the present invention, the composition preferably comprises further one or more of (E) a crosslinking agent, (G) a surfactant, and (H) an acid generator.

A composition for forming a metal-containing film containing the above additives is more excellent in coating properties, dry etching resistance, and filling and planarizing properties.

Further, in the present invention, the organic solvent (B) preferably contains one or more organic solvents having a boiling point of 180°C or higher as (B1) a high-boiling-point solvent.

By imparting thermal flowability to a composition for forming a metal-containing film through adding a high-boiling-point solvent thereto, the composition for forming a metal-containing film obtains advanced filling and planarizing properties.

Further, in the present invention, the composition for forming a metal-containing film preferably comprises further (BP) a flowability accelerator having any organic group represented by the following general formula (3') and an aromatic ring, 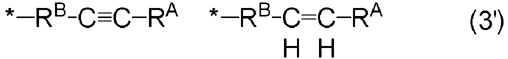 wherein "*" represents an attachment point to an oxygen atom; R^{B} represents a divalent organic group having 1 to 10 carbon atoms; and R^{A} represents a hydrogen atom or a monovalent organic group having 1 to 10 carbon atoms.

By imparting heat resistance and thermal flowability to a composition for forming a metal-containing film through adding a flowability accelerator (BP) thereto, the composition for forming a metal-containing film obtains more advanced filling and planarizing properties.

In this event, the flowability accelerator (BP) preferably has at least one constituent unit represented by the following general formulae (BP-1), (BP-2), (BP-3), (BP-4), and (BP-5), 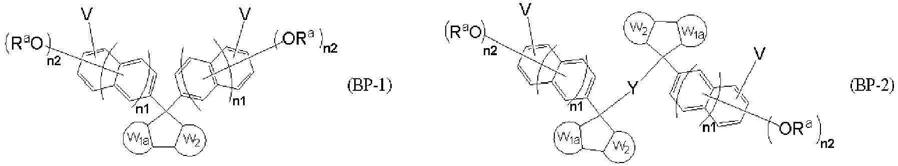
wherein W₁ₐ and W₂ each independently represent a benzene ring or a naphthalene ring, and the hydrogen atoms in the benzene ring and naphthalene ring may be substituted with a hydrocarbon group having 1 to 6 carbon atoms; R^{a} each independently represents a group represented by the following formula (4'); Y represents a group represented by the following formula (5'); n1 each independently represents 0 or 1; n2 each independently represents 1 or 2; and V each independently represents a hydrogen atom or an attachment point, 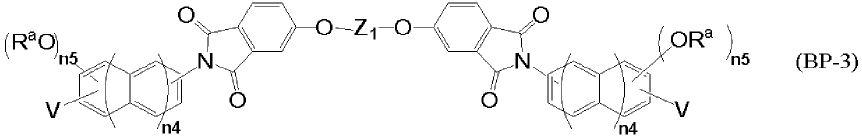
wherein Z₁ represents a group represented by the following general formula (6); R^{a} each independently represents a group represented by the following formula (4'); n4 each independently represents 0 or 1; n5 each independently represents 1 or 2; and V each independently represents a hydrogen atom or an attachment point, 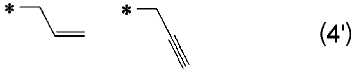
wherein "*" represents an attachment point to an oxygen atom, 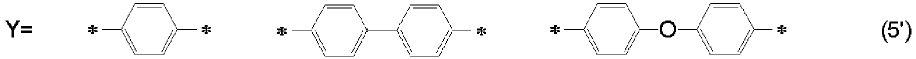
wherein "*" represent an attachment point, 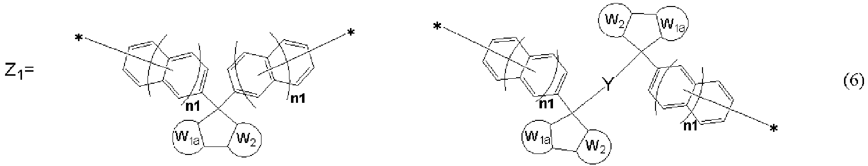
wherein W₁ₐ, W₂, Y, and n1 represent as defined above, and "*" represent an attachment point, 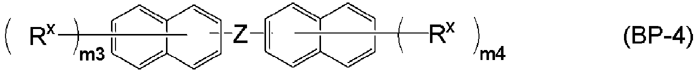
wherein m3 and m4 each independently represent 1 or 2; Z represents a single bond or any of structures represented by the following general formula (7); and R^{x} represents any of structures represented by the following general formula (8), 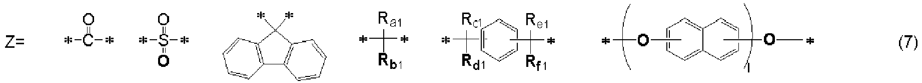
wherein "*" represents an attachment point; "l" represents an integer of 0 to 3; Rₐ₁ to R_{f1} each independently represent a hydrogen atom, an alkyl group having 1 to 10 carbon atoms, a phenyl group, or a phenylethyl group, groups which may be substituted with fluorine; and Rₐ₁ and R_{b1} may be bonded each other to form a cyclic compound, 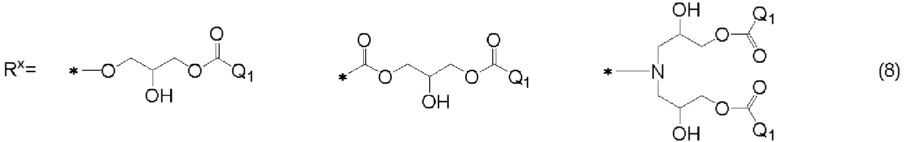
wherein "*" represents an attachment point to an aromatic ring; Q₁ is a linear saturated hydrocarbon group having 1 to 30 carbon atoms or has a structure represented by the following general formula (9), 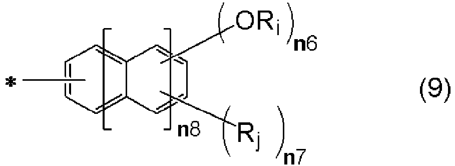
wherein "*" represents an attachment point to a carbonyl group; Rᵢ represents a group represented by the formula (4'); Rⱼ represents a linear or branched hydrocarbon group having 1 to 10 carbon atoms, a halogen atom, a nitro group, an amino group, a nitrile group, an alkoxycarbonyl group having 2 to 10 carbon atoms, or an alkanoyloxy group having 1 to 10 carbon atoms; n6 and n7 each represent the number of substituents on the aromatic ring, and each represents an integer from 0 to 7, provided that n6+n7 is an integer from 0 to 7; and n8 represents an integer from 0 to 2, 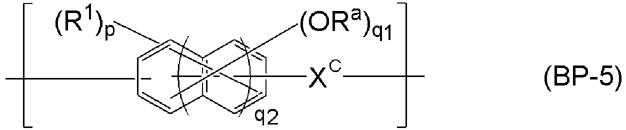
wherein R¹ represents a saturated monovalent organic group having 1 to 30 carbon atoms or an unsaturated monovalent organic group having 2 to 30 carbon atoms; X^{C} represents a divalent organic group having 1 to 30 carbon atoms; R^{a} represents a group represented by the formula (4'); "p" represents an integer from 0 to 5, q1 represents an integer from 1 to 6, and p+q1 is an integer from 1 to 6; and q2 represents 0 or 1.

When a composition for forming a metal-containing film contains a flowability accelerator (BP) having at least one constituent unit represented by the above general formulae (BP-1) to (BP-5), the composition for forming a metal-containing film is a resist material having more excellent filling and planarizing properties.

Further, the present invention provides a patterning process for forming a pattern in a substrate to be processed, comprising the steps of:
(I-1) applying the composition for forming a metal-containing film according to the above onto a substrate to be processed, followed by heating to form a metal-containing film;
(I-2) forming a resist upper layer film on the metal-containing film by using a photoresist material;
(I-3) subjecting the resist upper layer film to pattern exposure and then development with a developer to form a pattern in the resist upper layer film;
(I-4) transferring the pattern to the metal-containing film by dry etching while using the resist upper layer film having the formed pattern as a mask; and
(I-5) processing the substrate to be processed while using the metal-containing film having the transferred pattern as a mask to form the pattern in the substrate to be processed.

A fine pattern can be formed on a body to be processed (a substrate to be processed) by the patterning process using the two-layer resist process.

Further, the present invention provides a patterning process for forming a pattern in a substrate to be processed, comprising the steps of:
(II-1) applying the composition for forming a metal-containing film according to the above onto a substrate to be processed, followed by heating to form a metal-containing film;
(II-2) forming a silicon-containing resist middle layer film on the metal-containing film;
(II-3) forming a resist upper layer film on the silicon-containing resist middle layer film by using a photoresist material;
(II-4) subjecting the resist upper layer film to pattern exposure and then development with a developer to form a pattern in the resist upper layer film;
(II-5) transferring the pattern to the silicon-containing resist middle layer film by dry etching while using the resist upper layer film having the formed pattern as a mask;
(II-6) transferring the pattern to the metal-containing film by dry etching while using the silicon-containing resist middle layer film having the transferred pattern as a mask; and
(II-7) processing the substrate to be processed while using the metal-containing film having the transferred pattern as a mask to form the pattern in the substrate to be processed.

By the patterning process according to the three-layer resist process described above, a fine pattern can be formed in a body to be processed with high precision.

Further, the present invention provides a patterning process for forming a pattern in a substrate to be processed, comprising the steps of:
(III-1) applying the composition for forming a metal-containing film according to the above onto a substrate to be processed, followed by heating to form a metal-containing film;
(III-2) forming an inorganic hard mask middle layer film selected from the group consisting of a silicon oxide film, a silicon nitride film and a silicon oxynitride film on the metal-containing film;
(III-3) forming an organic thin film on the inorganic hard mask middle layer film;
(III-4) forming a resist upper layer film on the organic thin film by using a photoresist material;
(III-5) subjecting the resist upper layer film to pattern exposure and then development with a developer to form a pattern in the resist upper layer film;
(III-6) transferring the pattern to the organic thin film and the inorganic hard mask middle layer film by dry etching while using the resist upper layer film having the formed pattern as a mask;
(III-7) transferring the pattern to the metal-containing film by dry etching while using the inorganic hard mask middle layer film having the transferred pattern as a mask; and
(III-8) processing the substrate to be processed while using the metal-containing film having the transferred pattern as a mask to form the pattern in the substrate to be processed.

By the patterning process according to the four-layer resist process described above, a fine pattern can be formed in a body to be processed with high precision.

In this event, the inorganic hard mask middle layer film is preferably formed by a CVD method or an ALD method.

When the inorganic hard mask middle layer film is formed by a CVD method or an ALD method, a fine pattern can be formed in a body to be processed with higher precision.

Further, the present invention provides a patterning process for forming a pattern in a substrate to be processed, comprising the steps of:
(IV-1) applying the composition for forming a metal-containing film according to the above on a substrate to be processed, followed by heating to form a metal-containing film;
(IV-2) forming a resist underlayer film on the metal-containing film;
(IV-3) forming a silicon-containing resist middle layer film or a combination of an inorganic hard mask middle layer film selected from the group consisting of a silicon oxide film, a silicon nitride film, and a silicon oxynitride film and an organic thin film on the resist underlayer film;
(IV-4) forming a resist upper layer film on the silicon-containing resist middle layer film or the organic thin film by using a photoresist material;
(IV-5) subjecting the resist upper layer film to pattern exposure and then development with a developer to form a pattern in the resist upper layer film;
(IV-6) transferring the pattern to the silicon-containing resist middle layer film, or the organic thin film and the inorganic hard mask middle layer film by dry etching while using the resist upper layer film having the formed pattern as a mask;
(IV-7) transferring the pattern to the resist underlayer film by dry etching while using the silicon-containing resist middle layer film having the transferred pattern or the inorganic hard mask middle layer film having the transferred pattern as a mask;
(IV-8) transferring the pattern to the metal-containing film by dry etching while using the resist underlayer film having the transferred pattern as a mask; and
(IV-9) processing the substrate to be processed while using the metal-containing film having the transferred pattern as a mask to form the pattern in the substrate to be processed.

By using the patterning process according to the multilayer resist process, a fine pattern can be formed in a body to be processed with high precision.

Further, the present invention provides a tone-reversal patterning process for forming a pattern in a substrate to be processed, comprising the steps of:
(V-1) forming a resist underlayer film on a substrate to be processed;
(V-2) forming a resist middle layer film or a combination of an inorganic hard mask middle layer film selected from the group consisting of a silicon oxide film, a silicon nitride film, and a silicon oxynitride film and an organic thin film on the resist underlayer film;
(V-3) forming a resist upper layer film on the resist middle layer film or the combination of the inorganic hard mask middle layer film and the organic thin film by using a photoresist material;
(V-4) subjecting the resist upper layer film to pattern exposure and then development with a developer to form a pattern in the resist upper layer film;
(V-5) transferring the pattern to the resist middle layer film, or the organic thin film and the inorganic hard mask middle layer film by dry etching while using the resist upper layer film having the formed pattern as a mask;
(V-6) transferring the pattern to the resist underlayer film by dry etching while using the resist middle layer film having the transferred pattern, or the inorganic hard mask middle layer film having the transferred pattern as a mask;
(V-7) applying the composition for forming a metal-containing film according to the above onto the resist underlayer film having the transferred pattern, followed by heating to cover the resist underlayer film with a metal-containing film, thereby filling a space between the resist underlayer film patterns with the metal-containing film;
(V-8) etching back the metal-containing film covering the resist underlayer film having the transferred pattern by a chemical stripper or dry etching to expose an upper surface of the resist underlayer film having the formed pattern;
(V-9) removing the resist middle layer film or the inorganic hard mask middle layer film remaining on the upper surface of the resist underlayer film by dry etching;
(V-10) removing the resist underlayer film having the transformed pattern with its surface exposed by dry etching to form a reverse pattern of an original pattern in the metal-containing film; and
(V-11) processing the substrate to be processed while using the metal-containing film having the formed reverse pattern as a mask to form the reverse pattern in the substrate to be processed.

By the patterning process according to the reversal patterning process, a fine pattern can be formed on a body to be processed with even higher precision.

### ADVANTAGEOUS EFFECTS OF INVENTION

As described above, because the inventive compound for forming a metal-containing film contains one or more ligands having a crosslinking group represented by the above general formulae (a-1) to (a-3), when contained in a composition for forming a metal-containing film, it is possible to provide a composition for forming a metal-containing film that has small volume shrinkage during baking and excellent filling and planarizing properties even after high-temperature baking. Additionally, the inventive composition for forming a metal-containing film can also be used as a resist underlayer film material for forming a resist underlayer film.

In particular, in a fine patterning process according to a multilayer resist method in a semiconductor device manufacturing process, the inventive compound can be filled without causing defects such as voids and peeling off even on a substrate to be processed having a portion that is difficult to be filled and planarized, such as a dense portion of a fine pattern structure having a high aspect ratio exemplified by increasingly miniaturized DRAM. In addition, the inventive compound has better dry etching resistance than conventional coating-type organic resist underlayer film materials, and therefore, a fine pattern can be formed on a body to be processed with even higher precision.

### BRIEF DESCRIPTION OF DRAWINGS

FIG. 1 is an explanatory view of an example (three-layer resist process) of the inventive patterning process.
FIG. 2 is an explanatory view of an example (forming reverse SOC pattern of a three-layer resist process) of the inventive tone-reversal patterning process.
FIG. 3 is an explanatory view of a method for evaluating filling property.
FIG. 4 is an explanatory view of a method for evaluating planarizing property.

### DESCRIPTION OF EMBODIMENTS

As described above, in the fine patterning process according to the multilayer resist method, it has been desired to develop: a composition for forming a metal-containing film with excellent filling and planarizing properties, which is used to form a resist underlayer film that can transfer a resist pattern to a substrate to be processed with higher precision; a compound for forming a metal-containing film useful for such a composition; and a patterning process by using the composition.

The inventors have focused on metal materials that exhibit better etching resistance than conventional resist underlayer film materials and have conducted diligent study. Meanwhile, metal compounds for forming conventional resist underlayer films have poor heat resistance and undergo rapid volume shrinkage during baking. Therefore, it is difficult to fill and planarize the steps of a substrate to be processed after high-temperature baking. The present inventors considered that if the compound has an organic group with excellent heat resistance, it can reduce the sudden volume shrinkage during baking and improve thermal flowability, and therefore it is possible to fill the steps of the substrate to be processed without generating voids even after high-temperature baking. Furthermore, it was assumed that if the compound has a structure containing a crosslinking group at the terminal, it will have excellent thermosetting properties during baking, and therefore will be a compound for forming a metal-containing film with more excellent heat resistance.

As a result of further diligent study, the present inventors have found that a compound for forming a metal-containing film containing at least one compound represented by the following general formula (1) as a ligand, which contains at least one organic crosslinking group represented by the following general formulae (a-1) to (a-3), has excellent thermosetting properties, thereby reducing rapid volume shrinkage during baking, and also has good thermal flowability, thereby achieving advanced filling and planarizing properties, and have completed the present invention.

That is, the present invention is a compound for forming a metal-containing film to be contained in a composition for forming a metal-containing film, comprising: at least one metal atom selected from the group consisting of Ti, Zr, and Hf; and a ligand coordinated to the metal atom, wherein the ligand contains a ligand represented by the following general formula (1),

HO-X-R^{A1} (1)

wherein R^{A1} represents a monovalent organic group having 2 to 30 carbon atoms; X represents a single bond or a linear or branched divalent organic group having 2 to 10 carbon atoms; and either R^{A1} and X contains one or more structures represented by the following general formulae (a-1) to (a-3), 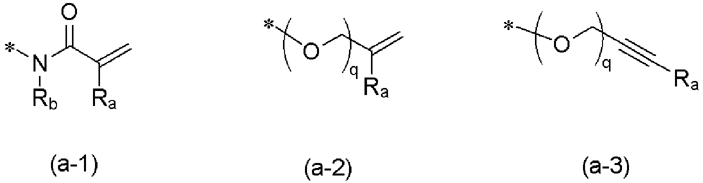
wherein Rₐ represents a hydrogen atom or a monovalent organic group having 1 to 10 carbon atoms; R_{b} represents a hydrogen atom or a monovalent organic group having 1 to 10 carbon atoms; "q" represents 0 or 1; and "*" represents an attachment point.

Hereinafter, the present invention will be specifically described, but the present invention is not limited thereto.

### <(A) compound for forming a metal-containing film>

The present invention is a compound for forming a metal-containing film to be contained in a composition for forming a metal-containing film, which includes at least one metal atom selected from the group consisting of Ti, Zr, and Hf and a ligand coordinated to the metal atom, wherein the ligand contains a ligand derived from a compound represented by the following general formula (1),

HO-X-R^{A1} (1)

wherein R^{A1} represents a monovalent organic group having 2 to 30 carbon atoms; X represents a single bond or a linear or branched divalent organic group having 2 to 10 carbon atoms; and either R^{A1} and X contains one or more structures represented by the following general formulae (a-1) to (a-3), 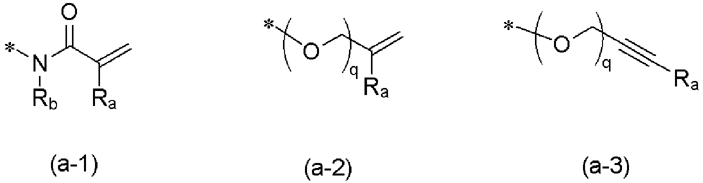
wherein Rₐ represents a hydrogen atom or a monovalent organic group having 1 to 10 carbon atoms; R_{b} represents a hydrogen atom or a monovalent organic group having 1 to 10 carbon atoms; "q" represents 0 or 1; and "*" represents an attachment point.

In the above general formula (1), R^{A1} represents a monovalent organic group having 2 to 30 carbon atoms; X represents a single bond or a linear or branched divalent organic group having 2 to 10 carbon atoms; and either R^{A1} and X contains one or more structures represented by the above general formulae (a-1) to (a-3).

In the above general formulae (a-1) to (a-3), Rₐ represents a hydrogen atom or a monovalent organic group having 1 to 10 carbon atoms, is preferably a hydrogen atom, a methyl group, or a phenyl group from the viewpoint of material availability, and more preferably a hydrogen atom from the viewpoint of thermosetting properties; R_{b} represents a hydrogen atom or a monovalent organic group having 1 to 10 carbon atoms, is preferably a hydrogen atom or a methyl group from the viewpoint of material availability; and "q" represents 0 or 1.

From the viewpoint of heat resistance, it is preferable that R^{A1} in the above general formula (1) preferably includes any one or more of aromatic rings, alicyclic hydrocarbons, and heterocycles.

When the ligand contains one or more of an aromatic ring, an alicyclic hydrocarbon, and a heterocycle, the heat resistance of the metal-containing compound is improved and the reaction of a crosslinking group contained in the ligand can be promoted, thereby suppressing volume shrinkage during high-temperature baking, and as a result, a metal-containing film with excellent filling properties can be formed.

From the viewpoint of heat resistance, it is preferable that the above ligand contains a ligand derived from a compound represented by the following general formula (2).

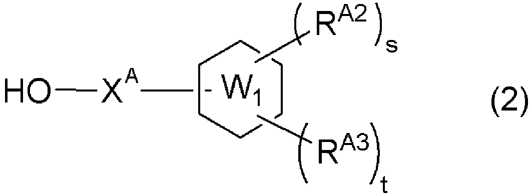

In the general formula (2), W₁ is a divalent or trivalent organic group having 3 to 20 carbon atoms and containing at least one of an aromatic ring, an alicyclic hydrocarbon, and a heterocycle; R^{A2} is a hydrogen atom, a halogen atom, or a monovalent organic group having 1 to 10 carbon atoms; R^{A3} is a monovalent organic group having 1 to 10 carbon atoms; X^{A} is a single bond or a linear or branched divalent organic group having 1 to 10 carbon atoms; either R^{A2} or X^{A} contains at least one structure represented by the general formulae (a-1) to (a-3); and "s" is an integer of 1 to 6, "t" is an integer of 0 to 5, and t+s is an integer of 1 to 6.

X^{A} in the above general formula (2) is a single bond or a linear or branched divalent organic group having 1 to 10 carbon atoms. When X^{A} contains a structure represented by the above general formulae (a-1) to (a-3), X^{A} is preferably a linear or branched divalent organic group having 3 to 10 carbon atoms, and more preferably a linear or branched divalent organic group having 3 to 5 carbon atoms. On the other hand, when X^{A} does not contain a structure represented by the above general formulae (a-1) to (a-3), X^{A} is preferably a single bond or a linear divalent organic group having 1 to 7 carbon atoms, and more preferably a single bond or a linear divalent organic group having 1 to 2 carbon atoms.

It is more preferable that W1 in the above general formula (2) has one of the following structures from the viewpoint of material availability.

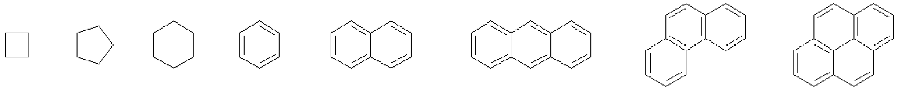

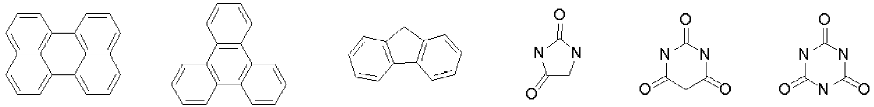

The compound for forming a metal-containing film is preferably a reaction product of a metal compound represented by the following general formula (3) or a metal-containing compound (hereinafter referred to as a metal-containing compound (a)) including any one of a hydrolysate, a condensate, and a hydrolysis condensate of a metal compound represented by the following general formula (3) and a compound represented by the above general formula (1) or (2).

LₐMX^{B}_{b} (3)

In the general formula (3), M represents any of Ti, Zr, and Hf; L represents a monodentate ligand having 0 to 30 carbon atoms or a polydentate ligand having 0 to 30 carbon atoms; X^{B} represents a hydrolyzable group selected from the group consisting of a halogen atom, an alkoxy group, a carboxylate group, an acyloxy group, and -NR^{a'}R^{b'}; R^{a'} and R^{b'} each independently represent a hydrogen atom or a monovalent organic group having 1 to 20 carbon atoms; and "a" and "b" represent integers from 0 to 4, satisfying a+b=4.

When such a compound for forming a metal-containing film is contained in a composition for forming a metal-containing film, the metal-containing film having excellent dry etching resistance with respect to oxygen gas can be formed.

### [(a) metal-containing compound]

### (Hydrolyzable group)

Examples of the hydrolyzable group X^{B} in the above general formula (3) includes a halogen atom, an alkoxy group, a carboxylate group, an acyloxy group, and - NR^{a'}R^{b'}. R^{a'} and R^{b'} are each independently a hydrogen atom or a monovalent organic group having 1 to 20 carbon atoms.

Examples of the halogen atom include a fluorine atom, a chlorine atom, a bromine atom, and an iodine atom.

Examples of the alkoxy group include a methoxy group, an ethoxy group, an n-propoxy group, an i-propoxy group, an n-butoxy group, and a t-butoxy group.

Examples of the carboxylate group include an acetate group, a propionate group, a butyrate group, an n-hexanecarboxylate group, and an n-octanecarboxylate group.

Examples of the acyloxy group include an acetoxy group, an ethyloxy group, a propionyloxy group, a butyryloxy group, a t-butyryloxy group, a t-amylyloxy group, an n-hexanecarbonyloxy group, and an n-octanecarbonyloxy group.

Examples of the -NR^{a'}R^{b'} include an unsubstituted amino group, a methylamino group, a dimethylamino group, a diethylamino group, and a dipropylamino group.

The hydrolyzable group X^{B} is preferably an alkoxy group, more preferably an i-propoxy group, an n-butoxy group, or a t-butoxy group.

L in the above general formula (3) represents a monodentate ligand having 0 to 30 carbon atoms or a polydentate ligand having 0 to 30 carbon atoms.

### (Monodentate Ligand)

Examples of the monodentate ligand include a hydroxo ligand, a carboxy ligands, an amide ligand, an amine ligand, an ammonia ligand (an ammine), an olefin ligand, etc.

Examples of the amide ligand include an unsubstituted amide ligand (NH₂), a methylamide ligand (NHMe), a dimethylamide ligand (NMe₂), a diethylamide ligand (NEt₂), a dipropylamide ligand (NPr₂), etc.

Examples of the amine ligand include pyridine, a trimethylamine ligand, a piperidine ligand, etc.

Examples of the olefin ligand include chain olefins such as ethylene and propylene; cyclic olefins such as cyclopentene, cyclohexene, and norbornene; etc.

### (Polydentate Ligand)

Examples of the polydentate ligand include ligands derived from hydroxy acid esters, ligands derived from β-diketones, ligands derived from β-ketoesters, ligands derived from α,α-dicarboxylic acid esters, hydrocarbons with a π bond, diphosphines, etc.

Examples of the hydroxy acid ester include glycolic acid ester, lactic acid ester, 2-hydroxycyclohexane-1-carboxylate, salicylic acid ester, etc.

Examples of the β-diketone include acetylacetone, 3-phenyl-2,4-pentanedione, 3-alkyl-2,4-pentanedione, 3,5-heptanedione, dipivaloylmethane, 1-phenyl-1,3-butanedione, 1,3-diphenyl-1,3-propanedione, etc.

Examples of the β-ketoester include acetoacetate ester, α-alkyl-substituted acetoacetate ester, β-ketopentanoate ester, benzoylacetate ester, 1,3-acetonedicarboxylate ester, etc.

Examples of the α,α-dicarboxylic acid ester include malonic acid diester, α-alkyl-substituted malonic acid diester, α-cycloalkyl-substituted malonic acid diester, α-aryl-substituted malonic acid diester, etc.

Examples of the hydrocarbon having a π bond include chain dienes such as butadiene and isoprene; cyclic dienes such as cyclopentadiene, methylcyclopentadiene, pentamethylcyclopentadiene, cyclohexadiene, and norbornadiene; and aromatic hydrocarbons such as benzene, toluene, xylene, hexamethylbenzene, naphthalene, and indene.

Examples of the diphosphine include 1,1-bis(diphenylphosphino)methane, 1,2-bis(diphenylphosphino)ethane, 1,3-bis(diphenylphosphino)propane, 2,2'-bis(diphenylphosphino)-1,1'-binaphthyl, and 1,1'-bis(diphenylphosphino)ferrocene.

In the above general formula (3), a+b=4, and "a" and "b" are integers of 0 to 4; "a" is preferably 0 to 3, more preferably 0 to 2, further preferably 1 or 2, and particularly preferably 2; and "b" is preferably 2 to 4, more preferably 2 or 3, and further preferably 2. By setting "a" and "b" within the above ranges, the stability of the inventive compound for forming a metal-containing film can be enhanced.

Examples of the metal-containing compound (a) include: compounds containing titanium such as diisopropoxybis(2,4-pentanedionato)titanium(IV), tetra n-butoxytitanium(IV), tetra n-propoxytitanium(IV), tri n-butoxymonostearate titanium(IV), titanium(IV) butoxide oligomer, aminopropyltrimethoxytitanium(IV), triethoxymono(2,4-pentanedionato)titanium(IV), tri-n-propoxymono(2,4-pentanedionato)titanium(IV), triisopropoxymono(2,4-pentanedionato)titanium, and di-n-butoxybis(2,4-pentanedionate)titanium(IV); compounds containing zirconium such as dibutoxybis(ethylacetoacetate)zirconium(IV), di-n-butoxybis(2,4-pentanedionato)zirconium(IV), tetra-n-butoxyzirconium(IV), tetra-n-propoxy zirconium(IV), tetraisopropoxy zirconium(IV), aminopropyl triethoxy zirconium(IV), 2-(3,4-epoxycyclohexyl)ethyl trimethoxy zirconium(IV), γ-glycidoxypropyl trimethoxy zirconium(IV), 3-isocyanopropyl trimethoxy zirconium(IV), triethoxy mono(2,4-pentanedionato)zirconium(IV), tri-n-propoxymono(2,4-pentanedionato)zirconium(IV), triisopropoxymono(2,4-pentanedionato)zirconium(IV, tri(3-methacryloxypropyl)methoxy zirconium(IV), and tri(3-acryloxypropyl) methoxy zirconium(IV); and compounds containing hafnium such as diisopropoxybis(2,4-pentanedionate)hafnium(IV), tetrabutoxyhafnium(IV), tetraisopropoxyhafnium(IV), tetraethoxyhafnium(IV), and dichlorobis(cyclopenta dienyl) hafnium(IV).

From the viewpoint of material availability, the general formula (3) preferably has a structure of the following general formula (4).

M(OR^{1A})₄ (4)

In the general formula (4), M is any one of Ti, Zr, and Hf, and R^{1A} is a monovalent organic group having 1 to 20 carbon atoms.

In the general formula (4), R^{1A} is a monovalent organic group having 1 to 20 carbon atoms, preferably a methyl group, an ethyl group, an n-propyl group, an i-propyl group, an n-butyl group, or a t-butyl group, more preferably an i-propyl group, an n-butyl group, or a t-butyl group.

At the time of the synthesis reaction of the compound (A) for forming a metal-containing film, a compound (hereinafter, a compound (b) for forming a ligand) that can be a monodentate ligand or a polydentate ligand in the compound for forming a metal-containing film can be contained besides the metal-containing compound (a) and the compound containing a group selected from the above general formulae (a-1) to (a-3) .

The compound (b) for forming a ligand include compounds having a plurality of hydroxy groups, besides compounds derived from a hydroxo ligand, a carboxy ligand, an amido ligand, an amine ligand, an ammonia ligand, an olefin ligand, etc., a ligand derived from a hydroxy acid ester, a ligand derived from a β-diketone, a ligand derived from a β-keto ester, and a ligand derived from an α,α-dicarboxylic acid ester, which were given as examples of the L in the above general formula (3) .

Furthermore, the compound (b) for forming a ligand may be an organic compound having 1 to 40 carbon atoms including at least one crosslinking group represented by any of the following general formulae (c-1) to (c-4), (d-1) to (d-4), and (e-1) to (e-3).

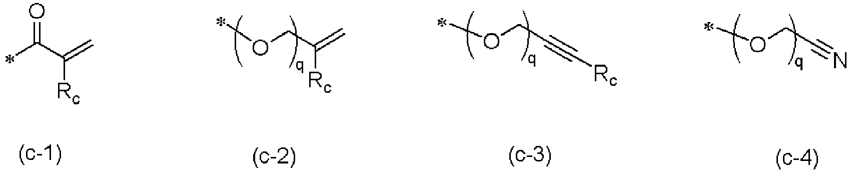

In the above general formulae (c-1) to (c-4), R_{c} represents a hydrogen atom or a monovalent organic group having 1 to 10 carbon atoms; "q" represents 0 or 1; and "*" represents a bond.

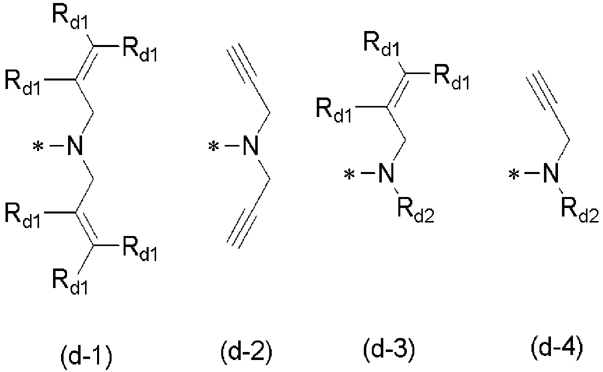

In the above general formulae (d-1) to (d-4), R_{d1}s represent a hydrogen atom or a methyl group and may be identical to or different from each other in a single formula; R_{d2} represents a hydrogen atom, a substituted or unsubstituted, saturated or unsaturated monovalent organic group having 1 to 20 carbon atoms, a substituted or unsubstituted aryl group having 6 to 30 carbon atoms, or a substituted or unsubstituted arylalkyl group having 7 to 31 carbon atoms; and "*" represents an attachment point.

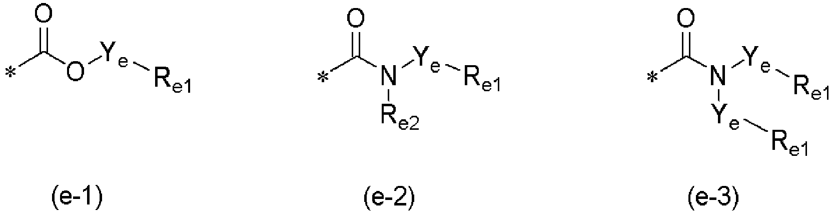

In the above general formulae (e-1) to (e-3), Yₑ represents a divalent organic group having 1 to 20 carbon atoms; Rₑ₂ represents a hydrogen atom, a substituted or unsubstituted saturated monovalent organic group having 1 to 20 carbon atoms, a substituted or unsubstituted unsaturated monovalent organic group having 2 to 20 carbon atoms, a substituted or unsubstituted aryl group having 6 to 30 carbon atoms, or a substituted or unsubstituted arylalkyl group having 7 to 31 carbon atoms; Rₑ₁ represents an organic group whose protecting group is to be removed by an action of an acid, heat, or both to generate one or more hydroxy groups or carboxy groups, Rₑ₁ being represented by one of the following general formulae (e-4); and "*" represents an attachment point.

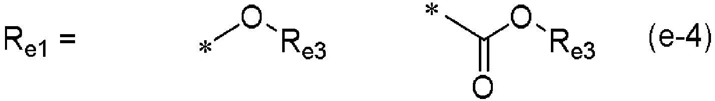

In the above general formulae (e-4), Rₑ₃ represents an organic group whose protecting group is to be removed by an action of an acid, heat, or both; and "*" represents an attachment point to Yₑ.

When the compound (b) for forming a ligand contains at least one crosslinking group represented by any of the general formulae (c-1) to (c-4), (d-1) to (d-4), and (e-1) to (e-3), the inventive compound for forming a metal-containing film synthesized by using this compound can obtain sufficient thermal flowability at the time of film formation. Therefore, when the compound is contained in a composition for forming a metal-containing film, it is possible to form a metal-containing film excellent in a planarizing property.

From the viewpoints of the productivity and stability of the compound (A) for forming a metal-containing film, the compound (b) for forming a ligand is more preferably a compound derived from a carboxy ligand, a compound derived from a ligand derived from a β-diketone, or a compound having a plurality of hydroxy groups. One kind of these compounds may be used, or two or more kinds thereof may be used in combination.

Examples of the above compound derived from the carboxy ligand include compounds represented by the following general formula (6').

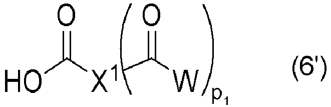

In the above general formula (6'), "p₁" represents 0 or 1. When "p₁" is 1, X¹ represents a divalent organic group having 2 to 20 carbon atoms, and W represents an alkoxy group having 1 to 10 carbon atoms, the following general formula (6-A), or any of the above general formulae (d-1) to (d-4). When "p₁" is 0, X¹ represents a monovalent organic group having 8 to 30 carbon atoms including an aromatic ring and a crosslinking group of either of the structures represented by the above general formulae (c-2) to (c-4).

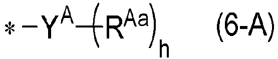

In the above general formula (6-a), Y^{A} represents a saturated divalent organic group having 1 to 20 carbon atoms or an unsaturated divalent organic group having 2 to 20 carbon atoms; R^{Aa} represents any of the structures represented by the above general formulae (c-1) to (c-4); "h" represents 1 to 6; and "*" represents an attachment point to a carbon atom of a carbonyl group.

When the inventive compound for forming a metal-containing film is synthesized with a compound derived from a carboxy ligand having such a structure, the inventive compound has improved thermosetting property. When the inventive compound is contained in a composition for forming a metal-containing film is formed, it possible to form a metal-containing film excellent in resistance against mixing with a resist upper layer film.

In the general formula (6'), it is preferable that "p₁" is 1 and W has a structure represented by the following general formula (6-B).

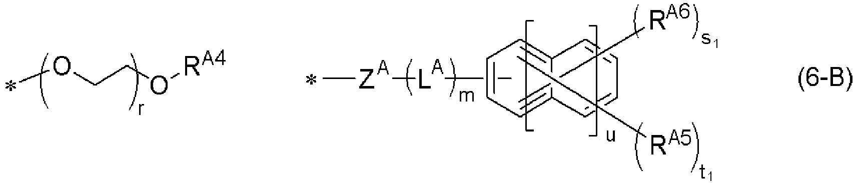

In the above general formula (6-B), R^{A4} represents a structure represented by the above general formula (c-1); R^{A5} represents a structure represented by the above general formula (c-2) or (c-3); Z^{A} represents an oxygen atom or a secondary amine; L^{A} represents a divalent hydrocarbon group having 1 to 10 carbon atoms; R^{A6} represents a saturated divalent organic group having 1 to 20 carbon atoms or an unsaturated divalent organic group having 2 to 20 carbon atoms; "t₁" is an integer of 1 to 6, "s₁" is an integer of 0 to 5, provided that t₁+s₁ is an integer of 1 or more to 6 or less; "r" is an integer of 1 to 10; "u" is 0 or 1; "m" is 0 or 1; and "*" represents an attachment point to a carbon atom of a carbonyl group.

When the inventive compound for forming a metal-containing film is synthesized by using a compound derived from a carboxy ligand having such a structure, the inventive compound has improved heat resistance characteristics. And therefore, when the inventive compound is contained in a composition for forming a metal-containing film, the formed metal-containing film exhibits excellent film-formability; the generation of sublimation products during heat-curing is suppressed; and it is possible to prevent the contamination of equipment.

In the general formula (6'), it is preferable that "p₁" is 1, W is an alkoxy group having 1 to 10 carbon atoms, and X¹ is any of the structures represented by the following formulae (6-C).

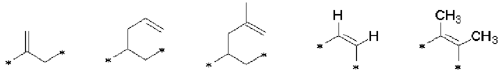

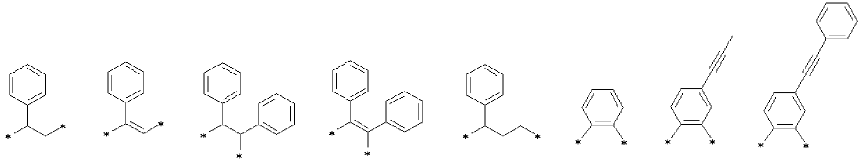

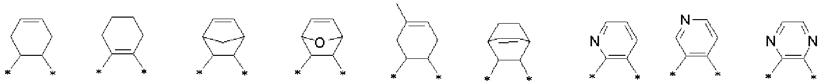

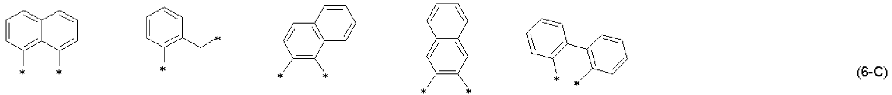

When the inventive compound for forming a metal-containing film is synthesized by using a compound derived from a carboxy ligand having such a structure, the inventive compound has improved heat resistance characteristics. And therefore, when the inventive compound is contained in a composition for forming a metal-containing film, the formed metal-containing film exhibits excellent film-formability; the generation of sublimation products during heat-curing is suppressed; and it is possible to prevent the contamination of equipment.

Alternatively, in the general formula (6'), it is preferable that "p₁" is 0, and X¹ has any of the structures represented by the following formulae (6-D).

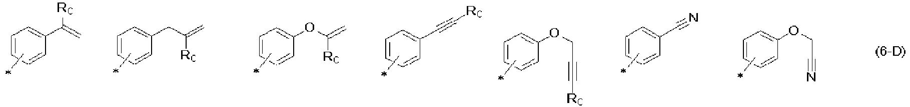

In the above formulae (6-D), R_{c} is as defined above, and "*" represents an attachment point to a carbon atom of a carbonyl group.

Examples of the above compound derived from the carboxy ligand include compounds represented by the following general formula (7').

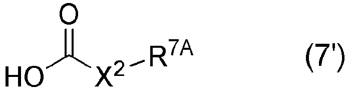

In the above general formula (7'), X² represents a divalent organic group having 1 to 31 carbon atoms; and R^{7A} represents any of the above general formulae (e-1) to (e-3).

In the above general formula (7'), X² represents a divalent organic group having 1 to 31 carbon atoms, and it is preferably a saturated divalent hydrocarbon group having 1 to 20 carbon atoms or an unsaturated divalent hydrocarbon having 2 to 20 carbon atoms from the viewpoint of thermosetting properties.

In the above general formula (e-2), Rₑ₂ is a hydrogen atom, a substituted or unsubstituted saturated monovalent organic group having 1 to 20 carbon atoms or a substituted or unsubstituted unsaturated monovalent organic group having 2 to 20 carbon atoms, a substituted or unsubstituted aryl group having 6 to 30 carbon atoms, or a substituted or unsubstituted arylalkyl group having 7 to 31 carbon atoms; and Rₑ₂ is preferably a saturated monovalent hydrocarbon group having 1 to 20 carbon atoms or an unsaturated monovalent hydrocarbon group having 2 to 20 carbon atoms.

In the general formulae (e-1) to (e-3), Yₑ is a divalent organic group having 1 to 20 carbon atoms, preferably a saturated divalent hydrocarbon group having 1 to 20 carbon atoms or an unsaturated divalent hydrocarbon group having 2 to 20 carbon atoms. When Yₑ is a saturated divalent hydrocarbon group having 1 to 20 carbon atoms or an unsaturated divalent hydrocarbon group having 2 to 20 carbon atoms, the inventive compound for forming a metal-containing film synthesized by using this can have further improved thermosetting property. Additionally, it is preferable also from the viewpoint of material availability.

Specific examples of Yₑ include the following structures but are not limited thereto. 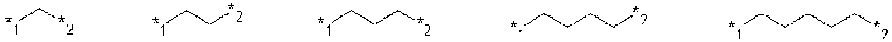 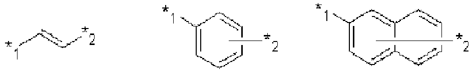 "*₁" represents an attachment point to an oxygen atom or a nitrogen atom; and "*₂" represents an attachment point to Rₑ₁.

In the above general formula (e-4), Rₑ₃ represents an organic group whose protecting group is to be removed by an action of an acid, heat, or both, preferably represents a tertiary hydrocarbyl group, or a group that forms an acetal structure together with an adjacent oxygen atom, and particularly preferably represents a tertiary hydrocarbyl group. The tertiary hydrocarbyl group preferably has 4 to 20 carbon atoms, and those having fewer carbon atoms are more preferable among those.

Examples of the compound derived from a ligand derived from a β-diketone include compounds represented by the following general formula (8').

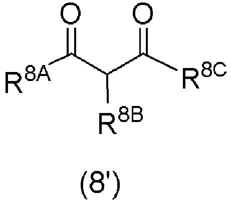

In the above general formula (8'), R^{8A} to R^{8C} each represent a hydrogen atom or a monovalent organic group having 1 to 20 carbon atoms and optionally containing a crosslinking group represented by any of the above general formulae (c-1) to (c-4) and (d-1) to (d-4).

The compounds of the above general formula (8') preferably contain at least one crosslinking group represented by any of the above general formulae (c-1) to (c-4) and (d-1) to (d-4). When the inventive composition for forming a metal-containing film is synthesized by using such a compound, the thermosetting property of the compound can be improved further, and therefore it is preferable.

Examples of the compound having a plurality of hydroxy groups include dihydric alcohols represented by any of the following general formulae (8-A) to (8-C), etc.

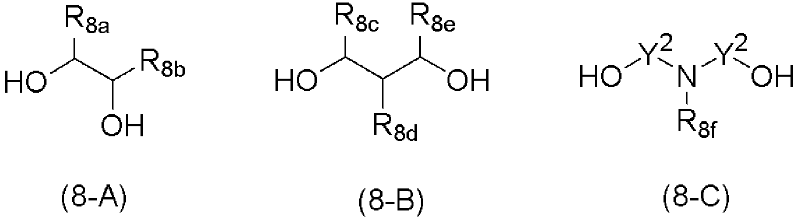

In the above general formulae (8-A) to (8-C), R₈ₐ to R_{8b} represent a hydrogen atom or a monovalent organic group having 1 to 20 carbon atoms and optionally containing a crosslinking group represented by any of the above general formulae (c-1) to (c-4); R_{8c} to R_{8f} represent a hydrogen atom or a monovalent organic group having 1 to 20 carbon atoms and optionally containing a crosslinking group represented by any of the following general formulae (c-1) to (c-4) and (d-1) to (d-4); and Y² represents a divalent organic group having 1 to 10 carbon atoms. In the above general formula (8-A), R₈ₐ and R_{8b} may be bonded to each other to form an unsaturated or saturated cyclic structure.

The compounds of the above general formulae (8-A) to (8-C) preferably contain at least one crosslinking group represented by any of the above general formulae (c-1) to (c-4) and (d-1) to (d-4). When a compound for forming a metal-containing film is synthesized by using such a compound, thermosetting property of the compound is further improved, and thus it is preferable.

In the compound (A) for forming a metal-containing film, the ligand containing any of the groups selected from the above formulae (a-1) to (a-3) is preferably contained in an amount of 10 mol% to 90 mol%, more preferably 20 mol% to 80 mol%, and further preferably 25 mol% to 75 mol% of the total amount of ligands coordinated to the metal atom. The ligand derived from the compound (b) for forming a ligand is preferably contained in an amount of 0 mol% to 50 mol%, more preferably 0 mol% to 30 mol% of the total amount of ligands coordinated to the metal atom. Ligands other than the ligand containing any of the groups selected from the above formulae (a-1) to (a-3) and the ligand derived from the compound (b) for forming a ligand, for example, a ligand derived from an alkoxy group having 1 to 10 carbon atoms, are preferably contained in an amount of 0 mol% to 90 mol%, more preferably 20 mol% to 80 mol% of the total amount of ligands coordinated to the metal atom.

Furthermore, on the occasion of the synthesis reaction of the compound (A) for forming a metal-containing film, a silicon compound (c) may be added besides the metal-containing compound (a) and compound containing the groups selected from the formulae (a-1) to (a-3).

By substituting a hydrolysable group of the metal compound (a) with the silicon-containing compound, it is possible to enhance stability of the compound for forming a metal-containing film in the composition for forming a metal-containing film.

That is, the compound for forming a metal-containing film preferably further contains a ligand derived from a silicon compound represented by the following general formula (5).

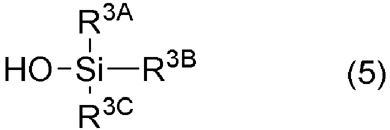

In the general formula (5), R^{3A}, R^{3B}, and R^{3C} represent any organic group selected from an organic group having 1 to 30 carbon atoms including a crosslinking group of a structure represented by any of the following general formulae (b-1) to (b-3), a substituted or unsubstituted alkyl group having 1 to 20 carbon atoms, and an aryl group having 6 to 20 carbon atoms.

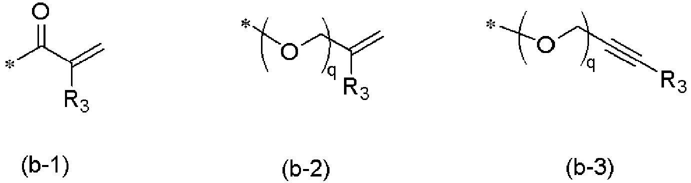

In the general formulae (b-1) to (b-3), R₃ represents a hydrogen atom or a monovalent organic group having 1 to 10 carbon atoms; "q" represents 0 or 1; and "*" represents an attachment point.

As the silicon compound (c), any group of the following formulae is preferable, and from the viewpoint of productivity, trimethylsilanol is more preferable.

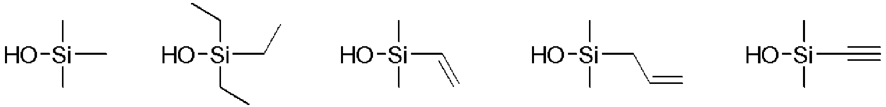

In the above formula, "*" represents an attachment point to a metal atom.

When the compound (A) for forming a metal-containing film contains the ligand containing a group selected from the above general formulae (a-1) to (a-3) and the ligand derived from the silicon compound (c), the amount of the ligand containing a group selected from the above general formulae (a-1) to (a-3) in the compound (A) for forming a metal-containing film is preferably 10 mol% to 90 mol%, more preferably 20 mol% to 80 mol%, and further preferably 25 mol% to 75 mol% of the total amount of ligands coordinated to the metal atom. The ligand derived from the silicon compound (c) is preferably contained in an amount of 10 mol% to 90 mol%, more preferably 20 mol% to 80 mol% of the total amount of ligands coordinated to the metal atom. Ligands other than the ligand containing a group selected from the above general formulae (a-1) to (a-3) and the ligand derived from the silicon compound (c), for example, a ligand derived from an alkoxy group having 1 to 10 carbon atoms, are preferably contained in an amount of 0 mol% to 90 mol%, more preferably 20 mol% to 80 mol% of the total amount of ligands coordinated to the metal atom.

When a compound (A) for forming a metal-containing film, containing a ligand containing a group selected from the above general formulae (a-1) to (a-3), is to be synthesized, the synthesis method is not particularly limited as long as the compound (A) for forming a metal-containing film contains a ligand containing a group selected from the above general formulae (a-1) to (a-3). For example, a metal alkoxide or a metal acetylacetonate (acac) can be used as the metal-containing compound (a), and the compound for forming a metal-containing film can be obtained by allowing the metal alkoxide or metal acac to react with a compound containing a group selected from the above general formulae (a-1) to (a-3). The metal-containing compound (a) may be subjected to hydrolysis condensation and then the product may be allowed to react with the compound containing a group selected from the above general formulae (a-1) to (a-3). Alternatively, the metal-containing compound (a) may be allowed to react with the compound containing a group selected from the above general formulae (a-1) to (a-3) and then the product may be subjected to hydrolysis condensation. When it is difficult to control the hydrolysis condensation, the reaction with the compound containing a group selected from the above general formulae (a-1) to (a-3) may be performed in a non-hydrous environment. The method is preferably adjusted appropriately in accordance with the properties required as the compound (A) for forming a metal-containing film. When a compound derived from the silicon compound (c) and a compound containing a group selected from the above general formulae (a-1) to (a-3) are to be contained as ligands, it is preferable to allow the metal-containing compound (a) and the silicon compound (c) to react and then allow the product to react with a compound containing a group selected from the above general formulae (a-1) to (a-3).

As a method for performing a hydrolysis condensation reaction using the metal compound (a), examples include a method of subjecting the metal compound (a) to a hydrolysis condensation reaction in a solvent containing water. In this case, another compound having a hydrolysable group may be added as necessary. In addition, as a catalyst for the hydrolysis condensation reaction, an acid such as acetic acid may be added. The lower limit of the amount of water used in this hydrolysis condensation reaction is preferably 0.2 times in mole, more preferably 1 time in mole, and further preferably 3 times in mole, relative to the hydrolysable groups contained in the metal-containing compound (a), etc. The upper limit of the amount of the water is preferably 20 times in mole, more preferably 15 times in mole, and further preferably 10 times in mole.

The solvent used in the synthesis reaction of the compound (A) for forming a metal-containing film is not particularly limited, and for example, a solvent as given as an example of the organic solvent (B) described later can be used. Typical solvents and solvent mixtures contain an ester, ether, or alcohol functional group, and examples thereof include a mixture of propylene glycol monomethyl ether acetate (PGMEA) and propylene glycol monomethyl ether (PGME) at a volume ratio of 70/30. Examples of other solvents that can be used include butanediol monomethyl ether, ethylene glycol monomethyl ether, butanediol monoethyl ether, propylene glycol monoethyl ether, ethylene glycol monoethyl ether, butanediol monopropyl ether, propylene glycol monopropyl ether, ethylene glycol monopropyl ether, ethylene glycol monobutyl ether, diethylene glycol monomethyl ether, diethylene glycol monoethyl ether, diethylene glycol monopropyl ether, diethylene glycol monobutyl ether, propylene glycol monobutyl ether, 1-butanol, 2-butanol, 2-methyl-1-propanol, 4-methyl-2-pentanol, acetone, tetrahydrofuran, toluene, hexane, ethyl acetate, cyclohexanone, methyl amyl ketone, propylene glycol dimethyl ether, diethylene glycol dimethyl ether, diamyl ether, isoamyl ether, propylene glycol monomethyl ether acetate, propylene glycol monoethyl ether acetate, ethyl pyruvate, butyl acetate, methyl 3-methoxypropionate, ethyl 3-ethoxypropionate, t-butyl acetate, t-butyl propionate, propylene glycol mono-t-butyl ether acetate, γ-butyrolactone, methyl isobutyl ketone, cyclopentyl methyl ether, etc.

### <Composition for Forming Metal-Containing Film>

In addition, the present invention provides a composition for forming a metal-containing film used for manufacturing a semiconductor, containing the compound (A) for forming a metal-containing film which is described above, and an organic solvent (B.

Such a composition for forming a metal-containing film contains a compound for forming a metal-containing film that achieve both high thermal flowability and high thermosetting property, and therefore it is possible to provide a composition for forming a metal-containing film as a resist underlayer film material that has better dry etching resistance than conventional resist underlayer film materials and also has advanced filling and planarizing properties.

Hereinafter, the components contained in the inventive composition for forming a metal-containing film other than the compound (A) for forming a metal-containing film will be described.

### <(B) Organic Solvent>

The organic solvent (B) usable in the inventive composition for forming a metal-containing film is not particularly limited as long as the organic solvent is capable of dissolving or dispersing the above compound (A) for forming a metal-containing film, and when contained, (E) a crosslinking agent, (G) a surfactant, (H) an acid generator, other additives, etc. described later.

Specifically, for example, an organic solvent disclosed in paragraphs [0091] and [0092] in JP2007-199653A may be contained. Further specifically, it is preferable to use propylene glycol monomethyl ether acetate, propylene glycol monomethyl ether, propylene glycol monoethyl ether, propylene glycol monopropyl ether, 2-heptanone, cyclopentanone, cyclohexanone, γ-butyrolactone, or a mixture containing one or more of these solvents.

The organic solvent (B) is preferably contained in an amount of 200 to 10,000 parts by mass, more preferably 250 to 5,000 parts by mass relative to 100 parts by mass of the compound (A) for forming a metal-containing film.

The composition for forming a metal-containing film only need to contain one or more the compound (A) for forming a metal-containing film and the organic solvent (B), and may contain an additive such as a crosslinking agent (E), a surfactant (G), an acid generator (H), etc. as necessary. That is, the composition preferably further contains at least one or more of a crosslinking agent (E), a surfactant (G), and an acid generator (H).

Hereinafter, components other than the compound (A) for forming a metal-containing film and the organic solvent (B), will be described, which is contained in the inventive composition for forming a metal-containing film.

### <(B1) High-Boiling-Point Solvent>

In the inventive composition for forming a resist underlayer film, the organic solvent (B) may be a mixture of one or more kinds of organic solvent having a boiling point of lower than 180°C and one or more kinds of organic solvent having a boiling point of 180°C or higher (high-boiling-point solvent (B1)). That is, the organic solvent (B) preferably contains one or more organic solvents having a boiling point of 180°C or higher as the high-boiling-point solvent (B1). Note that, the boiling point is a boiling point at normal pressure.

The high-boiling-point solvent (B1) is not particularly limited to hydrocarbons, alcohols, ketones, esters, ethers, or chlorine-based solvents, etc., as long as the solvent is capable of dissolving or dispersing each component of the inventive composition for forming a metal-containing film. Specific examples include 1-octanol, 2-ethylhexanol, 1-nonanol, 1-decanol, 1-undecanol, ethylene glycol, 1,2-propylene glycol, 1,3-butylene glycol, 2,4-pentanediol, 2-methyl-2,4-pentanediol, 2,5-hexanediol, 2,4-heptanediol, 2-ethyl-1,3-hexanediol, diethylene glycol, dipropylene glycol, triethylene glycol, tripropylene glycol, glycerin, n-nonyl acetate, ethylene glycol monohexyl ether, ethylene glycol mono-2-ethylhexyl ether, ethylene glycol monophenyl ether, ethylene glycol monobenzyl ether, diethylene glycol monoethyl ether, diethylene glycol monoisopropyl ether, diethylene glycol mono-n-butyl ether, diethylene glycol monoisobutyl ether, diethylene glycol monohexyl ether, diethylene glycol monophenyl ether, diethylene glycol monobenzyl ether, diethylene glycol diethyl ether, diethylene glycol dibutyl ether, diethylene glycol butyl methyl ether, triethylene glycol dimethyl ether, triethylene glycol monomethyl ether, triethylene glycol-n-butyl ether, triethylene glycol butyl methyl ether, tetraethylene glycol dimethyl ether, dipropylene glycol monomethyl ether, dipropylene glycol mono-n-propyl ether, dipropylene glycol mono-n-butyl ether, tripropylene glycol dimethyl ether, tripropylene glycol monomethyl ether, tripropylene glycol mono-n-propyl ether, tripropylene glycol mono-n-butyl ether, ethylene glycol monoethyl ether acetate, ethylene glycol monobutyl ether acetate, diethylene glycol monomethyl ether acetate, diethylene glycol monoethyl ether acetate, diethylene glycol monobutyl ether acetate, triacetin, propylene glycol diacetate, dipropylene glycol methyl-n-propyl ether, dipropylene glycol methyl ether acetate, 1,4-butanediol diacetate, 1,3-butylene glycol diacetate, 1,6-hexanediol diacetate, triethylene glycol diacetate, γ-butyrolactone, methyl benzoate, ethyl benzoate, propyl benzoate, butyl benzoate, dihexyl malonate, diethyl succinate, dipropyl succinate, dibutyl succinate, dihexyl succinate, dimethyl adipate, diethyl adipate, dibutyl adipate, etc. These may be used alone or in combination.

The high-boiling-point solvent (B1) may be selected suitably from the solvents above, for example, according to the temperature at which the inventive composition for forming a metal-containing film is heat-treated, etc. The boiling point of the high-boiling-point solvent is preferably 180°C to 300°C, further preferably 200°C to 300°C. When the boiling point is as such, sufficient thermal flowability can be achieved at the time of film formation, because evaporation rate at the baking (heating) is not excessive. Therefore, it is assumed that a resist underlayer film excellent in filling and planarizing properties can be formed. Moreover, a solvent having such a boiling point evaporates after the baking and does not remain in the film. Therefore, there is no adverse effect on the physical properties of the film, such as etching resistance.

When the high-boiling-point solvent (B1) is used, the amount to be used is preferably 1 to 30 parts by mass relative to 100 parts by mass of the organic solvent having a boiling point of lower than 180°C. When the contained amount is as such, sufficient thermal flowability can be imparted at the time of baking, and the solvent does not remain in the film and lead to degradation of physical properties, such as filling property, of the film. Therefore, such an amount is preferable.

### [(E) Crosslinking Agent]

The inventive composition for forming a metal-containing film can further contain a crosslinking agent (E) to increase curability of the compound (A) for forming a metal-containing film and further inhibit intermixing with a resist upper layer film. The crosslinking agent (E) is not particularly limited, and various known crosslinking agents can be widely used. Examples include melamine-based crosslinking agents, acrylate-based crosslinking agent, glycoluril-based crosslinking agents, benzoguanamine-based crosslinking agents, urea-based crosslinking agents, β-hydroxyalkylamide-based crosslinking agents, isocyanurate-based crosslinking agents, aziridine-based crosslinking agents, oxazoline-based crosslinking agents, epoxy-based crosslinking agents, and phenol-based crosslinking agents (e.g. polynuclear phenol-based, such as methylol or alkoxymethyl-based crosslinking agents). The contained amount of the crosslinking agent (E) is preferably 5 to 50 parts by mass, more preferably 10 to 40 parts by mass based on 100 parts by mass of the compound (A) for forming a metal-containing film.

Specific examples of the melamine-based crosslinking agents include hexamethoxymethylated melamine, hexabutoxymethylated melamine, alkoxy- and/or hydroxy-substituted derivatives thereof, and partial self-condensates thereof.

Specific examples of the acrylate-based crosslinking agents include dipentaerythritol hexaacrylate.

Specific examples of the glycoluril-based crosslinking agents include tetramethoxymethylated glycoluril, tetrabutoxymethylated glycoluril, alkoxy-and/or hydroxy-substituted derivatives thereof, and partial self-condensates thereof.

Specific examples of the benzoguanamine-based crosslinking agents include tetramethoxymethylated benzoguanamine, tetrabutoxymethylated benzoguanamine, alkoxy- and/or hydroxy-substituted derivatives thereof, and partial self-condensates thereof.

Specific examples of the urea-based crosslinking agents include dimethoxymethylated dimethoxyethyleneurea, alkoxy- and/or hydroxy-substituted derivatives thereof, and partial self-condensates thereof.

Specific examples of the β-hydroxyalkylamide-based crosslinking agent include N,N,N',N'-tetra(2-hydroxyethyl)adipic acid amide.

Specific examples of the isocyanurate-based crosslinking agents include triglycidyl isocyanurate and triallyl isocyanurate.

Specific examples of the aziridine-based crosslinking agents include 4,4'-bis(ethyleneiminocarbonylamino)diphenylmethane and 2,2-bishydroxymethylbutanol-tris[3-(1-aziridinyl)propionate] .

Specific examples of the oxazoline-based crosslinking agents include 2,2'-isopropylidene bis(4-benzyl-2-oxazoline), 2,2'-isopropylidene bis(4-phenyl-2-oxazoline), 2,2'-methylene bis-4,5-diphenyl-2-oxazoline, 2,2'-methylene bis-4-phenyl-2-oxazoline, 2,2'-methylene bis-4-tert-butyl-2-oxazoline, 2,2'-bis(2-oxazoline), 1,3-phenylene bis(2-oxazoline), 1,4-phenylene bis(2-oxazoline), and a 2-isopropenyloxazoline copolymer.

Specific examples of the epoxy-based crosslinking agent include diglycidyl ether, ethylene glycol diglycidyl ether, 1,4-butanediol diglycidyl ether, 1,4-cyclohexanedimethanol diglycidyl ether, poly(glycidyl methacrylate), trimethylolethane triglycidyl ether, trimethylolpropane triglycidyl ether, and pentaerythritol tetraglycidyl ether.

Specific examples of the polynuclear phenol-based crosslinking agents include compounds represented by the following general formula (XL-1).

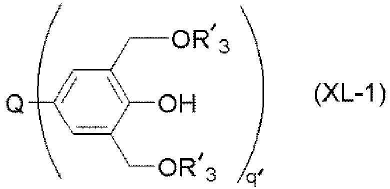

In the general formula (XL-1), Q represents a single bond or a q'-valent hydrocarbon group having 1 to 20 carbon atoms; R'₃ represents a hydrogen atom or an alkyl group having 1 to 20 carbon atoms; and "q'" represents an integer of 1 to 5.

Q represents a single bond or a q'-valent hydrocarbon group having 1 to 20 carbon atoms. "q'" represents an integer of 1 to 5, more preferably 2 or 3. Specific examples of Q include groups obtained by removing "q'" hydrogen atoms from methane, ethane, propane, butane, isobutane, pentane, cyclopentane, hexane, cyclohexane, methylpentane, methylcyclohexane, dimethylcyclohexane, trimethylcyclohexane, benzene, toluene, xylene, ethylbenzene, ethylisopropylbenzene, diisopropylbenzene, methylnaphthalene, ethylnaphthalene, and eicosane. R'₃ represents a hydrogen atom or an alkyl group having 1 to 20 carbon atoms. Specific examples of the alkyl groups having 1 to 20 carbon atoms include a methyl group, an ethyl group, a propyl group, an isopropyl group, a butyl group, an isobutyl group, a pentyl group, an isopentyl group, a hexyl group, an octyl group, an ethylhexyl group, a decyl group, and an eicosanyl group. A hydrogen atom or a methyl group is preferable.

Specific examples of the compounds represented by the above general formula (XL-1) include the following compounds. Among these, a hexamethoxymethylated derivative of triphenolmethane, triphenolethane, 1,1,1,-tris(4-hydroxyphenyl)ethane, and tris(4-hydroxyphenyl)-1-ethyl-4-isopropylbenzene are preferable from the viewpoint of improving the curability and film thickness uniformity of the metal-containing film. R'₃ is the same as defined above.

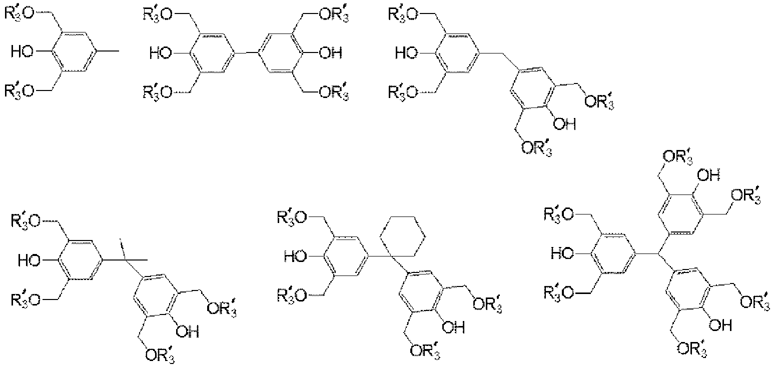

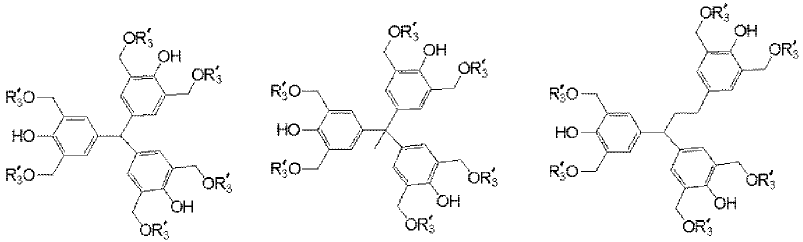

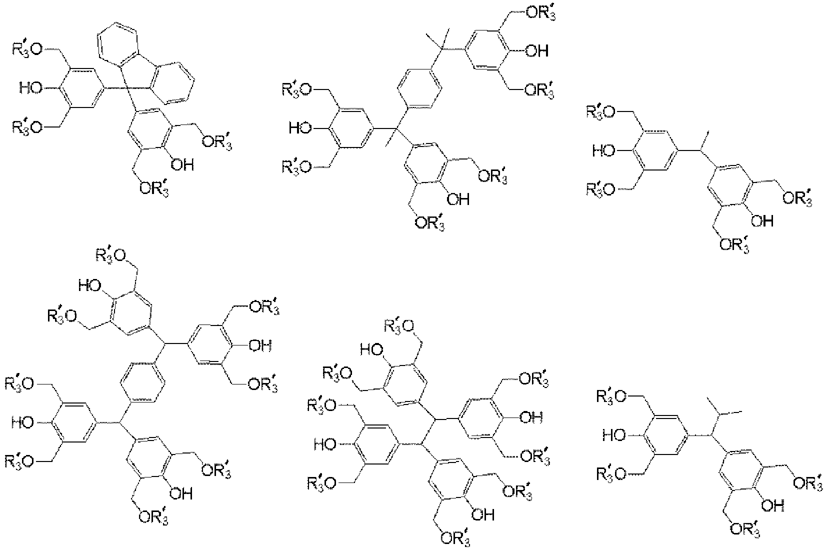

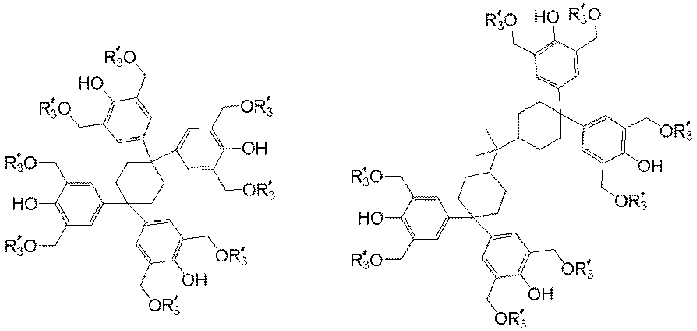

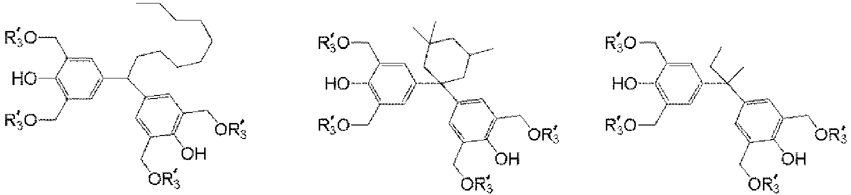

### <(G) Surfactant>

A surfactant (G) may be contained in the inventive composition for forming a metal-containing film in order to improve coating properties in spin-coating. One surfactant (G) can be used, or two or more surfactants (G) can be used in combination. Examples of the surfactant (G) to be used include those disclosed in paragraphs [0142] to [0147] of JP2009-269953A. When the surfactant (G) is contained, the contained amount is preferably 0.01 to 20 parts by mass, more preferably 0.01 to 10 parts by mass per 100 parts by mass of the compound (A) for forming a metal-containing film. When the amount is in such a range, the coating properties are certainly improved, and a thin, uniform, metal-containing film can be formed.

### <(H) Acid Generator>

An acid generator (H) may be contained in the inventive composition for forming a metal-containing film in order to promote curing reaction of the compound (A) for forming a metal-containing film further. The acid generators (H) include those that generate an acid by thermal decomposition and those that generate an acid by light irradiation, but any acid generators can be included. Specifically, the materials disclosed in paragraphs [0061] to [0085] of JP2007-199653A can be included but are not limited thereto.

One kind of the acid generators can be used, or two or more kinds thereof may be used in combination. When the acid generator (H) is contained, the contained amount is preferably 0.05 to 50 parts by mass, more preferably 0.1 to 10 parts by mass relative to 100 parts by mass of the compound (A) for forming a metal-containing film.

### <Other Additives>

In addition, in the inventive composition for forming a metal-containing film, it is preferable to use, as an additive for imparting filling and planarizing properties, for example, a liquid additive having a polyethylene glycol or polypropylene glycol structure, or a thermo-decomposable polymer having a weight reduction rate of 40 mass% or more from 30°C to 250°C and a weight-average molecular weight of 300 to 200,000. This thermo-decomposable polymer preferably contains a repeating unit having an acetal structure represented by the following general formula (DP1) or (DP1a).

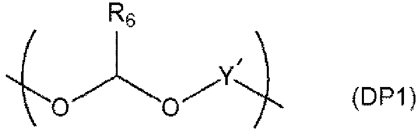

In the general formula (DP1), R₆ represents a hydrogen atom or a substituted or unsubstituted, saturated or unsaturated monovalent organic group, which may be substituted, having 1 to 30 carbon atoms, and Y' represents a saturated or unsaturated divalent organic group having 2 to 30 carbon atoms.

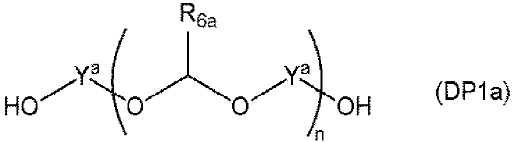

In the general formula (DP1a), R₆ₐ represents an alkyl group having 1 to 4 carbon atoms; Y^{a} represents a saturated or unsaturated divalent hydrocarbon group having 4 to 10 carbon atoms and optionally having an ether bond; and "n" represents an average repeating unit number of 3 to 500.

### <(BP) Flowability Accelerator>

Furthermore, a flowability accelerator (BP) can be added to the inventive composition for forming a metal-containing film as an additive for imparting filling and planarizing properties. The flowability accelerator (BP) preferably has any of the organic groups represented by the following general formula (3') and an aromatic ring. That is, the composition for forming a metal-containing film further contains a flowability accelerator (BP) having any of the organic groups represented by the following general formula (3') and an aromatic ring.

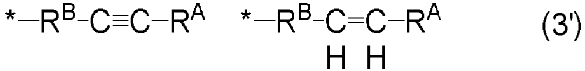

In the general formula (3'), "*" represents an attachment point to an oxygen atom; R^{B} is a divalent organic group having 1 to 10 carbon atoms; and R^{A} is a hydrogen atom or a monovalent organic group having 1 to 10 carbon atoms.

The flowability accelerator (BP) has an organic group of the above general formula (3'), so that thermal flowability and thermosetting property can be imparted to the composition for forming a metal-containing film. In addition, the flowability accelerator (BP) has an aromatic ring, so that the composition for forming a metal-containing film can have deterioration of dry etching resistance reduced.

The flowability accelerator (BP) preferably has at least one constituent unit represented by the following general formulae (BP-1), (BP-2), (BP-3), (BP-4), and (BP-5).

### (Constituent Unit: BP-1, BP-2, and BP-3)

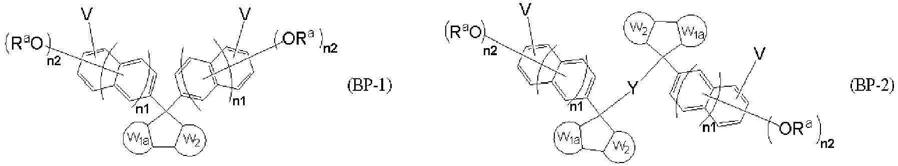

In the general formulae (BP-1) and (BP-2), W₁ₐ and W₂ each independently represent a benzene ring or a naphthalene ring, and the hydrogen atoms in the benzene ring and naphthalene ring may be substituted with a hydrocarbon group having 1 to 6 carbon atoms; R^{a} each independently represents a group represented by the following formula (4'); Y represents a group represented by the following formula (5'); "n1" each independently represents 0 or 1; "n2" each independently represents 1 or 2; and V each independently represents a hydrogen atom or an attachment point.

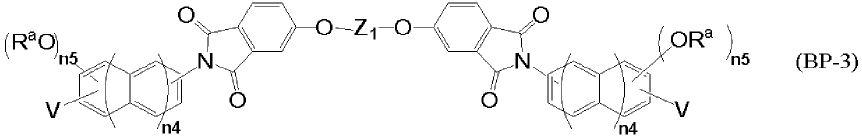

In the general formula (BP-3), Z₁ represents a group represented by the following general formula (6); R^{a} each independently represents a group represented by the following formula (4'); "n4" each independently represents 0 or 1; "n5" each independently represents 1 or 2; and V each independently represents a hydrogen atom or an attachment point.

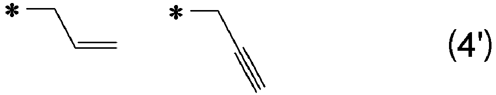

In the formula (4'), "*" represents an attachment point to an oxygen atom.

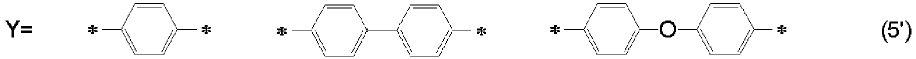

In the formula (5'), "*" represents an attachment point.

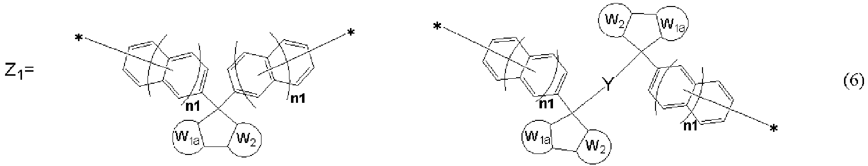

In the general formula (6), W₁ₐ, W₂, Y, and "n1" represent as defined above, and "*" represent an attachment point.

Resins having constituent units represented by the above general formulae (BP-1), (BP-2), and (BP-3) have excellent heat resistance because they have a condensed carbon ring with high carbon density, including a cardo structure. Because of the above characteristics, a metal-containing film capable of filling a stepped substrate without generating a void can be formed even when subjected to high-temperature baking. In addition, because the resin has excellent dry etching resistance, even when added to the inventive composition for forming a metal-containing film, the composition for forming a metal-containing film can impart heat resistance and thermal flowability without significantly deteriorating the excellent dry etching resistance of the composition for forming a metal-containing film.

The resin having the constituent unit represented by the above general formulae (BP-1), (BP-2), and (BP-3) may be a compound represented by the following general formulae (bp-1), (bp-2), and/or (bp-3).

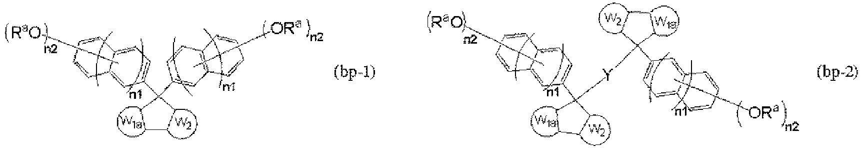

In the general formulae (bp-1) and (bp-2), W₁ₐ, W₂, R^{a}, Y, "n1", and "n2" are the same as defined above.

In the above general formulae (bp-1) and (bp-2), W₁ₐ, W₂, R^{a}, Y, "n1", and "n2" are as explained for the above general formulae (BP-1) and (BP-2).

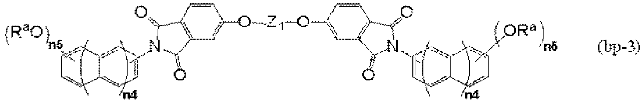

In the general formula (bp-3), Z₁, R^{a}, "n4", and "n5" are the same as defined above.

In the above general formula (bp-3), Z₁, R^{a}, "n4", and "n5" are as explained for the above general formula (BP-3).

Specific examples of the resin having the constituent unit represented by the above general formulae (BP-1), (BP-2), and (BP-3) include the following compounds but are not limited thereto.

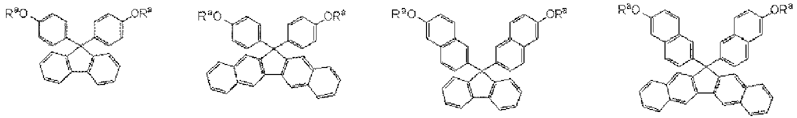

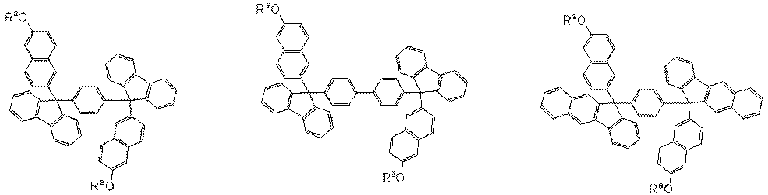

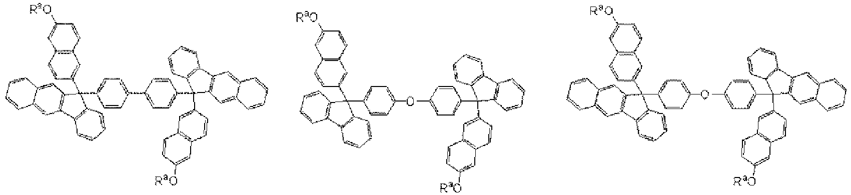

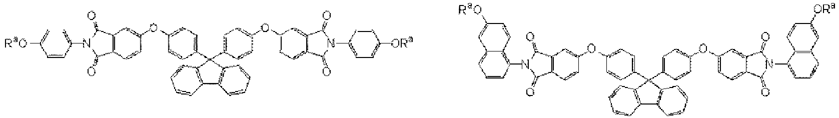

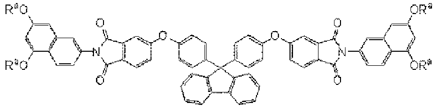

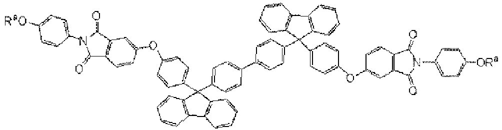

The compounds represented by the above general formulae (bp-1), (bp-2), and (bp-3) preferably have a ratio Mw/Mn (i.e., dispersity) of the weight average molecular weight Mw and the number average molecular weight Mn in terms of polystyrene, which are determined by a gel permeation chromatography method, within the range of 1.00≤Mw/Mn≤1.25, and more preferably 1.00≤Mw/Mn≤1.10.

With a compound having a dispersity in such a range, the composition for forming a metal-containing film has better thermal flowability, and thus, when incorporated into the composition, can provide a composition for forming a metal-containing film having more excellent filling and planarizing properties.

The resin having the constituent unit represented by the above general formulae (BP-1), (BP-2), and (BP-3) may be a polymer having a repeating unit represented by the following general formulae (bp-4), (bp-5), and/or (bp-6).

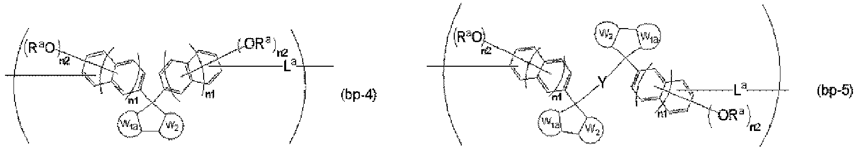

In the general formulae (bp-4) and (bp-5), W₁ₐ, W₂, R^{a}, Y, "n1", and "n2" are the same as defined above, and L^{a} is a divalent organic group having 1 to 40 carbon atoms.

In the above general formulae (bp-4) and (bp-5), W₁ₐ, W₂, R^{a}, Y, "n1", and "n2" are as explained in the above general formulae (BP-1) and (BP-2).

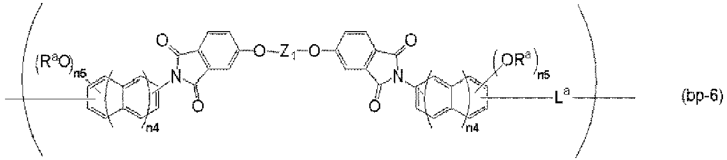

In the general formula (bp-6), Z₁, R^{a}, "n4", and "n5" are the same as defined above, and L^{a} is a divalent organic group having 1 to 40 carbon atoms.

In the above general formula (bp-6), Z₁, R^{a}, "n4", and "n5" are as explained in the above general formula (BP-3).

These are polymers obtained by using the compounds represented by the above general formulae (bp-1), (bp-2), and (bp-3), and because of using the above compounds they have excellent dry etching resistance and heat resistance. In addition, because they are polymers having a repeating unit rather than a monomer, they have fewer outgassing components. Furthermore, because the polymers have molecular weight distribution, their crystallinity is reduced and improvement in film formability can be expected.

L^{a}, which is a linking group constituting the repeating units of the above general formulae (bp-4), (bp-5), and (bp-6), is a divalent organic group having 1 to 40 carbon atoms, and specific examples thereof include the following.

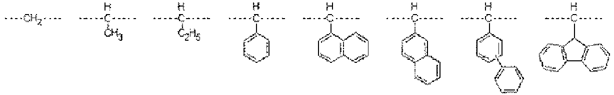

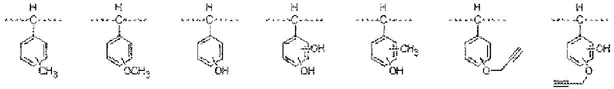

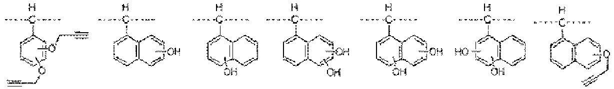

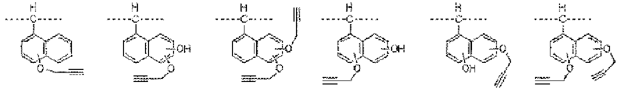

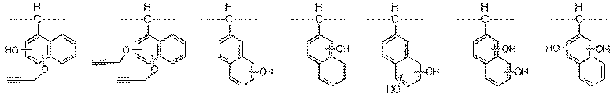

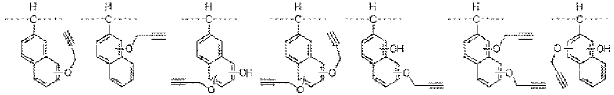

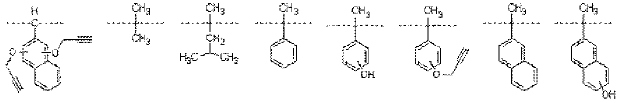

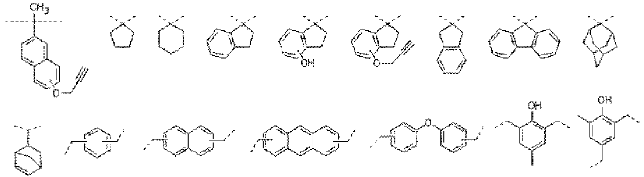

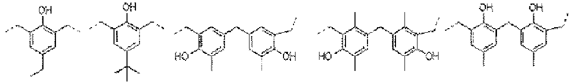

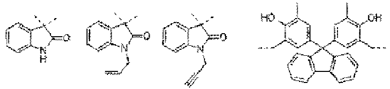

Furthermore, the linking group L^{a} of the polymer is preferably a group represented by the following general formula (10).

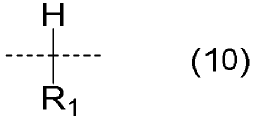

In the above general formula (10), R₁ is a hydrogen atom or an organic group containing an aromatic ring having 6 to 20 carbon atoms, and the dashed lines represent attachment points.

Specific examples of the above general formula (10) include the following, and among the following, a methylene group, that is, R₁ is preferably a hydrogen atom from the viewpoint of material availability.

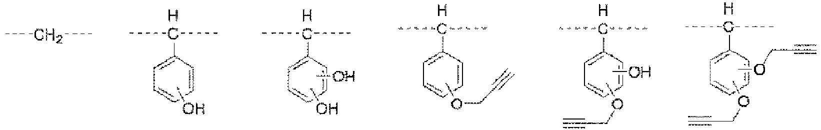

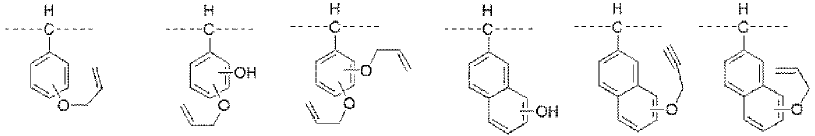

Furthermore, the weight average molecular weight (Mw) of the polymer having repeating units represented by the above general formulae (bp-4), (bp-5), and (bp-6) in terms of polystyrene according to gel permeation chromatography is preferably 1,000 to 12,000, and more preferably 2,000 to 10,000.

Within this molecular weight range, solubility in organic solvents can be ensured, and sublimates produced during baking can be reduced. In addition, the thermal flowability of the composition for forming a metal-containing film is improved, so that a composition for forming a metal-containing film having more excellent filling and planarizing properties when the composition is blended with the composition can be provided.

### (Constituent Unit: BP-4)

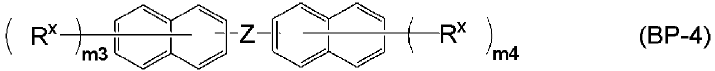

In the general formula (BP-4), "m3" and "m4" each independently represent 1 or 2; Z represents a single bond or any of structures represented by the following general formula (7); and R^{x} represents any of structures represented by the following general formula (8).

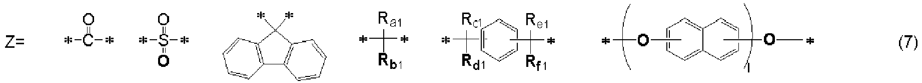

In the general formula (7), "*" represents an attachment point; "l" represents an integer of 0 to 3; Rₐ₁ to R_{f1} each independently represent a hydrogen atom, an alkyl group having 1 to 10 carbon atoms, a phenyl group, or a phenylethyl group, groups which may be substituted with fluorine; and Rₐ₁ and R_{b1} may be bonded each other to form a cyclic compound.

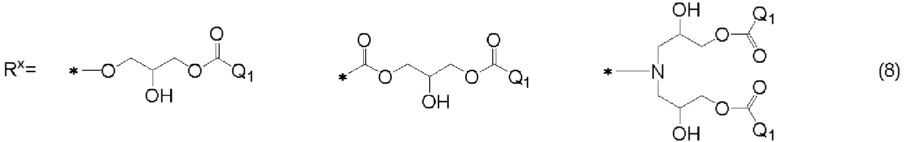

In the general formula (8), "*" represents an attachment point to an aromatic ring; Q₁ is a linear saturated hydrocarbon group having 1 to 30 carbon atoms or has a structure represented by the following general formula (9).

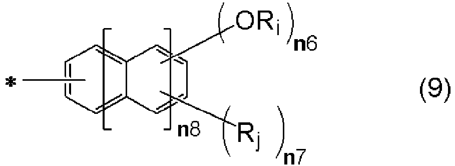

In the general formula (9), "*" represents an attachment point to a carbonyl group; Rᵢ represents a group represented by the formula (4'); Rⱼ represents a linear or branched hydrocarbon group having 1 to 10 carbon atoms, a halogen atom, a nitro group, an amino group, a nitrile group, an alkoxycarbonyl group having 2 to 10 carbon atoms, or an alkanoyloxy group having 1 to 10 carbon atoms; "n6" and "n7" each represent the number of substituents on the aromatic ring, and each represents an integer from 0 to 7, provided that n6+n7 is an integer from 0 to 7; and "n8" represents an integer from 0 to 2.

In the above general formula (BP-4), "m3" and "m4" each independently represent 1 or 2; Z is a single bond or any of the structures represented by the above general formula (7); and R^{x} each independently represents any of the structures represented by the above general formula (8).

From the viewpoints of dry etching resistance and heat resistance, in the above general formula (BP-4), Z is preferably a single bond or any of the structures represented by the following formula (4A).

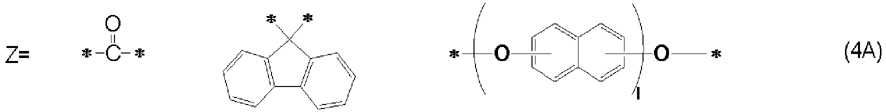

In formula (4A), "*" represents an attachment point, and "l" is the same as in the above general formula (7).

In the above general formula (8), "*" represents an attachment point to the aromatic ring and Q₁ is a linear saturated hydrocarbon group having 1 to 30 carbon atoms or a structure represented by the above general formula (9). When Q₁ represents a linear hydrocarbon group having 1 to 30 carbon atoms, a methylene group constituting Q₁ may be substituted with an oxygen atom or a carbonyl group. From the viewpoints of dry etching resistance and heat resistance, Q₁ is preferably a structure represented by the above general formula (9).

In the above general formula (9), "*" represents an attachment point to a carbonyl group; Rᵢ is a group represented by the above formula (4'); Rⱼ represents a linear or branched hydrocarbon group having 1 to 10 carbon atoms, a halogen atom, a nitro group, an amino group, a nitrile group, an alkoxycarbonyl group having 2 to 10 carbon atoms, or an alkanoyloxy group having 1 to 10 carbon atoms; "n6" and "n7" each represent the number of substituents on the aromatic ring and an integer of 0 to 7, provided n6+n7 is an integer of 0 to 7; and "n8" represents an integer of 0 to 2.

Because compounds containing the constituent unit represented by the above general formula (BP-4) have a structure in which aromatic rings are linked by a single bond or a structure represented by the general formula (7), the compounds have high carbon density. Therefore, compositions for forming a metal-containing film containing these compounds have excellent heat resistance. As shown in the above formula (7), the linking group Z can be appropriately selected from various linking groups according to the desired performance. In particular, by introducing the structure represented by the above formula (4A) as Z, heat resistance and etching resistance can be imparted without impairing film formability. In addition, because the compounds have a highly flexible terminal R^{x}, it is possible to form a thick film of a composition for forming a metal-containing film without generating defects such as cracks, despite containing a rigid aromatic ring structure. Furthermore, the terminal R^{x} contains a terminal group Q₁ that imparts thermal flowability, and to the terminal group Q₁ it is possible to introduce a flexible hydrocarbon structure contributing to improving thermal flowability, and a rigid aromatic ring structure contributing to etching resistance and heat resistance in any ratio according to the required performance. As described above, the composition for forming a metal-containing film containing these compounds can achieve both filling and planarizing properties and heat resistance at a high level and can form a thick film according to the required properties.

### (Constituent Unit: BP-5)

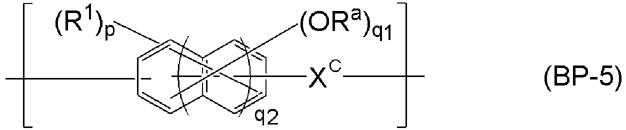

In the general formula (BP-5), R¹ represents a saturated monovalent organic group having 1 to 30 carbon atoms or an unsaturated monovalent organic group having 2 to 30 carbon atoms; X^{C} represents a divalent organic group having 1 to 30 carbon atoms; R^{a} represents a group represented by the formula (4'); "p" represents an integer from 0 to 5, "q1" represents an integer from 1 to 6, and p+q1 is an integer from 1 to 6; and "q2" represents 0 or 1.

In the above general formula (BP-5), examples of the divalent organic group having 1 to 30 carbon atoms represented by X^{C} include alkanediyl groups such as a methylene group, an ethanediyl group, a propanediyl group, a butanediyl group, a pentanediyl group, a hexanediyl group, an octanediyl group, and a decanediyl group; monocyclic cycloalkanediyl groups such as a cyclopropanediyl group, a cyclobutanediyl group, a cyclopentanediyl group, a cyclohexanediyl group, a cycloheptanediyl group, a cyclooctanediyl group, a cyclodecanediyl group, a methylcyclohexanediyl group, and an ethyl cyclohexanediyl group; polycyclic cycloalkanediyl groups such as a bicyclo[2.2.1]heptanediyl group, a bicyclo[2.2.2]octanediyl group, a tricyclo[5.2.1.0^{2,6}]decanediyl group (dicyclopentylene group), a tricyclo [3.3.1.1^{3,7}] decanediyl group, a tetracyclo[6.2.1.1^{3,6}.0^{2,7}] dodecanediyl group, and an adamantanediyl group; arenediyl groups such as a phenylene group and a naphthylene group; etc.

Examples of the alkanediyloxy group represented by X^{C} include a group formed by combining the above alkanediyl group with an oxygen atom, etc. Examples of the cycloalkanediyloxy group represented by X^{C} include a group formed by combining the above cycloalkanediyl group with an oxygen atom, etc.

Some or all of the hydrogen atoms contained in the alkanediyl groups, the cycloalkanediyl groups, the alkanediyloxy groups, the cycloalkanediyloxy groups, the arenediyl groups, etc. may be substituted, and examples of the substituent include the same groups as the examples of the substituents that the organic group represented by R^{a} may contain, etc.

Examples of the organic group represented by X^{C} include groups represented by the following formulae, etc.

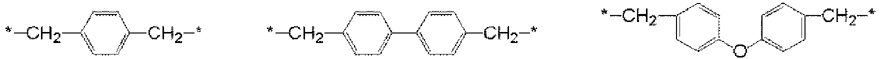

In the formula, "*" represents a bond attachment point.

From the viewpoint of material availability, a preferable example of X^{C} is a methylene group.

Specific examples of the resin having the structural unit represented by the above general formula (BP-5) include the following.

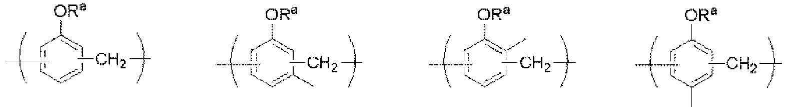

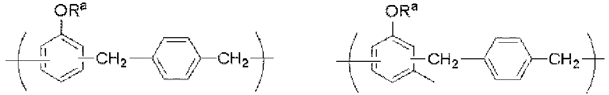

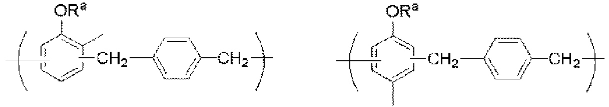

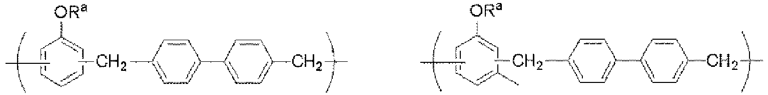

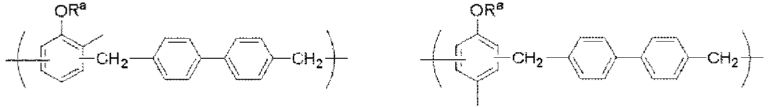

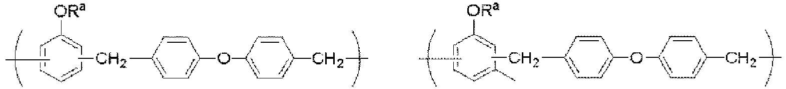

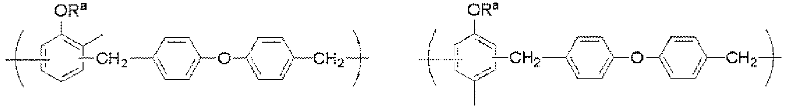

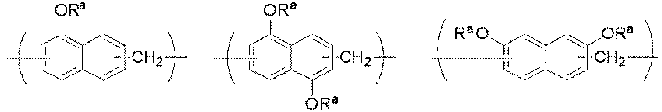

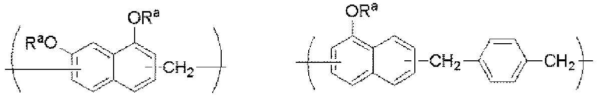

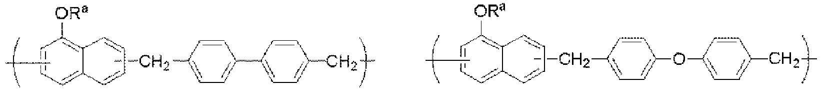

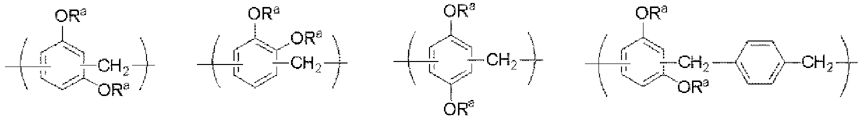

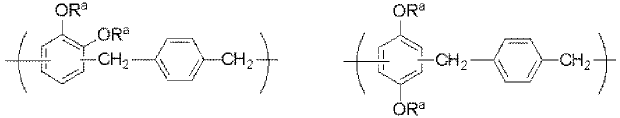

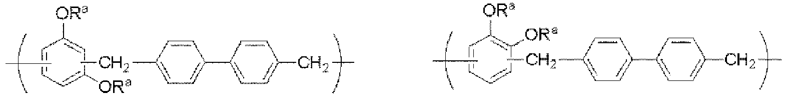

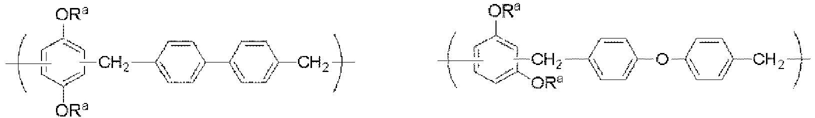

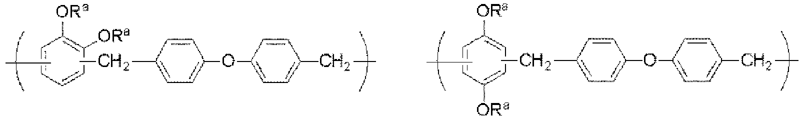

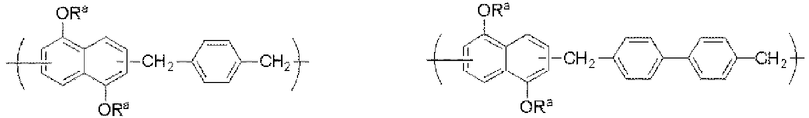

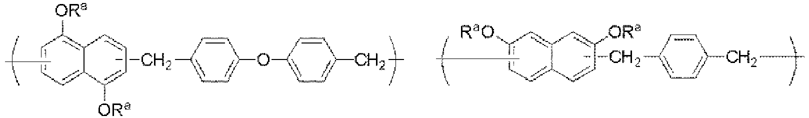

In the formulae, R^{a} is the same as defined above.

The polymer containing the constituent unit represented by the above general formula (BP-5) has a structure in which aromatic rings are linked by an organic group (X^{C}), and thus has a high carbon density. Therefore, the composition for forming a metal-containing film containing these polymers exhibits high dry etching resistance and excellent heat resistance. Furthermore, because the organic group of the structure represented by the formula (4') that contributes to the improvement of thermal flowability is directly bonded to the aromatic ring, which is the mother nucleus structure of the resin via an oxygen atom, the composition for forming a metal-containing film containing these polymers can achieve both filling and planarizing properties and heat resistance/etching resistance at a high level. In addition, because the aromatic ring structure of the mother nucleus is not too rigid and forms a repeating structure via the organic group (X^{C}) that is the linking group, it is possible to form a metal-containing film without generating defects such as cracks.

In the inventive composition for forming a metal-containing film, the flowability accelerator (BP) is preferably contained in an amount of 50 mass% or less, more preferably 30 mass% or less, and further preferably 20 mass% or less, relative to 100 parts by mass of the compound for forming a metal-containing film.

The added amount of the flowability accelerator can be adjusted to any ratio according to the required characteristics of the process using the inventive composition for forming a metal-containing film. When it is desired to minimize the deterioration of dry etching resistance, the ratio of the flowability accelerator may be reduced. When it is desired to tolerate some deterioration of dry etching resistance and improve filling and planarizing properties further, the ratio of the flowability accelerator may be increased.

### <Method for Forming Resist Underlayer Film and Filling Film>

The present invention provides a method of forming, by using the above composition for forming a metal-containing film, a resist underlayer film of a multilayer resist film used in lithography or a filling film that serves as a planarizing film for manufacturing a semiconductor.

In a method for forming a resist underlayer film by using the inventive composition for forming a metal-containing film, the substrate to be processed is coated with the above composition for forming a metal-containing film by a spin-coating method or the like. By using the spin-coating method or the like, a good filling property can be obtained. After spin-coating, baking (heating) is performed so as to evaporate the solvent and promote a crosslinking reaction to prevent mixing with the resist upper layer film and the resist middle layer film. The baking is preferably performed at a temperature of 100°C or higher and 600°C or lower for 10 to 600 seconds, and more preferably at a temperature of 200°C or higher and 500°C or lower for 10 to 300 seconds. In consideration of influences on device damage and wafer deformation, the upper limit of the heating temperature in the wafer process of lithography is preferably not more than 600°C, and more preferably not more than 500°C.

In the method for forming a resist underlayer film by using the inventive composition for forming a metal-containing film, a substrate to be processed may also be coated with the inventive composition for forming a metal-containing film by spin-coating or the like in the same manner as described above, and then the composition for forming a metal-containing film may be baked and cured under an atmosphere having an oxygen concentration of 0.1 volume% or more and 21 volume% or less to form a metal-containing film as a resist underlayer film.

By baking the inventive composition for forming a metal-containing film in such an oxygen atmosphere, a sufficiently cured film can be obtained. The atmosphere during the baking may be air, but for preventing oxidation of the metal-containing film, it is preferable to enclose an inert gas such as N₂, Ar, and He to reduce the amount of oxygen therein. Control of oxygen concentration is necessary to prevent oxidation, the oxygen concentration is preferably 1,000 ppm or less, and more preferably 100 ppm or less (volumetric basis). By preventing oxidation of the metal-containing film during the baking, the absorption does not increase, and the etching resistance does not decrease, which is preferable.

A method for forming a filling film may be the same as the above method for forming a resist underlayer film.

### <Patterning Process by Using Composition for Forming Metal-Containing Film>

Furthermore, the present invention provides a patterning process for forming a pattern in a substrate to be processed, as a patterning process according to a two-layer resist process by using the above composition for forming a metal-containing film, comprising the steps of:
(I-1) applying the composition for forming a metal-containing film according to the above onto a substrate to be processed, followed by heating to form a metal-containing film;
(I-2) forming a resist upper layer film on the metal-containing film by using a photoresist material;
(I-3) subjecting the resist upper layer film to pattern exposure and then development with a developer to form a pattern in the resist upper layer film;
(I-4) transferring the pattern to the metal-containing film by dry etching while using the resist upper layer film having the formed pattern as a mask; and
(I-5) processing the substrate to be processed while using the metal-containing film having the transferred pattern as a mask to form the pattern in the substrate to be processed.

The resist upper layer film in the two-layer resist process described above exhibits etching resistance with respect to chlorine-based gas. Therefore, the dry etching of the metal-containing film that is performed while using the resist upper layer film as a mask in the two-layer resist process is preferably performed using an etching gas mainly containing a chlorine-based gas.

Further, the present invention provides a patterning process for forming a pattern in a substrate to be processed, as a patterning process according to a three-layer resist process by using such a composition for forming a metal-containing film, comprising the steps of:
(II-1) applying the composition for forming a metal-containing film according to the above onto a substrate to be processed, followed by heating to form a metal-containing film;
(II-2) forming a silicon-containing resist middle layer film on the metal-containing film;
(II-3) forming a resist upper layer film on the silicon-containing resist middle layer film by using a photoresist material;
(II-4) subjecting the resist upper layer film to pattern exposure and then development with a developer to form a pattern in the resist upper layer film;
(II-5) transferring the pattern to the silicon-containing resist middle layer film by dry etching while using the resist upper layer film having the formed pattern as a mask;
(II-6) transferring the pattern to the metal-containing film by dry etching while using the silicon-containing resist middle layer film having the transferred pattern as a mask; and
(II-7) processing the substrate to be processed while using the metal-containing film having the transferred pattern as a mask to form the pattern in the substrate to be processed.

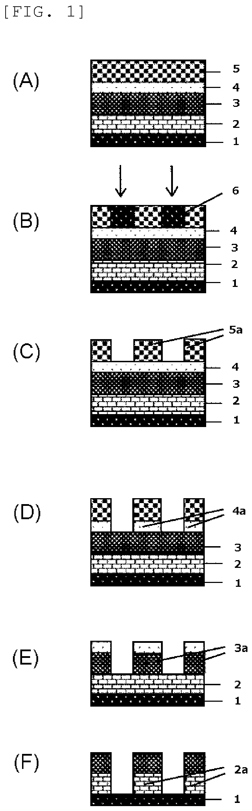

Here, with reference to FIG. 1, an example of a patterning process according to the three-layer resist process will be described. The present invention, as a patterning process according to the three-layer resist process with such a composition for forming a metal-containing film, provides a patterning process as follows. As in FIG. 1(A), a metal-containing film 3 is formed on a layer 2 to be processed on a substrate 1 to be processed by using the composition for forming a metal-containing film; a silicon-containing resist middle layer film 4 is formed on the metal-containing film by using a silicon-containing resist middle layer film composition; a resist upper layer film 5 is formed on the silicon-containing resist middle layer film by using a photoresist material. Subsequently, as in FIG. 1(B), an exposure portion 6 of the resist upper layer film is subjected to pattern exposure. Then, development is performed with a developer to form a resist upper layer film pattern 5a in the resist upper layer film as in FIG. 1(C). Next, the silicon-containing resist middle layer film pattern 4a is transferred to the silicon-containing resist middle layer film by dry etching while using the resist upper layer film having the formed pattern as a mask as in FIG. 1(D). Subsequently, the metal-containing film pattern 3a is transferred to the metal-containing film by dry etching while using the silicon-containing resist middle layer film having the transferred pattern as a mask as in FIG. 1(E). Then, the layer 2 to be processed on the substrate 1 to be processed is processed while using the metal-containing film having the formed pattern as a mask to form a pattern 2a in the substrate 1 to be processed as in FIG. 1(F).

The silicon-containing resist middle layer film in the three-layer resist process exhibits etching resistance with respect to a chlorine-based gas. Therefore, the dry etching of the metal-containing film that is performed while using the silicon-containing resist middle layer film as a mask in the three-layer resist process is preferably performed using an etching gas mainly containing a chlorine-based gas.

As the silicon-containing resist middle layer film in the three-layer resist process, a polysiloxane-based resist middle layer film is also favorably used. By giving an antireflective effect to the silicon-containing resist middle layer film, it is possible to suppress reflection. When a material containing many aromatic groups and having a high etching selectivity with respect to the substrate is used as the organic film especially for 193-nm exposure, the k-value increases and thus the substrate reflection increases; however, the reflection can be suppressed by imparting absorption so that the silicon-containing resist middle layer film has an appropriate k-value. In this manner, the substrate reflection can be reduced to 0.5% or less. Preferably used as the silicon-containing resist middle layer film having an antireflective effect is a polysiloxane, which has a pendant anthracene for exposure at 248 nm or 157 nm, or a pendant phenyl group or a pendant light-absorbing group having a silicon-silicon bond for exposure at 193 nm, and which is crosslinked by an acid or heat.

In addition, the present invention provides a patterning process according to a four-layer resist process using such a composition for forming a metal-containing film, the patterning process including the steps of:
forming a metal-containing film on a substrate to be processed by using the above-described composition for forming a metal-containing film;
forming a silicon-containing resist middle layer film on the metal-containing film by using a silicon-containing resist middle layer film material;
forming an organic antireflective film (BARC) or an adhesive film on the silicon-containing resist middle layer film;
forming a resist upper layer film on the BARC or the adhesive film by using a photoresist material;
subjecting the resist upper layer film to pattern exposure and then development with a developer, thereby forming a pattern in the resist upper layer film;
transferring the pattern to the BARC or the adhesive film and the silicon-containing resist middle layer film by dry etching while using the resist upper layer film having the formed pattern as a mask;
transferring the pattern to the metal-containing film by dry etching while using the silicon-containing resist middle layer film having the transferred pattern as a mask; and
processing the substrate to be processed while using the metal-containing film having the transferred pattern as a mask, thereby forming the pattern in the substrate to be processed.

Alternatively, an inorganic hard mask middle layer film may be formed instead of the silicon-containing resist middle layer film. In this case, a semiconductor device circuit pattern can be formed in a substrate, at least, by:
forming a metal-containing film on a substrate to be processed by using the inventive composition for forming a metal-containing film;
forming an inorganic hard mask middle layer film selected from the group consisting of a silicon oxide film, a silicon nitride film, and a silicon oxynitride film on the metal-containing film;
forming a resist upper layer film on the inorganic hard mask middle layer film by using a photoresist composition, and then forming a circuit pattern in the resist upper layer film;
transferring the pattern to the inorganic hard mask middle layer film while using the resist upper layer film having the formed pattern as a mask;
transferring the pattern to the metal-containing film while using the inorganic hard mask middle layer film having the formed pattern as a mask; and
furthermore, processing the substrate to be processed while using the metal-containing film having the transferred pattern as a mask to form the pattern in the substrate to be processed.

The present invention provides a patterning process for forming a pattern in a substrate to be processed, as a patterning process according to a four-layer resist process using such a composition for forming a metal-containing film, comprising the steps of:
(III-1) applying the composition for forming a metal-containing film according to the above onto a substrate to be processed, followed by heating to form a metal-containing film;
(III-2) forming an inorganic hard mask middle layer film selected from the group consisting of a silicon oxide film, a silicon nitride film and a silicon oxynitride film on the metal-containing film;
(III-3) forming an organic thin film on the inorganic hard mask middle layer film;
(III-4) forming a resist upper layer film on the organic thin film by using a photoresist material;
(III-5) subjecting the resist upper layer film to pattern exposure and then development with a developer to form a pattern in the resist upper layer film;
(III-6) transferring the pattern to the organic thin film and the inorganic hard mask middle layer film by dry etching while using the resist upper layer film having the formed pattern as a mask;
(III-7) transferring the pattern to the metal-containing film by dry etching while using the inorganic hard mask middle layer film having the transferred pattern as a mask; and
(III-8) processing the substrate to be processed while using the metal-containing film having the transferred pattern as a mask to form the pattern in the substrate to be processed.

As described above, when forming an inorganic hard mask middle layer film on a metal-containing film, a silicon oxide film, a silicon nitride film, and a silicon oxynitride film (SiON film) can be formed by a CVD method, an ALD method, or the like. That is, the inorganic hard mask middle layer film is preferably formed by a CVD method or an ALD method. For example, methods for forming a silicon nitride film are described in JP2002-334869A and WO2004/066377A1. The thickness of the inorganic hard mask middle layer film is preferably 5 to 200 nm, and more preferably 10 to 100 nm. As the inorganic hard mask middle layer film, a SiON film, which has a high effect as an anti-reflective film, is most preferably used. Because the substrate temperature is 300 to 500°C in forming of the SiON film, the metal-containing film needs to withstand temperatures of 300 to 500°C. The composition for forming a metal-containing film used in the present invention has high heat resistance and can withstand high temperatures of 300°C to 500°C. Therefore, it is possible to combine an inorganic hard mask middle layer film formed by a CVD method or an ALD method and a metal-containing film formed by a spin coating method.

A photoresist film may be formed as the resist upper layer film on the inorganic hard mask middle layer film as described above. Alternatively, an organic antireflective film (BARC) or an adhesive film may be formed on the inorganic hard mask by spin-coating, and a photoresist film may be formed thereon. In particular, when a SiON film is used as the inorganic hard mask middle layer film, the reflection can be suppressed by the two antireflective films, i.e., the SiON film and the BARC film, even in liquid immersion exposure at a high NA exceeding 1.0. Another merit of forming BARC is that it has an effect to reduce trailing of a photoresist pattern immediately above the SiON film.

In the above patterning process, the resist upper layer film may be either a positive type or a negative type, and it is possible to use a composition similar to the typically used photoresist composition. Further, the photoresist composition may also contain metal atoms such as Sn, In, Ga, Ge, Al, Ce, La, Cs, Zr, Hf, Ti, Bi, Sb, and Zn. In the case where the resist upper layer film is formed with the above photoresist composition, a spin coating method, and a forming method by vapor deposition treatment using a CVD method or an ALD method may be used.

In the case where the resist upper layer film is formed by a spin-coating method, prebaking is conducted after applying the photoresist composition. The prebaking is preferably performed at 60 to 180°C for 10 to 300 seconds. Thereafter, exposure is conducted according to the ordinary method, followed by post-exposure baking (PEB) and development, and thereby, a resist pattern is obtained. Although the thickness of the resist upper layer film is not particularly limited, the thickness is preferably 10 to 500 nm, particularly preferably 20 to 400 nm.

When the resist upper layer film is formed by vapor deposition treatment using a CVD method or an ALD method, the photoresist composition is an EUV photosensitive metal oxide-containing film, and the metal is selected from Sn, Zr, Hf, Ti, Bi, Sb, etc. Among them, Sn, which has excellent EUV photosensitivity, is preferable. The metal oxide-containing film may be a photosensitive organometallic oxide film such as an organotin oxide (e.g., haloalkyl Sn, alkoxyalkyl Sn, or amidoalkyl Sn). Some specific examples of suitable precursors include trimethyltin chloride, dimethyltin dichloride, methyltin trichloride, tris(dimethylamino)methyltin(IV), and (dimethylamino)trimethyltin(IV).

Metal oxide containing films may be vapor-deposited by a PECVD method or a PEALD method, for example using a tool Lam Vector(registered trademark). In an ALD embodiment the Sn oxide precursor is separated from the O precursor/plasma. Vapor Deposition temperature is preferably in the range of 50°C to 600°C. Vapor Deposition pressure is preferably between 100 and 6,000 mTorr. A flow rates of the precursor liquid of metal oxide-containing film (e.g., organotin oxide precursors) may be 0.01-10 cmm, and a gas flow rate (CO₂, CO, Ar, N₂) may be 100-10,000 sccm. Plasma power may be 200-1,000 W per 300 mm wafer station using high frequency plasma (e.g., 13.56 MHz, 27.1 MHz, or higher). Vapor deposition thickness is preferably 100-2,000 Å.

Examples of exposure light include high-energy beams having a wavelengths of 300 nm or less. Specific examples include excimer laser beams at 248 nm, 193 nm, and 157 nm, soft X-rays at 3 to 20 nm, an electron beam, and X-rays.

As the method for forming a pattern in the resist upper layer film, it is preferable to use a patterning process using a photolithography with a wavelength of 5 nm or more and 300 nm or less, a direct drawing using an electron beam, nanoimprinting, or a combination thereof.

The development method in the patterning process is preferably alkali development or development using an organic solvent.

Next, etching is performed while using the obtained resist pattern as a mask. The etching of the silicon-containing resist middle layer film or the inorganic hard mask middle layer film in the three-layer resist process is performed while using the resist upper layer film pattern as a mask by using a fluorocarbon-based gas. In this manner, a silicon-containing resist middle layer film pattern and an inorganic hard mask middle layer film pattern is formed.

Next, the metal-containing film is etched while using the obtained silicon-containing resist middle layer film pattern or the obtained inorganic hard mask middle layer film pattern as a mask. The etching of the metal-containing film is preferably performed using an etching gas mainly containing a chlorine-based gas.

The subsequent etching of the substrate to be processed may also be performed according to the ordinary method. For example, in the case where a substrate to be processed is made of SiO₂, SiN or silica-based low dielectric constant insulating film, the etching is performed using an etching gas mainly containing a fluorocarbon-based gas. When the substrate is etched with a fluorocarbon-based gas, the silicon-containing resist middle layer film pattern in the three-layer resist process is stripped simultaneously with the substrate processing.

The metal-containing film obtained by using the inventive composition for forming a metal-containing film is characterized by its excellent etching resistance at the time of etching of these substrates to be processed.

Incidentally, examples of the body to be processed (substrate to be processed) include, but are not particularly limited to, substrates made of Si, α-Si, p-Si, SiO₂, SiN, SiON, W, TiN, Al, etc., and those in which a layer to be processed are formed on the substrate. Examples of the layers to be processed include various low-k films such as those made of Si, SiO₂, SiON, SiN, p-Si, α-Si, W, W-Si, Al, Cu, Al-Si, and stopper films therefor, which can be typically formed in a thickness of 50 to 10,000 nm, particularly 100 to 5,000 nm. When a film of the layer to be processed is formed, the substrate and the layer to be processed are made of different materials.

In the patterning process by using the inventive composition for forming a metal-containing film, it is preferable to use a substrate to be processed having a structure or step having a height of 30 nm or more. As described above, the inventive composition for forming a metal-containing film has excellent filling and planarizing properties, so that a flat cured film can be formed even when the substrate to be processed has a structure or step (asperities) having a height of 30 nm or more. The height of the structure or step of the above substrate to be processed is preferably 30 nm or more, more preferably 50 nm or more, and further preferably 100 nm or more. In the method of processing a stepped substrate having a pattern of the above-described height, filling and planarizing by forming a film of the inventive composition for forming a metal-containing film makes it possible to achieve a uniform film thickness of a resist middle layer film and a resist upper layer film, which is formed in the subsequent process. Therefore, it is easy to ensure the exposure depth margin (DOF) at the time of photolithography, which is very desirable.

Further, the present invention provides a patterning process, as a patterning process according to a four-layer resist process using such a composition for forming a metal-containing film, comprising the steps of:
forming a metal-containing film on a substrate to be processed by using the above composition for forming a metal-containing film;
forming a resist underlayer film on the metal-containing film by using an organic resist underlayer film material;
forming a silicon-containing resist middle layer film on the resist underlayer film by using a silicon-containing resist middle layer film material;
forming an organic antireflective film (BARC) or an adhesive film on the silicon-containing resist middle layer film as necessary;
forming a resist upper layer film on the silicon-containing film, the BARC, or the adhesive film by using a photoresist material;
subjecting the resist upper layer film to pattern exposure and then development with a developer, thereby forming a pattern in the resist upper layer film;
transferring the pattern to the BARC or the adhesive film and the silicon-containing resist middle layer film by dry etching while using the resist upper layer film having the formed pattern as a mask;
transferring the pattern to the resist underlayer film by dry etching while using the silicon-containing resist middle layer film having the transferred pattern as a mask;
transferring the pattern to the metal-containing film while using the resist underlayer film having the transferred pattern as a mask; and
processing the substrate to be processed while using the metal-containing film having the transferred pattern as a mask, thereby forming the pattern in the substrate to be processed.

As an organic resist underlayer film material that can be used for the resist underlayer film, it is possible to use: materials already known as materials for three-layer resist methods or materials for an underlayer film containing a silicon resist composition for two-layer resist methods; materials known as resist underlayer film materials for two-layer resist methods and three-layer resist methods, the materials including various resins, such as a 4,4'-(9-fluorenylidene)bisphenol novolak resin (molecular weight: 11,000) disclosed in JP2005-128509A, and also other novolak resins; etc. Furthermore, if it is desired for the resin to have a higher heat resistance than ordinary novolaks, a polycyclic skeleton can be introduced, as in a 6,6'-(9-fluorenylidene)-di(2-naphthol) novolak resin, and furthermore, a polyimide-based resin can also be selected (e.g., JP2004-153125A).

The resist underlayer film can be formed on a substrate to be processed by spin coating or the like using a composition solution in the same manner as the photoresist composition. After forming the organic underlayer film by spin coating or the like, it is desirable to bake the film to evaporate the organic solvent. The baking temperature is preferably in the range of 80 to 400°C, and the baking time is preferably in the range of 10 to 300 seconds.

Instead of the above resist underlayer film, an organic hard mask formed by the CVD method or the ALD method can also be used.

Since the resist underlayer film in the above multilayer resist process exhibits etching resistance with respect to chlorine-based gas, it is preferable that in the above multilayer resist process, dry etching that a metal-containing film undergoes while using the resist underlayer film as a mask is preferably performed using an etching gas mainly containing a chlorine-based gas.

Further, the present invention provides a patterning process for forming a pattern in a substrate to be processed, as a patterning process according to a four-layer resist process using such a composition for forming a metal-containing film, comprising the steps of:
(IV-1) applying the composition for forming a metal-containing film according to the above onto a substrate to be processed, followed by heating to form a metal-containing film;
(IV-2) forming a resist underlayer film on the metal-containing film;
(IV-3) forming a silicon-containing resist middle layer film or a combination of an inorganic hard mask middle layer film selected from the group consisting of a silicon oxide film, a silicon nitride film, and a silicon oxynitride film and an organic thin film on the resist underlayer film;
(IV-4) forming a resist upper layer film on the silicon-containing resist middle layer film or the organic thin film by using a photoresist material;
(IV-5) subjecting the resist upper layer film to pattern exposure and then development with a developer to form a pattern in the resist upper layer film;
(IV-6) transferring the pattern to the silicon-containing resist middle layer film, or the organic thin film and the inorganic hard mask middle layer film by dry etching while using the resist upper layer film having the formed pattern as a mask;
(IV-7) transferring the pattern to the resist underlayer film by dry etching while using the silicon-containing resist middle layer film having the transferred pattern or the inorganic hard mask middle layer film having the transferred pattern as a mask;
(IV-8) transferring the pattern to the metal-containing film by dry etching while using the resist underlayer film having the transferred pattern as a mask; and
(IV-9) processing the substrate to be processed while using the metal-containing film having the transferred pattern as a mask to form the pattern in the substrate to be processed.

### <Tone-Reversal Patterning Process Using Composition for Forming Metal-Containing Film>

Furthermore, the present invention provides a tone-reversal patterning process for forming a pattern in a substrate to be processed, as a patterning process according to a tone-reversal patterning process using such a composition for forming a metal-containing film, comprising the steps of:
(V-1) forming a resist underlayer film on a substrate to be processed;
(V-2) forming a resist middle layer film or a combination of an inorganic hard mask middle layer film selected from the group consisting of a silicon oxide film, a silicon nitride film, and a silicon oxynitride film and an organic thin film on the resist underlayer film;
(V-3) forming a resist upper layer film on the resist middle layer film or the combination of the inorganic hard mask middle layer film and the organic thin film by using a photoresist material;
(V-4) subjecting the resist upper layer film to pattern exposure and then development with a developer to form a pattern in the resist upper layer film;
(V-5) transferring the pattern to the resist middle layer film, or the organic thin film and the inorganic hard mask middle layer film by dry etching while using the resist upper layer film having the formed pattern as a mask;
(V-6) transferring the pattern to the resist underlayer film by dry etching while using the resist middle layer film having the transferred pattern, or the inorganic hard mask middle layer film having the transferred pattern as a mask;
(V-7) applying the composition for forming a metal-containing film according to the above onto the resist underlayer film having the transferred pattern, followed by heating to cover the resist underlayer film with a metal-containing film, thereby filling a space between the resist underlayer film patterns with the metal-containing film;
(V-8) etching back the metal-containing film covering the resist underlayer film having the transferred pattern by a chemical stripper or dry etching to expose an upper surface of the resist underlayer film having the formed pattern;
(V-9) removing the resist middle layer film or the inorganic hard mask middle layer film remaining on the upper surface of the resist underlayer film by dry etching;
(V-10) removing the resist underlayer film having the transformed pattern with its surface exposed by dry etching to form a reverse pattern of an original pattern in the metal-containing film; and
(V-11) processing the substrate to be processed while using the metal-containing film having the formed reverse pattern as a mask to form the reverse pattern in the substrate to be processed.

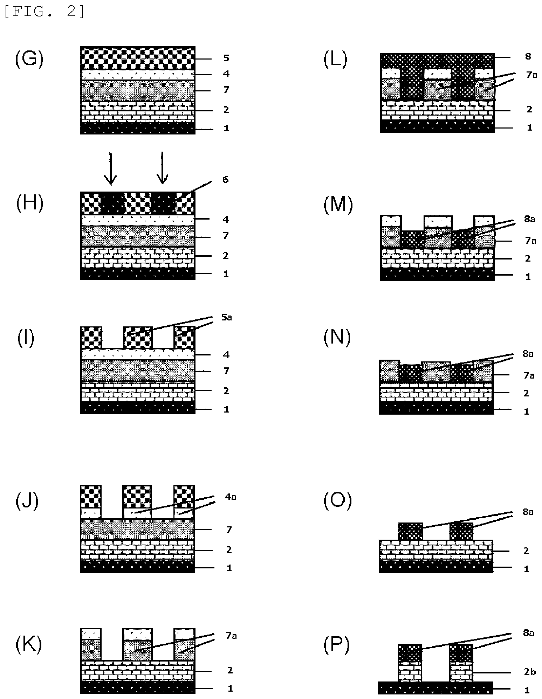

With reference to FIG. 2, a tone-reversal patterning process by using a composition for forming a metal-containing film will be described. The present invention provides a tone-reversal patterning process by using such a composition for forming a metal-containing film, as follows. As in FIG. 2(G), a resist underlayer film 7 is formed on a layer 2 to be processed stacked on a substrate 1 to be processed; thereafter, a resist middle layer film 4 or a combination of an inorganic hard mask middle layer film selected from the group consisting of a silicon oxide film, a silicon nitride film, and a silicon oxynitride film and an organic thin film is formed on the resist underlayer film 7; and a resist upper layer film 5 is formed on the resist middle layer film 4 or the combination of the inorganic hard mask middle layer film and the organic thin film by using a photoresist material. Subsequently, after subjecting an exposed portion 6 of the resist underlayer film to pattern exposure as in FIG. 2(H), the resist upper layer film are developed with a developer as in FIG. 2(I) to form a resist upper layer film pattern 5a in the resist upper layer film; a resist middle layer film pattern 4a or an inorganic hard mask middle layer film pattern is transferred to the resist middle layer film, or the organic thin film and the inorganic hard mask middle layer film by dry etching while using the resist upper layer film having the formed pattern as a mask as in FIG. 2(J); a resist underlayer film pattern 7a was transferred to the resist underlayer film by dry etching while using the resist middle layer film having the transferred pattern or the inorganic hard mask middle layer film having the transferred pattern as a mask as in FIG. 2(K); the resist underlayer film having the formed pattern is covered with a metal-containing film 8 by using the above composition for forming a metal-containing film, thereby a space between the resist underlayer film patterns 7a was filled with the metal-containing film 8 as in FIG. 2(L); the metal-containing film covering the resist underlayer film having the formed pattern was etched back by a chemical stripper or dry etching to form a reverse pattern 8a of the metal-containing film and expose an upper surface of the resist underlayer film having the formed pattern as in FIG. 2(M); the resist middle layer film or the hard mask middle layer film remaining on the upper surface of the resist underlayer film pattern 7a was removed by dry etching as in FIG. 2(N); the resist underlayer film having the formed pattern with its surface exposed was removed by dry etching to form a reverse pattern of an original pattern in the metal-containing film as in FIG. 2(O); and the substrate 2 to be processed is processed while using the metal-containing film having the formed reverse pattern as a mask to form the reverse pattern 2b in the substrate 2 to be processed as in FIG. 2(P).

As described above, when the resist underlayer film is formed on the substrate to be processed, the resist underlayer film can be formed by a method using a coating-type resist underlayer film material, a CVD method, an ALD method, etc. Examples of the coating-type resist underlayer film material include resins and compositions disclosed in JP2012-001687A, JP2012-077295A, JP2004-264710A, JP2005-043471A, JP2005-250434A, JP2007-293294A, JP2008-065303A, JP2004-205685A, JP2007-171895A, JP2009-014816A, JP2007-199653A, JP2008-274250A, JP2010-122656A, JP2012-214720A, JP2014-029435A, WO2012/077640A1, WO2010/147155A1, WO2012/176767A1, JP2005-128509A, JP2006-259249A, JP2006-259482A, JP2006-293298A, JP2007-316282A, JP2012-145897A, JP2017-119671A, JP2019-044022A, etc.

In the above tone-reversal patterning process, it is preferable to coat the obtained resist underlayer film pattern with a composition for forming a metal-containing film, and then remove the metal-containing film using a dry etching gas mainly composed of a chlorine-based gas to expose the upper surface of the resist underlayer film pattern. Then, the resist middle layer film or the inorganic hard mask middle layer film remaining on the resist underlayer film is removed by dry etching with a fluorocarbon-based gas, and the resist underlayer film pattern with the exposed surface is removed by dry etching with an oxygen-based gas to form a metal-containing film reversal pattern.

In the tone-reversal patterning process, the resist underlayer film pattern preferably has a structure or step having a height of 30 nm or more. As described above, the inventive composition for forming a metal-containing film has excellent filling and planarizing properties, so that even if the film to be processed has a structure or step (asperities) having a height of 30 nm or more, a flat cured film can be formed. The height of the structure or step of the resist underlayer film pattern is preferably 30 nm or more, more preferably 50 nm or more, and further preferably 100 nm or more. In the method for reversing a resist underlayer film pattern having the above height, a film of the inventive composition for forming a metal-containing film is formed and used for filling and planarizing, enabling the reversal/transfer of the pattern with high precision, which is very desirable. When compared to resist underlayer films using conventional coating-type resist underlayer film materials, the inventive composition for forming a metal-containing film has excellent dry etching resistance in using fluorocarbon-based gases, so that by reversing the resist underlayer film pattern with the composition for forming a metal-containing film a desired resist pattern can be formed on a film to be processed with high precision.

### EXAMPLE

Hereinafter, the present invention will be specifically described by showing Synthesis Example, Comparative Synthesis Example, Examples, and Comparative Examples. However, the present invention is not limited thereto.

### [Synthesis Example]

In the following Synthesis Examples and Comparative Synthesis Example, the following organic group starting material group G ((G1) to (G24)) and the following silicon-containing organic group starting material group H ((H1) and (H2)) were used.

The organic group starting material group G ((G1) to (G24)) are shown below.

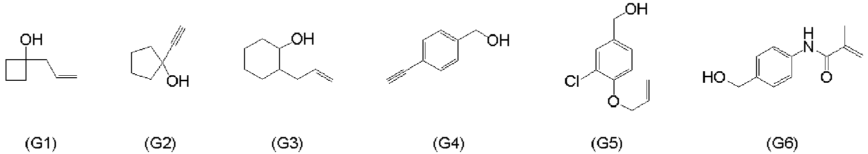

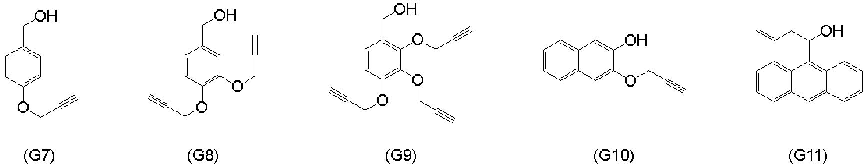

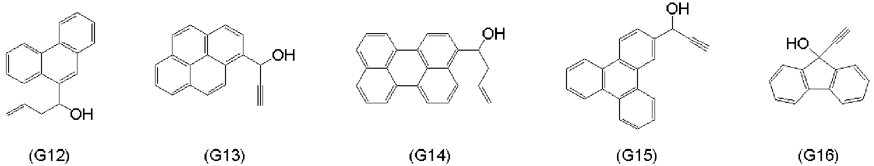

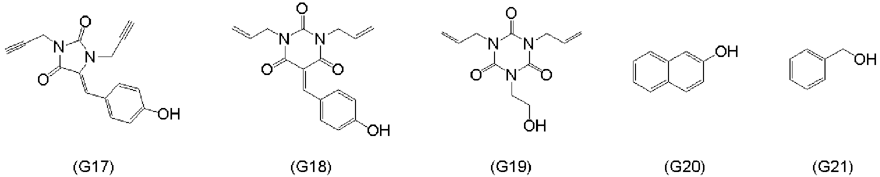

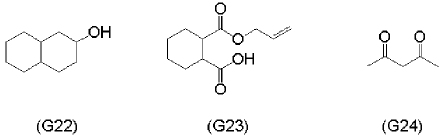

The Silicon-containing organic group starting material group H ((H1) to (H2)) are shown below.

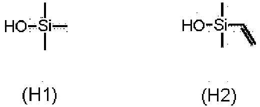

As a metal source M, the following metal compounds were used.
(M1): Zr(OBu)₄: Zirconium(IV) tetrabutoxide (80 mass% solution in 1-butanol) (Z0016, available from Tokyo Chemical Industry Co., Ltd.)
(M2): Hf(OBu)₄: hafnium(IV) n-butoxide (6 67 94 3, available from Sigma-Aldrich Corp)
(M3): Ti(OBu)₄: tetrabutyl orthotitanate (B0742, available from Tokyo Chemical Industry Co., Ltd.)

### [Synthesis Example 1] Synthesis of Compound (A-1) for Forming Metal-Containing Film

Under a nitrogen atmosphere, 10.0 g of 1-butanol and 5.6 g of the organic group starting material G1 were added to 12.0 g of Zirconium(IV) tetrabutoxide (M1) (80 mass% solution in 1-butanol) and the mixture was agitated at room temperature for 30 minutes. After the solution was concentrated under reduced pressure at 30°C, it was further heated to 60°C, and heating was continued under reduced pressure until no more distillates came out. When distillates were no longer observed, the solution was added with 120.0 g of a PGMEA/PGME (ratio by mass: 70/30) solution and 4.5 g of silicon-containing organic group starting material H1 and agitated continuously for another 1 hour while keeping a reaction temperature of 60°C. After cooling down to room temperature, the obtained reaction solution was filtered through a 0.45-um PTFE filter. Thus, a PGMEA/PGME solution of a compound (A-1) for forming a metal-containing film was obtained. The concentration of components other than the solvent in the solution was 22.0 mass%.

### [Synthesis Examples 2 to 19 and Comparative Synthesis Examples 1 to 5] Synthesis of Compounds (A-2) to (A-19) for Forming Metal-Containing Film and Comparative Compounds (R-1) to (R-5)

The compounds (A-2) to (A-19) and the comparative compounds (R-1) to (R-5) shown in Tables 1 and 2 were obtained under the same reaction conditions as in Synthesis Example 1, except that the metal source M, the organic group starting material group G, and the silicon-containing organic group starting material group H were used at the charging amounts shown in Tables 1 and 2.

**[Table 1]**

| Synthesis Example | Metal source | | Organic group starting material group G | | Silicon-containing organic group starting material group H | | Compound for forming metal-containing film |
|---|---|---|---|---|---|---|---|
| | Starting material | 9 | Starting material | 9 | Starting material | 9 | |
| 1 | M1 | 12.0 | G1 | 5.6 | H1 | 4.5 | A-1 |
| 2 | M2 | 11.8 | G2 | 5.5 | H1 | 4.5 | A-2 |
| 3 | M3 | 8.5 | G3 | 7.0 | H1 | 4.5 | A-3 |
| 4 | M1 | 12.0 | G4 | 6.6 | H2 | 5.1 | A-4 |
| 5 | M1 | 12.0 | G5 | 9.9 | H2 | 5.1 | A-5 |
| 6 | M1 | 12.0 | G6 | 9.6 | H2 | 5.1 | A- 6 |
| 7 | M1 | 12.0 | G7 | 8.1 | H1 | 4.5 | A-7 |
| 8 | M1 | 12.0 | G8 | 10.8 | H1 | 4.5 | A-8 |
| 9 | M1 | 12.0 | G9 | 13.5 | H1 | 4.5 | A-9 |
| 10 | M1 | 12.0 | G10 | 9.9 | H2 | 5.1 | A-10 |
| 11 | M1 | 12.0 | G11 | 12.4 | - | - | A-11 |
| 12 | M1 | 12.0 | G12 | 12.4 | H1 | 4.5 | A-12 |
| 13 | M1 | 12.0 | G13 | 12.8 | H1 | 4.5 | A-13 |
| 14 | M1 | 12.0 | G14 | 16.1 | H1 | 4.5 | A-14 |
| 15 | M1 | 12.0 | G15 | 14.1 | - | - | A-15 |
| 16 | M1 | 12.0 | G16 | 10.3 | - | - | A-16 |
| 17 | M1 | 12.0 | G17 | 14.0 | H2 | 5.1 | A-17 |
| 18 | M1 | 12.0 | G18 | 15.6 | H1 | 4.5 | A-18 |
| 19 | M1 | 12.0 | G19 | 12.7 | H1 | 4.5 | A-19 |

**[Table 2]**

| Comparative Synthesis Examples | Metal source | | Organic group starting material group G | | Silicon-containing organic group starting material group H | | Comparative compound |
|---|---|---|---|---|---|---|---|
| | Starting material | 9 | Starting material | 9 | Starting material | 9 | |
| 1 | M1 | 12.0 | G20 | 7.2 | H1 | 4.5 | R-1 |
| 2 | M1 | 12.0 | G21 | 5.4 | H1 | 4.5 | R-2 |
| 3 | M1 | 12.0 | G22 | 7.7 | H1 | 4.5 | R-3 |
| 4 | M1 | 12.0 | G23 | 10.6 | - | - | R-4 |
| 5 | M1 | 12.0 | G24 | 5.0 | H1 | 4.5 | R-5 |

### [Synthesis Example 20] Synthesis of Compound (A-20) for Forming Metal-Containing Film

Under a nitrogen atmosphere, 20.5 g of an n-butanol solution containing 24.0 g of Zirconium(IV) tetrabutoxide (M1) was added with 27.5 g of an n-butanol solution of 0.68 g of deionized water dropwise at room temperature over 2 hours with keeping agitation of the solution. To the obtained solution, 9.6 g of the organic group starting material group (G5) was added, and the mixture was agitated at room temperature for 30 minutes. After the solution was concentrated under reduced pressure at 30°C, it was further heated to 60°C and heating was continued under reduced pressure to generate no distillate. When distillates were no longer observed, 45.0 g of a PGMEA/PGME (ratio by mass: 70/30) solution was added thereto and heated under reduced pressure at 40°C. Thus, a PGMEA/PGME solution of a compound (A-20) for forming a metal-containing film was obtained. The concentration of components other than the solvent in the solution was 19 mass%.

### [Synthesis Examples 21 to 22] Synthesis of Compounds (A-21) to (A-22) for Forming Metal-Containing Film

The compounds (A-21) to (A-22) for forming a metal-containing film shown in Table 3 were obtained under the same reaction conditions as in Synthesis Example 20, except that the metal source M, and the organic group starting material group G were used at the charging amounts shown in Table 3.

**[Table 3]**

| Synthesis Example | Metal source | | Organic group starting material group G | | Compound for forming metal-containing film |
|---|---|---|---|---|---|
| | Starting material | g | Starting material | g | |
| 20 | M1 | 24.0 | G5 | 9.6 | A-20 |
| 21 | M2 | 23.5 | G5 | 9.6 | A-21 |
| 22 | M3 | 17.0 | G5 | 9.6 | A-22 |

### [Comparative Synthesis Example 6] Synthesis of Comparative Compound (R-6)

The titanium compound disclosed in [Synthesis Example A-II] of JP6189758B2 was synthesized. 500 g of an IPA solution containing 284 g of titanium tetraisopropoxide (manufactured by Tokyo Chemical Industry Co., Ltd.) was added with 500 g of an IPA solution of 27 g of deionized water dropwise at room temperature over 2 hours with keeping agitation of the solution. To the obtained solution, 120 g of 2-methyl-2,4-pentanediol was added, and the mixture was agitated at room temperature for 30 minutes. After the solution was concentrated under reduced pressure at 30°C, it was further heated to 60°C and heating was continued under reduced pressure to generate no distillate. When distillates were no longer observed, 1,200 g of PGMEA was added thereto and heated under reduced pressure at 40°C to generate no distillate of IPA. Thus, 1,000 g of a PGMEA solution of a titanium-containing compound (R-6) (compound concentration 20 mass%) was obtained.

### [Comparative Synthesis Example 7] Synthesis of Comparative Compound (R-7)

Under a nitrogen atmosphere, 160.2 g of 1,5-dihydroxynaphthalene, 56.8 g of formaldehyde, and 300 g of PGME (propylene glycol monomethyl ether) were added and homogenized at an internal temperature of 100°C. After that, a mixed solution of 8.0 g of p-toluenesulfonic acid monohydrate and 8.0 g of PGME, the solution which had been mixed and homogenized beforehand, was added dropwise slowly, and a reaction was allowed to take place at an internal temperature of 80°C for 8 hours. After the reaction was completed, the product was cooled to room temperature, and 2,000 ml of MIBK was added thereto. The product was washed six times with 500 ml of pure water, and the organic layer was dried under reduced pressure. After adding 300 g of THF to the residue to yield a homogeneous solution, a crystal was precipitated in 2,000 g of hexane. The precipitated crystal was separated by filtration, washed twice with 500 g of hexane, and collected. The collected crystal was vacuum-dried at 70°C to obtain a resin (R-7).

The weight-average molecular weight (Mw) and the dispersity (Mw/Mn) were determined by gel permeation chromatography (GPC) using tetrahydrofuran as an eluent, and the following results were obtained.
(R-7): Mw=3,300, Mw/Mn=2.54

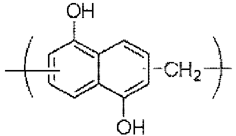

### [Synthesis of Flowability Accelerator]

To synthesize the flowability accelerator, the organic group starting material group G ((G25) to (G27)) and the modifying agent K ((K1) to (K2)) shown below were used.

The organic group starting material group G ((G25) to (G27)) are shown below.

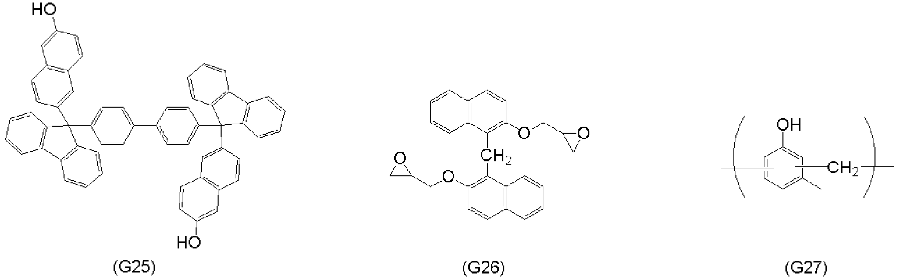

The modifying agent K ((K1) to (K2)) are shown below.

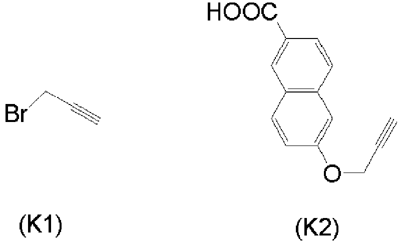

### [Synthesis of Flowability Accelerator (BPA-1)]

Under a nitrogen atmosphere, 45.5 g of the compound (G25) of the organic group starting material group G, 9.8 g of potassium carbonate, and 150 g of DMF were added to form a homogeneous dispersion at an internal temperature of 50°C. 17.6 g of the modifying agent (K1) was slowly added thereto, and a reaction was allowed to take place at an internal temperature of 50°C for 24 hours. 300 ml of methyl isobutyl ketone and 300 g of pure water were added to the reaction solution to dissolve the precipitated salt, and then the separated aqueous layer was removed. Furthermore, the organic layer was washed six times with 100 g of a 3% aqueous nitric acid solution and 100 g of pure water, and then by drying the organic layer under reduced pressure to obtain a flowability accelerator (BPA-1).

The weight-average molecular weight (Mw) and the dispersity (Mw/Mn) were determined by GPC, and the following results were obtained.
(BPA-1): Mw=965, Mw/Mn=1.08

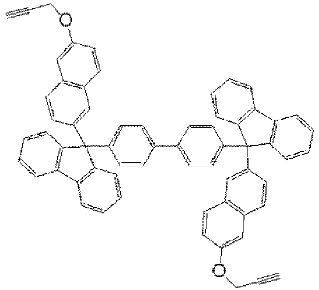

### [Synthesis of Flowability Accelerator (BPA-2)]

Under a nitrogen atmosphere, 80.0 g of the compound (G26) of the organic group starting material group G, 51.0 g of the modifying agent (K2), 600 g of 2-methoxy-1-propanol were formed a homogeneous solution at an internal temperature of 100°C. And then, 5.7 g of benzyltriethylammonium chloride was added to the solution and agitated at an internal temperature of 120°C for 12 hours. After cooling down to room temperature, 1,500 g of methyl isobutyl ketone was added to the solution and the organic layer was washed five times with 300 g of pure water. The organic layer was dried under reduced pressure to obtain a flowability accelerator (BPA-2).

The weight-average molecular weight (Mw) and the dispersity (Mw/Mn) were determined by GPC, and the following results were obtained.
(BPA-2): Mw=900, Mw/Mn=1.04

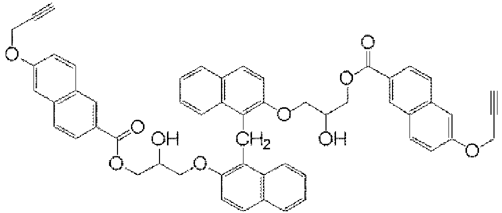

### [Synthesis of Flowability Accelerator (BPA-3)]

Under a nitrogen atmosphere, 20.0 g of the resin (G27) of the organic group starting material group G, 34.5 g of potassium carbonate, and 100 g of DMF were added to form a homogeneous dispersion at an internal temperature of 50°C. 23.8 g of the modifying agent (K1) was slowly added thereto, and a reaction was allowed to take place at an internal temperature of 50°C for 24 hours. 300 ml of methyl isobutyl ketone and 300 g of pure water were added to the reaction solution to dissolve the precipitated salt, and then the separated aqueous layer was removed. Furthermore, the organic layer was washed six times with 100 g of a 3% aqueous nitric acid solution and 100 g of pure water, and then by drying the organic layer under reduced pressure to obtain a flowability accelerator (BPA-3).

The weight-average molecular weight (Mw) and the dispersity (Mw/Mn) were determined by GPC, and the following results were obtained.
(BPA-3): Mw=9,400, Mw/Mn=3.59

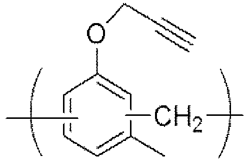

### [Preparation of Composition (UDL-1) for Forming Metal-Containing Film]

The compound (A-1) for forming a metal-containing film was dissolved in a mixed solvent of propylene glycol monomethyl ether acetate (PGMEA) containing 0.5 mass% of surfactant FC-4430 (manufactured by Sumitomo 3M Limited) and propylene glycol monomethyl ether (PGME) at the proportion shown in Table 4, and filtered through a 0.02 um membrane filter to prepare the composition (UDL-1) for forming a metal-containing film.

### [Preparation of Compositions (UDL-2 to 29) for Forming Metal-Containing Film and Comparative Compositions (Comparative UDL-1 to 7) for Forming Metal-Containing Film]

Each chemical liquid was prepared in the same manner as UDL-1, except that the type and the contained amount of each component were as shown in Tables 4 and 5. Incidentally, in Tables 4 and 5, "-" indicates that the corresponding component was not used. The following formula (C-1) was used for the crosslinking agent; the following formula (F-1) was used for the acid generator (TAG); ethylene glycol dibenzyl ether (boiling point: 364°C) was used for the high-boiling-point solvent (B1); and ZrO₂ nanoparticles (5 nm core, 915505, available from Sigma-Aldrich Corp) was used for the metal nanoparticles (G-1).

### [Crosslinking Agent and Acid Generator]

The crosslinking agent (C-1) and the acid generator (F-1) used in the composition for forming a metal-containing film are shown below.

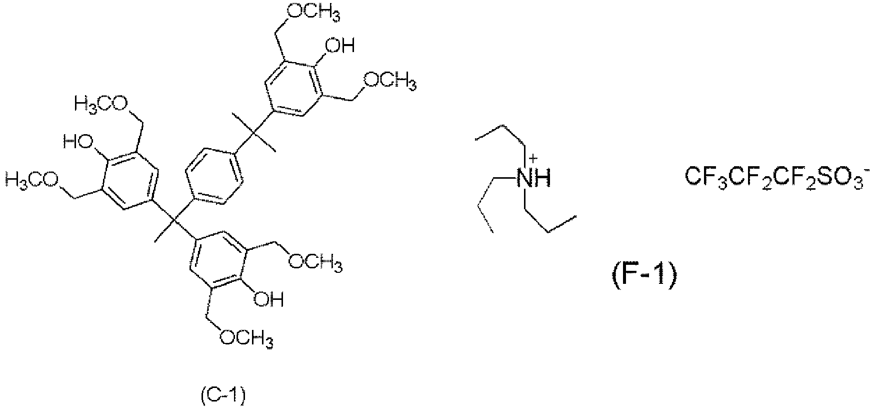

**[Table 4]**

| Composition for forming a metal-containing film | Compound for forming a metal-containing film (parts by mass) | Additive (parts by mass) | Solvent (parts by mass) |
|---|---|---|---|
| UDL-1 | A-1 (10) | - | PGMEA/PGME (60/30) |
| UDL-2 | A-2 (10) | - | PGMEA/PGME (60/30) |
| UDL-3 | A-3 (10) | - | PGMEA/PGME (60/30) |
| UDL-4 | A-4 (10) | - | PGMEA/PGME (60/30) |
| UDL-5 | A-5 (10) | - | PGMEA/PGME (60/30) |
| UDL-6 | A-6 (10) | - | PGMEA/PGME (60/30) |
| UDL-7 | A-7 (10) | - | PGMEA/PGME (60/30) |
| UDL-8 | A-8 (10) | - | PGMEA/PGME (60/30) |
| UDL-9 | A-9 (10) | - | PGMEA/PGME (60/30) |
| UDL-10 | A-10 (10) | - | PGMEA/PGME (60/30) |
| UDL-11 | A-11 (10) | - | PGMEA/PGME (60/30) |
| UDL-12 | A-12 (10) | - | PGMEA/PGME (60/30) |
| UDL-13 | A-13 (10) | - | PGMEA/PGME (60/30) |
| UDL-14 | A-14 (10) | - | PGMEA/PGME (60/30) |
| UDL-15 | A-15 (10) | - | PGMEA/PGME (60/30) |
| UDL-16 | A-16 (10) | - | PGMEA/PGME (60/30) |
| UDL-17 | A-17 (10) | - | PGMEA/PGME (60/30) |
| UDL-18 | A-18 (10) | - | PGMEA/PGME (60/30) |
| UDL-19 | A-19 (10) | - | PGMEA/PGME (60/30) |
| UDL-20 | A-20 (10) | - | PGMEA/PGME (60/30) |
| UDL-21 | A-21 (10) | - | PGMEA/PGME (60/30) |
| UDL-22 | A-22 (10) | - | PGMEA/PGME (60/30) |
| UDL-23 | A-4 (10) | F-1 (0.2) | PGMEA/PGME (60/30) |
| UDL-24 | A-6 (10) | C-1 (2) | PGMEA/PGME (60/30) |
| UDL-25 | A-7 (10) | G-1 (2) | PGMEA/PGME (60/30) |
| UDL-26 | A-8 (10) | B1 (5) | PGMEA/PGME (60/30) |
| UDL-27 | A-9 (10) | BPA-1 (1) | PGMEA/PGME (60/30) |
| UDL-28 | A-10 (10) | BPA-2 (1) | PGMEA/PGME (60/30) |
| UDL-29 | A-11 (10) | BPA-3 (1) | PGMEA/PGME (60/30) |

**[Table 5]**

| Comparative composition for forming metal-containing film | Comparative compound (parts by mass) | Additive (parts by mass) | Solvent (parts by mass) |
|---|---|---|---|
| Comparative UDL-1 | R-1 (10) | - | PGMEA/PGME (60/30) |
| Comparative UDL-2 | R-2 (10) | - | PGMEA/PGME (60/30) |
| Comparative UDL-3 | R-3 (10) | - | PGMEA/PGME (60/30) |
| Comparative UDL-4 | R-4 (10) | - | PGMEA/PGME (60/30) |
| Comparative UDL-5 | R-5 (10) | - | PGMEA/PGME (60/30) |
| Comparative UDL-6 | R-6 (10) | - | PGMEA (90) |
| Comparative UDL-7 | R-7 (5) | - | PGMEA (95) |

### [Filling Property Evaluation (Examples 1-1 to 1-29 and Comparative Examples 1-1 to 1-7)]

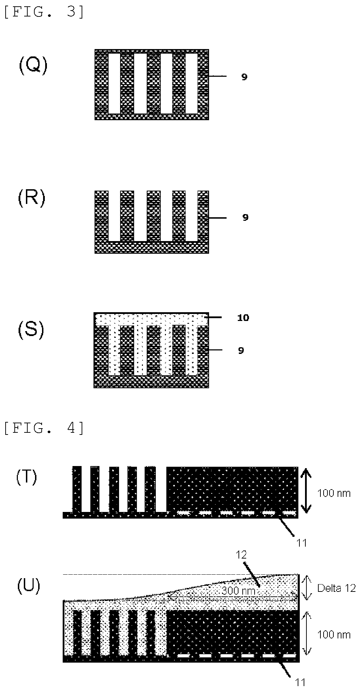

Each of the compositions (UDL-1 to 29 and Comparative UDL-1 to 1-7) prepared above was respectively applied to a SiO₂ wafer substrate having a dense line-and-space pattern (line width = 60 nm, line depth = 100 nm, distance between the centers of two adjacent lines = 120 nm), followed by heating at 350°C for 60 seconds by using a hot plate to form a metal-containing film having a film thickness of 80 nm. The substrate used was a base substrate 9 (SiO₂ wafer substrate) having a dense line-and-space pattern as shown in FIG. 3(Q) (downward view) and (R) (cross-sectional view). The cross-sectional shape of each of the wafer substrates obtained in the filling evaluation A and B was observed using an electron microscope (S-4700, manufactured by Hitachi, Ltd.), and it was confirmed whether or not there was any void (gap) in the metal-containing film with which a space between lines was filled. The results are shown in Tables 6 and 7. In this evaluation, when a composition for forming a metal-containing film having a poor filling property is used, a void is generated in the metal-containing film with which a space between lines is filled. In this evaluation, when a composition for forming a metal-containing film having a good filling property is used, as shown in FIG. 3(S), inside of the metal-containing film filled in the space between the lines of the base substrate 9 having the dense line-and-space pattern can be filled with the metal-containing film 10 having no void.

**[Table 6]**

| Example | Composition for forming metal-containing film | 350°C Void |
|---|---|---|
| Example 1-1 | UDL-1 | Absent |
| Example 1-2 | UDL-2 | Absent |
| Example 1-3 | UDL-3 | Absent |
| Example 1-4 | UDL-4 | Absent |
| Example 1-5 | UDL-5 | Absent |
| Example 1-6 | UDL-6 | Absent |
| Example 1-7 | UDL-7 | Absent |
| Example 1-8 | UDL-8 | Absent |
| Example 1-9 | UDL-9 | Absent |
| Example 1-10 | UDL-10 | Absent |
| Example 1-11 | UDL-11 | Absent |
| Example 1-12 | UDL-12 | Absent |
| Example 1-13 | UDL-13 | Absent |
| Example 1-14 | UDL-14 | Absent |
| Example 1-15 | UDL-15 | Absent |
| Example 1-16 | UDL-16 | Absent |
| Example 1-17 | UDL-17 | Absent |
| Example 1-18 | UDL-18 | Absent |
| Example 1-19 | UDL-19 | Absent |
| Example 1-20 | UDL-20 | Absent |
| Example 1-21 | UDL-21 | Absent |
| Example 1-22 | UDL-22 | Absent |
| Example 1-23 | UDL-23 | Absent |
| Example 1-24 | UDL-24 | Absent |
| Example 1-25 | UDL-25 | Absent |
| Example 1-26 | UDL-26 | Absent |
| Example 1-27 | UDL-27 | Absent |
| Example 1-28 | UDL-28 | Absent |
| Example 1-29 | UDL-29 | Absent |

**[Table 7]**

| Comparative Example | Comparative composition for forming metal-containing film | 350°C Void |
|---|---|---|
| Comparative Example 1-1 | Comparative UDL-1 | Present |
| Comparative Example 1-2 | Comparative UDL-2 | Present |
| Comparative Example 1-3 | Comparative UDL-3 | Present |
| Comparative Example 1-4 | Comparative UDL-4 | Absent |
| Comparative Example 1-5 | Comparative UDL-5 | Present |
| Comparative Example 1-6 | Comparative UDL-6 | Present |
| Comparative Example 1-7 | Comparative UDL-7 | Absent |

As shown in Table 6, in Examples 1-1 to 1-29 using the inventive compositions (UDL-1 to 29) for forming a metal-containing film, it was possible to fill the dense line-and-space pattern without generating a void even after baking at 350°C, and it was confirmed that the inventive composition has a good filling property even under high-temperature baking conditions. On the other hand, as shown in Table 7, in Comparative Examples 1-1 to 1-3 and 1-5 to 1-6 using metal compounds (Comparative UDL-1 to 3 and 5 to 6) that do not contain any of the crosslinking groups represented by general formulae (a-1) to (a-3), unlike the inventive compound for forming a metal-containing film, voids were observed at the bottom of the pattern after baking at 350°C. It is presumed that these have low durability of the organic ligand coordinated to the metal, so that the volume shrinkage due to high-temperature baking is large, resulting in the generation of voids.

### [Planarizing Property Evaluation (Examples 2-1 to 2-29 and Comparative Examples 2-1 to 2-2)]

Regarding base substrates 11 (SiO₂ wafer substrates) having a dense line-and-space pattern as shown in FIG. 4(T), the cross sectional shape of each of the wafer substrates in which no void was observed in the above filling property evaluation conducted after 350°C baking as shown in FIG. 4(U) was observed using a scanning electron microscope (SEM), and the step (Delta 12 in FIG. 4(U)) between the line-pattern-dense portion and the non-line-pattern portion of the filling film 12 was observed using an electron microscope (S-4700, manufactured by Hitachi, Ltd.). The results are shown in Tables 8 and 9. In the present evaluation, it can be said that the smaller the step, the better the planarizing property.

**[Table 8]**

| Example | Composition for forming metal-containing film | Flatness nm |
|---|---|---|
| Example 2-1 | UDL-1 | 59 |
| Example 2-2 | UDL-2 | 55 |
| Example 2-3 | UDL-3 | 57 |
| Example 2-4 | UDL-4 | 51 |
| Example 2-5 | UDL-5 | 53 |
| Example 2-6 | UDL-6 | 49 |
| Example 2-7 | UDL-7 | 52 |
| Example 2-8 | UDL-8 | 47 |
| Example 2-9 | UDL-9 | 45 |
| Example 2-10 | UDL-10 | 50 |
| Example 2-11 | UDL-11 | 51 |
| Example 2-12 | UDL-12 | 50 |
| Example 2-13 | UDL-13 | 49 |
| Example 2-14 | UDL-14 | 48 |
| Example 2-15 | UDL-15 | 49 |
| Example 2-16 | UDL-16 | 50 |
| Example 2-17 | UDL-17 | 51 |
| Example 2-18 | UDL-18 | 52 |
| Example 2-19 | UDL-19 | 56 |
| Example 2-20 | UDL-20 | 49 |
| Example 2-21 | UDL-21 | 47 |
| Example 2-22 | UDL-22 | 48 |
| Example 2-23 | UDL-23 | 50 |
| Example 2-24 | UDL-24 | 48 |
| Example 2-25 | UDL-25 | 51 |
| Example 2-26 | UDL-26 | 46 |
| Example 2-27 | UDL-27 | 44 |
| Example 2-28 | UDL-28 | 49 |
| Example 2-29 | UDL-29 | 50 |

**[Table 9]**

| Comparative Example | Comparative composition for forming metal-containing film | Flatness nm |
|---|---|---|
| Comparative Example 2-1 | Comparative UDL-4 | 59 |
| Comparative Example 2-2 | Comparative UDL-7 | 49 |

As shown in Tables 8 and 9, in Examples 2-1 to 2-29 using the inventive compositions (UDL-1 to 29) for forming a metal-containing film, the step between the patterned portion and the non-patterned portion of the film is small, and it was found to have as good planarizing performance as Comparative Example 2-1 using a carboxylic acid as a ligand and Comparative Example 2-2 using an organic resist underlayer film material. When comparing with UDL-7 to 9 in which the number of crosslinking groups contained in the organic ligand is changed, the results show that the more crosslinking groups contained in the organic ligand, the better the planarizing property. In addition, it was found that UDL-4 to 18 and 20 to 29 having an aromatic ring-containing ligand exhibit a good planarizing property, probably due to their excellent heat resistance properties. In addition, UDL-26 to which a high-boiling-point solvent (B1) was added and UDL-27 to 29 to which a flowability accelerator (BPA-1 to 3) was added showed a more excellent planarizing property. It is presumed that the use of the additive further improved the thermal flowability of the composition for forming a metal-containing film.

### [Etching Resistance Evaluation (Examples 3-1 to 3-29 and Comparative Examples 3-1 to 3-7)]

Each of the compositions (UDL-1 to 29 and comparative UDL-1 to 7) for forming a metal-containing film was respectively applied onto a silicon substrate and heated by using a hot plate at 350°C for 60 seconds to form a metal-containing film having a film thickness of 80 nm, and then the film thickness "a" was measured. Subsequently, using an etching apparatus TE8500 (manufactured by Tokyo Electron Ltd.), etching was each performed under the following conditions, and the film thickness "b" was measured. Then, the film thickness etched in one minute was calculated as an etching rate (nm/min) from the film thickness etched (film thickness "a" - film thickness "b") in a specified time. When the etching rate was 50 nm/min or lower, the evaluation "A (very good)" was given, when greater than 50 nm/min and not greater than 70 nm/min, "B (good)", and when over 70 nm/min, "C (poor)". The results are shown in Tables 10 and 11.

### Conditions of dry etching with CF₄ gas

Pressure: 1 Pa
Antenna RF power: 100 W
Bias RF power: 15 W
CF₄ gas flow rate: 15 sccm
Time: 30 sec

**[Table 10]**

| Example | Composition for forming metal-containing film | Etching resistance nm/min |
|---|---|---|
| Example 3-1 | UDL-1 | A |
| Example 3-2 | UDL-2 | A |
| Example 3-3 | UDL-3 | A |
| Example 3-4 | UDL-4 | A |
| Example 3-5 | UDL-5 | A |
| Example 3-6 | UDL-6 | A |
| Example 3-7 | UDL-7 | A |
| Example 3-8 | UDL-8 | A |
| Example 3-9 | UDL-9 | A |
| Example 3-10 | UDL-10 | A |
| Example 3-11 | UDL-11 | A |
| Example 3-12 | UDL-12 | A |
| Example 3-13 | UDL-13 | A |
| Example 3-14 | UDL-14 | A |
| Example 3-15 | UDL-15 | A |
| Example 3-16 | UDL-16 | A |
| Example 3-17 | UDL-17 | A |
| Example 3-18 | UDL-18 | A |
| Example 3-19 | UDL-19 | A |
| Example 3-20 | UDL-20 | A |
| Example 3-21 | UDL-21 | A |
| Example 3-22 | UDL-22 | A |
| Example 3-23 | UDL-23 | A |
| Example 3-24 | UDL-24 | A |
| Example 3-25 | UDL-25 | A |
| Example 3-26 | UDL-26 | A |
| Example 3-27 | UDL-27 | A |
| Example 3-28 | UDL-28 | A |
| Example 3-29 | UDL-29 | A |

**[Table 11]**

| Comparative Example | Comparative composition for forming metal-containing film | Etching resistance nm/min |
|---|---|---|
| Comparative Example 3-1 | Comparative UDL-1 | A |
| Comparative Example 3-2 | Comparative UDL-2 | A |
| Comparative Example 3-3 | Comparative UDL-3 | A |
| Comparative Example 3-4 | Comparative UDL-4 | B |
| Comparative Example 3-5 | Comparative UDL-5 | A |
| Comparative Example 3-6 | Comparative UDL-6 | A |
| Comparative Example 3-7 | Comparative UDL-7 | C |

As shown in Tables 10 and 11, it was found that Examples 3-1 to 3-29 using the inventive compositions (UDL-1 to 29) for forming a metal-containing film showed extremely excellent etching resistance with respect to CF₄ gas compared to Comparative Example 3-7 using the organic resist underlayer film material (Comparative UDL-7). Also, in Comparative Example 3-4 using carboxylic acid as a ligand, deterioration of the etching resistance with respect to CF₄ gas was confirmed, but this is presumably because the coordination ability of carboxylic acid to metal atoms is large, and more organic components remain in the metal-containing film after baking.

### [Patterning Process (Examples 4-1 to 4-29 and Comparative Examples 4-1 and 4-7)]

Each of the above compositions (UDL-1 to 29, Comparative UDL-1 to 7) for forming a metal-containing film was respectively applied onto an SiO₂ wafer substrate having a trench pattern (trench width: 10 um, trench depth: 0.10 um), and baked at 350°C for 60 seconds in the atmosphere to form a metal-containing film having a thickness of 70 nm. A silicon-containing resist middle layer film composition (SOG-1) was applied thereto, followed by baking at 220°C for 60 seconds to form a resist middle layer film having a thickness of 50 nm. A monolayer resist for ArF as a resist upper layer film material was applied thereto, followed by baking at 105°C for 60 seconds to form a photoresist film having a thickness of 100 nm. A liquid immersion top coat composition (TC-1) was applied onto the photoresist film, followed by baking at 90°C for 60 seconds to form a top coat having a thickness of 50 nm.

The silicon-containing resist middle layer film composition (SOG-1) was prepared by dissolving a polymer represented by an ArF silicon-containing resist middle layer film polymer (SiP1) and a thermal crosslinking catalyst (CAT1) in an organic solvent containing 0.1 mass% of FC-4430 (manufactured by Sumitomo 3M Limited) in the proportion shown in Table 12 and filtering the solution through a filter made of a fluororesin and having a pore size of 0.1 um.

**[Table 12]**

| | Polymer (parts by mass) | Thermal crosslinking catalyst (parts by mass) | Organic solvent (parts by mass) |
|---|---|---|---|
| SOG-1 | SiP1 (100) | CAT1 (1) | Propylene glycol monoethyl ether (4, 000) |

The structural formulae of the used ArF silicon-containing resist middle layer film polymer (SiP1) and thermal crosslinking catalyst (CAT1) are shown below.

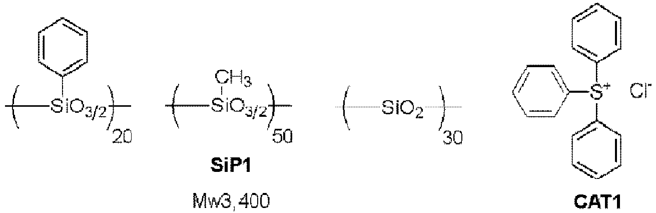

The resist upper layer film material (monolayer resist for ArF) was prepared by dissolving a polymer (RP1), an acid generator (PAG1), and a basic compound (Amine1), each in the proportion shown in Table 13, in a solvent containing 0.1 mass% of a surfactant FC-4430 (manufactured by Sumitomo 3M Limited), and filtering the solution through a 0.1-pm filter made of a fluororesin.

**[Table 13]**

| | Polymer (parts by mass) | Acid generator (parts by mass) | Basic compound (parts by mass) | Solvent (parts by mass) |
|---|---|---|---|---|
| Monolayer resist for ArF | RP1 (100) | PAG1 (6.6) | Amine1 (0.8) | PGMEA (2, 500) |

The polymer (RP1), the acid generator (PAG1), and the basic compound (Amine1) used for the resist upper layer film material (monolayer resist for ArF) are shown below.

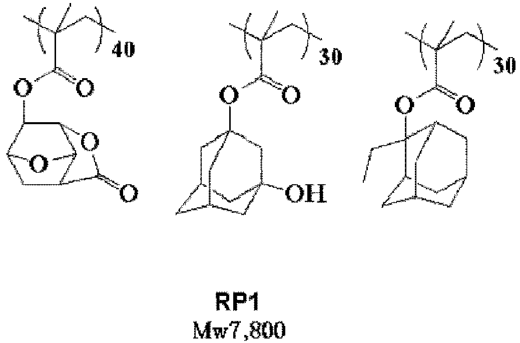

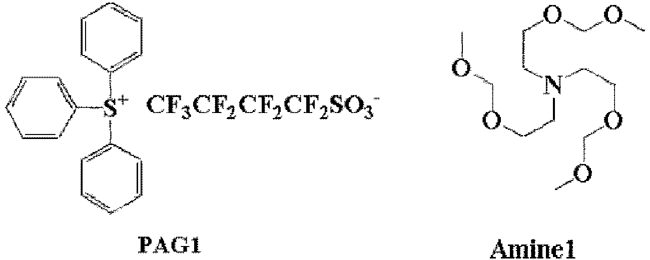

The liquid immersion top coat composition (TC-1) was prepared by dissolving a top coat polymer (PP1) in an organic solvent at the proportion shown in Table 14, and filtering the solution through a 0.1-pm filter made of a fluororesin.

**[Table 14]**

| | Top coat polymer (parts by mass) | Organic solvent (parts by mass) |
|---|---|---|
| TC-1 | PP1 (100) | Diisoamyl ether (2,700) |
| | | 2-methyl-1-butanol (270) |

The top coat polymer (PP1) used for the liquid immersion top coat composition (TC-1) is shown below.

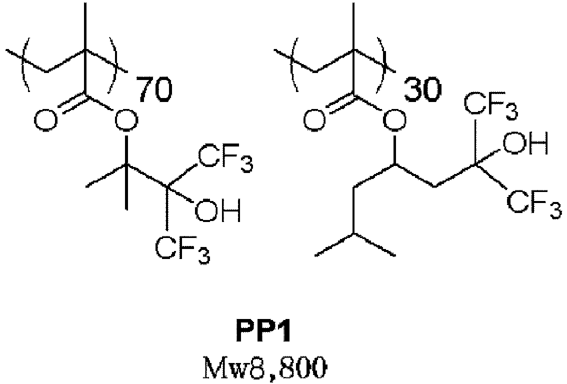

Then, the substrate was exposed to light with an ArF liquid-immersion exposure apparatus (NSR-S610C manufactured by Nikon Corporation, NA: 1.30, σ: 0.98/0.65, 35° s-polarized dipole illumination, 6% halftone phase shift mask), baked at 100°C for 60 seconds (PEB), and developed with a 2.38 mass% aqueous solution of tetramethylammonium hydroxide (TMAH) for 30 seconds, thereby obtaining a 55 nm 1:1 positive line-and-space pattern (a resist upper layer film pattern).

Subsequently, the resist middle layer film was etched by dry etching while using the resist upper layer film pattern as a mask to form a hard mask pattern, the metal-containing film was etched while using the obtained hard mask pattern as a mask to form a metal-containing film pattern, and the SiO₂ film was etched while using the obtained metal-containing film pattern as a mask. The etching conditions were as follows.

### Conditions in transferring the resist upper layer film pattern to the resist middle layer film Conditions of dry etching with CF₄ gas

Pressure: 1 Pa
Antenna RF power: 100 W
Bias RF power: 15 W
CF₄ gas flow rate: 15 sccm
Time: 60 sec

### Conditions in transferring the hard mask pattern to the metal-containing film

### Conditions of dry etching with Cl₂ gas

Pressure: 1 Pa
Antenna RF power: 320 W
Bias RF power: 30 W
Cl₂ gas flow rate: 25 sccm
Time: 45 sec

### Conditions in transferring the metal-containing film pattern to the SiO₂ film

### Conditions of dry etching with CF₄ gas

Pressure: 1 Pa
Antenna RF power: 100 W
Bias RF power: 15 W
CF₄ gas flow rate: 15 sccm
Time: 60 sec

The pattern cross section was observed with an electron microscope (S-4700) manufactured by Hitachi, Ltd. The results are shown in Tables 15 and 16.

**[Table 15]**

| Example | Composition for forming metal-containing film | Pattern profile after etching for transfer to substrate |
|---|---|---|
| Example 4-1 | UDL-1 | Vertical profile |
| Example 4-2 | UDL-2 | Vertical profile |
| Example 4-3 | UDL-3 | Vertical profile |
| Example 4-4 | UDL-4 | Vertical profile |
| Example 4-5 | UDL-5 | Vertical profile |
| Example 4-6 | UDL-6 | Vertical profile |
| Example 4-7 | UDL-7 | Vertical profile |
| Example 4-8 | UDL-8 | Vertical profile |
| Example 4-9 | UDL-9 | Vertical profile |
| Example 4-10 | UDL-10 | Vertical profile |
| Example 4-11 | UDL-11 | Vertical profile |
| Example 4-12 | UDL-12 | Vertical profile |
| Example 4-13 | UDL-13 | Vertical profile |
| Example 4-14 | UDL-14 | Vertical profile |
| Example 4-15 | UDL-15 | Vertical profile |
| Example 4-16 | UDL-16 | Vertical profile |
| Example 4-17 | UDL-17 | Vertical profile |
| Example 4-18 | UDL-18 | Vertical profile |
| Example 4-19 | UDL-19 | Vertical profile |
| Example 4-20 | UDL-20 | Vertical profile |
| Example 4-21 | UDL-21 | Vertical profile |
| Example 4-22 | UDL-22 | Vertical profile |
| Example 4-23 | UDL-23 | Vertical profile |
| Example 4-24 | UDL-24 | Vertical profile |
| Example 4-25 | UDL-25 | Vertical profile |
| Example 4-26 | UDL-26 | Vertical profile |
| Example 4-27 | UDL-27 | Vertical profile |
| Example 4-28 | UDL-28 | Vertical profile |
| Example 4-29 | UDL-29 | Vertical profile |

**[Table 16]**

| Comparative Example | Comparative composition for forming metal-containing film | Pattern profile after etching for transfer to substrate |
|---|---|---|
| Comparative Example 4-1 | Comparative UDL-1 | Pattern collapse |
| Comparative Example 4-2 | Comparative UDL-2 | Pattern collapse |
| Comparative Example 4-3 | Comparative UDL-3 | Pattern collapse |
| Comparative Example 4-4 | Comparative UDL-4 | Pattern collapse |
| Comparative Example 4-5 | Comparative UDL-5 | Pattern collapse |
| Comparative Example 4-6 | Comparative UDL-6 | Pattern collapse |
| Comparative Example 4-7 | Comparative UDL-7 | Distorted pattern |

As shown in Table 15, in Examples 4-1 to 4-29 where the inventive compositions (UDL-1 to 29) for forming a metal-containing film were used, the resist upper layer film pattern was successfully transferred finally to the substrate in every case. Thus, it was confirmed that the inventive composition for forming a metal-containing film can be used suitably for fine processing according to a multilayer resist method. On the other hand, in Comparative Examples 4-1 to 4-6 using the comparative UDL-1 to 6 that were confirmed to have insufficient performance in the filling property evaluation and the etching resistance evaluation, pattern collapse occurred during patterning and it was not possible to obtain a good pattern finally as shown in Table 16. Meanwhile, in Comparative Example 4-7 using the comparative UDL-7 that was confirmed to have insufficient performance in the etching resistance evaluation, distortion of the pattern profile occurred in pattern processing, and it was not possible to obtain a good pattern finally.

### [Reverse SOC Patterning Process (Examples 5-1 to 5-29 and Comparative Examples 5-1 to 5-8)]

As a resist underlayer film a coating-type resist underlayer film material (SOC-1) was applied onto a silicon wafer substrate having a formed SiO₂ film of a 300 nm and baked at 350°C for 60 seconds to form a resist underlayer film having a thickness of 80 nm. A silicon-containing resist middle layer film composition (SOG-1) was applied thereto, followed by baking at 220°C for 60 seconds to form a resist middle layer film having a thickness of 40 nm. A monolayer resist for ArF as a resist upper layer film material was applied thereto, followed by baking at 105°C for 60 seconds to form a photoresist film having a thickness of 100 nm. A liquid immersion top coat composition (TC-1) was applied onto the photoresist film, followed by baking at 90°C for 60 seconds to form a top coat having a thickness of 50 nm.

For the silicon-containing resist middle layer film composition (SOG-1), the resist upper layer film material (monolayer resist for ArF), and the liquid immersion top coat composition (TC-1) on the photoresist film, the same material as the above patterning process (Example 4) were used.

The coating-type resist underlayer film material (SOC-1) was prepared by dissolving a polymer represented by a polymer (SOP1) for forming a resist underlayer film in an organic solvent containing 0.1 mass% of FC-4430 (manufactured by Sumitomo 3M Limited) in the proportion shown in Table 17 and then filtering the solution through a filter made of a fluororesin and having a pore size of 0.2 um.

**[Table 17]**

| | Polymer for resist underlayer film (parts by mass) | Organic solvent (parts by mass) |
|---|---|---|
| SOC-1 | SOP1 (5) | Propylene glycol monoethyl ether (95) |

The structural formula of the used polymer (SOP1) for forming a resist underlayer film is shown in Table 18.

**[Table 18]**

| Compound or composition | Mw | Mw/Mn |
|---|---|---|
| 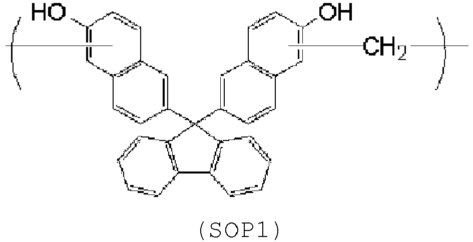 | 3, 700 | 2.82 |

Next, the material was exposed to light with an ArF liquid immersion exposure apparatus (NSR-S610C manufactured by Nikon Corporation, NA: 1.30, σ: 0.98/0.65, 35° s-polarized dipole illumination, 6% halftone phase shift mask), baked at 100°C for 60 seconds (PEB), and developed with a 2.38 mass% aqueous solution of tetramethylammonium hydroxide (TMAH) for 30 seconds, thereby obtaining a 55 nm 1:1 positive line-and-space pattern (a resist upper layer film pattern).

Subsequently, using an etching apparatus Telius (manufactured by Tokyo Electron Ltd.) the resist middle layer film was etched by dry etching while using the resist upper layer film pattern as a mask to form a hard mask pattern. The resist underlayer film (SOC-1) was then etched while using the obtained hard mask pattern as a mask to form a SOC-1 film pattern. The etching conditions were as follows.

### Conditions in transferring the resist upper layer film pattern to the resist middle layer film

| | |
|---|---|
| Chamber pressure: | 50 mT |
| RF power (Upper) : | 500 W |
| RF power (Lower) : | 300 W |
| CF₄ gas flow rate: | 150 sccm |
| CHF₃ gas flow rate: | 50 sccm |
| Time: | 20 sec |

### Conditions in transferring the hard mask pattern to the resist underlayer film

| | |
|---|---|
| Chamber pressure: | 10 mT |
| RF power (Upper) : | 1,000 W |
| RF power(Lower): | 300 W |
| CO₂ gas flow rate: | 150 sccm |
| CO gas flow rate: | 50 sccm |
| N₂ gas flow rate: | 50 sccm |
| H₂ gas flow rate: | 150 sccm |
| Time: | 60 sec |

Next, each of the above compositions (UDL-1 to 29 and Comparative UDL-1 to 7) for forming a metal-containing film was respectively applied onto the obtained SOC-1 film pattern, and baked at 350°C for 60 seconds in the atmosphere to form a metal-containing film having a film thickness of 80 nm. Thereafter, the metal-containing film covering the SOC-1 film pattern was etched to expose an upper surface of the SOC-1 film pattern. The resist middle layer film remaining on the surface of the SOC-1 film pattern with its upper surface exposed was removed by etching, and then the exposed SOC-1 film pattern was removed by etching, the above pattern was inverted onto the metal-containing film, and the SiO₂ film was etched while using the obtained metal-containing film pattern as a mask. As a comparative example, the SiO₂ film was etched while using the SOC-1 film pattern as a mask without using the composition for forming a metal-containing film (Comparative Example 5-8). The etching conditions are as shown below.

### Conditions of etching back the metal-containing film

### (Exposure of SOC-1 film pattern)

Pressure: 1 Pa
Antenna RF power: 320 W
Bias RF power: 30 W
Cl₂ gas flow rate: 25 sccm
Time: 15 sec

### Removal of the resist middle layer film remaining on the surface of the SOC-1 film pattern

### Conditions of dry etching with CF₄ gas

Pressure: 1 Pa
Antenna RF power: 100 W
Bias RF power: 15 W
CF₄ gas flow rate: 15 sccm
Time: 45 sec

### Removal of the SOC-1 film pattern

### Conditions of dry etching with O₂ gas

Pressure: 1 Pa
Antenna RF power: 300 W
Bias RF power: 0 W
O₂ gas flow rate: 25 sccm
Time: 30 sec

### Conditions in transferring the metal-containing film pattern to the SiO₂ film

### Conditions of dry etching with CF₄ gas

Pressure: 1 Pa
Antenna RF power: 100 W
Bias RF power: 15 W
CF₄ gas flow rate: 15 sccm
Time: 60 sec

### Comparative Example 5-8: Conditions in transferring the SOC-1 film pattern to the SiO₂ film

### Conditions of dry etching with CF₄ gas

Pressure: 1 Pa
Antenna RF power: 100 W
Bias RF power: 15 W
CF₄ gas flow rate: 15 sccm
Time: 60 sec

The pattern cross section was observed with an electron microscope (S-4700) manufactured by Hitachi, Ltd. The results are shown in Tables 19 and 20.

**[Table 19]**

| Example | Composition for forming metal-containing film | Pattern profile after SOC-pattern reversal |
|---|---|---|
| Example 5-1 | UDL-1 | Vertical profile |
| Example 5-2 | UDL-2 | Vertical profile |
| Example 5-3 | UDL-3 | Vertical profile |
| Example 5-4 | UDL-4 | Vertical profile |
| Example 5-5 | UDL-5 | Vertical profile |
| Example 5-6 | UDL-6 | Vertical profile |
| Example 5-7 | UDL-7 | Vertical profile |
| Example 5-8 | UDL-8 | Vertical profile |
| Example 5-9 | UDL-9 | Vertical profile |
| Example 5-10 | UDL-10 | Vertical profile |
| Example 5-11 | UDL-11 | Vertical profile |
| Example 5-12 | UDL-12 | Vertical profile |
| Example 5-13 | UDL-13 | Vertical profile |
| Example 5-14 | UDL-14 | Vertical profile |
| Example 5-15 | UDL-15 | Vertical profile |
| Example 5-16 | UDL-16 | Vertical profile |
| Example 5-17 | UDL-17 | Vertical profile |
| Example 5-18 | UDL-18 | Vertical profile |
| Example 5-19 | UDL-19 | Vertical profile |
| Example 5-20 | UDL-20 | Vertical profile |
| Example 5-21 | UDL-21 | Vertical profile |
| Example 5-22 | UDL-22 | Vertical profile |
| Example 5-23 | UDL-23 | Vertical profile |
| Example 5-24 | UDL-24 | Vertical profile |
| Example 5-25 | UDL-25 | Vertical profile |
| Example 5-26 | UDL-26 | Vertical profile |
| Example 5-27 | UDL-27 | Vertical profile |
| Example 5-28 | UDL-28 | Vertical profile |
| Example 5-29 | UDL-29 | Vertical profile |

**[Table 20]**

| Comparative Example | Comparative composition for forming metal-containing film | Pattern profile after SOC-pattern reversal |
|---|---|---|
| Comparative Example 5-1 | Comparative UDL-1 | Pattern collapse |
| Comparative Example 5-2 | Comparative UDL-2 | Pattern collapse |
| Comparative Example 5-3 | Comparative UDL-3 | Pattern collapse |
| Comparative Example 5-4 | Comparative UDL-4 | Pattern collapse |
| Comparative Example 5-5 | Comparative UDL-5 | Pattern collapse |
| Comparative Example 5-6 | Comparative UDL-6 | Pattern collapse |
| Comparative Example 5-7 | Comparative UDL-7 | Distorted pattern |
| Comparative Example 5-8 | Absent | Distorted pattern |

As shown in Table 19, in Examples 5-1 to 5-29 using the inventive compositions (UDL-1 to 29) for forming a metal-containing film, the SOC-1 film pattern was accurately reversed in any cases, and the reverse pattern was transferred finally to the substrate suitably without pattern collapse. Thus, it was confirmed that the inventive composition for forming a metal-containing film can be used suitably for fine processing using a tone-reversal etching process according to a multilayer resist method. On the other hand, in Comparative Example 5-8 in which the SOC-1 film pattern was transferred to the SiO₂ film as it is, the SOC-1 film has insufficient etching resistance and distortion of the pattern profile was confirmed as shown in Table 20. Meanwhile, in Comparative Examples 5-1 to 5-6 using the comparative UDL-1 to 6 that were confirmed to have insufficient performance in the filling property evaluation and the etching resistance evaluation, pattern collapse occurred during patterning, and it was not possible to obtain a good reverse pattern finally. Further, in Comparative Example 5-7 using the comparative UDL-7 that were confirmed to have insufficient performance in the etching resistance evaluation, distortion of the pattern profile occurred in reverse patterning, and it was not possible to obtain a good reverse pattern finally.

### [Storage Stability evaluation (Examples 6-1 to 6-29 and Comparative Examples 6-1 to 6-7)]

Each of the compositions (UDL-1 to 29 and Comparative UDL-1 to 7) for forming a metal-containing film was respectively applied onto a silicon substrate and heated by using a hot plate at 350°C for 60 seconds to form a metal-containing film having a film thickness of 80 nm. And then, after these compositions for forming a metal-containing film were stored at 23°C for 30 days, a metal-containing film was formed on the silicon substrate under the same conditions as above, and the film thickness was measured.

The results of the storage stability evaluation are shown in Tables 21 and 22.

**[Table 21]**

| Example | Composition for forming metal-containing film | Film thickness nm | Film thickness after 30 days nm |
|---|---|---|---|
| Example 6-1 | UDL-1 | 80 | 81 |
| Example 6-2 | UDL-2 | 80 | 81 |
| Example 6-3 | UDL-3 | 80 | 82 |
| Example 6-4 | UDL-4 | 80 | 81 |
| Example 6-5 | UDL-5 | 80 | 83 |
| Example 6-6 | UDL-6 | 80 | 83 |
| Example 6-7 | UDL-7 | 80 | 81 |
| Example 6-8 | UDL-8 | 80 | 82 |
| Example 6-9 | UDL-9 | 80 | 81 |
| Example 6-10 | UDL-10 | 80 | 81 |
| Example 6-11 | UDL-11 | 80 | 85 |
| Example 6-12 | UDL-12 | 80 | 82 |
| Example 6-13 | UDL-13 | 80 | 83 |
| Example 6-14 | UDL-14 | 80 | 81 |
| Example 6-15 | UDL-15 | 80 | 86 |
| Example 6-16 | UDL-16 | 80 | 85 |
| Example 6-17 | UDL-17 | 80 | 82 |
| Example 6-18 | UDL-18 | 80 | 83 |
| Example 6-19 | UDL-19 | 80 | 81 |
| Example 6-20 | UDL-20 | 80 | 82 |
| Example 6-21 | UDL-21 | 80 | 82 |
| Example 6-22 | UDL-22 | 80 | 81 |
| Example 6-23 | UDL-23 | 80 | 82 |
| Example 6-24 | UDL-24 | 80 | 83 |
| Example 6-25 | UDL-25 | 80 | 81 |
| Example 6-26 | UDL-26 | 80 | 81 |
| Example 6-27 | UDL-27 | 80 | 81 |
| Example 6-28 | UDL-28 | 80 | 82 |
| Example 6-29 | UDL-29 | 80 | 83 |

**[Table 22]**

| Comparative Example | Comparative composition for forming metal-containing film | Film thickness nm | Film thickness after 30 days nm |
|---|---|---|---|
| Comparative Example 6-1 | Comparative UDL-1 | 80 | 82 |
| Comparative Example 6-2 | Comparative UDL-2 | 80 | 81 |
| Comparative Example 6-3 | Comparative UDL-3 | 80 | 82 |
| Comparative Example 6-4 | Comparative UDL-4 | 80 | 86 |
| Comparative Example 6-5 | Comparative UDL-5 | 80 | 92 |
| Comparative Example 6-6 | Comparative UDL-6 | 80 | 82 |
| Comparative Example 6-7 | Comparative UDL-7 | 80 | 81 |

As shown in Table 21, even after the inventive compositions (UDL-1 to 29) for forming a metal-containing film were left at 23°C for 30 days, the thickness of the metal-containing film formed from these compositions for forming a metal-containing film was almost unchanged, showing excellent storage stability (Examples 6-1 to 6-29). In addition, in Examples 6-1 to 6-10, 6-12 to 6-14, and 6-17 to 6-19 containing a silicon-containing ligand, the change in thickness of the metal-containing film formed on the silicon substrate after leaving the composition for forming a metal-containing film at 23°C for 30 days was smaller than those in Examples 6-11, and 6-15 to 6-16 not containing a silicon-containing ligand, suggesting that the presence of the silicon-containing ligand contributes to improving the storage stability of the inventive composition for forming a metal-containing film. On the other hand, as shown in Table 22, in Comparative Example 6-5 using a ligand other than alcohol, a large increase in film thickness was confirmed after the composition was left at 23°C for 30 days.

From the above, the composition for forming a metal-containing film containing the inventive compound for forming a metal-containing film has both high filling and planarizing properties and high dry etching resistance, and therefore is extremely useful as a resist underlayer film material used for the multilayer resist method and a reverse material used for the tone-reversal etching process. Furthermore, it has become clear that the inventive patterning process using these can form a fine pattern with high precision even in a substrate in which a body to be processed has a step.

The present description includes the following embodiments.
[1]: A compound for forming a metal-containing film to be contained in a composition for forming a metal-containing film, comprising: at least one metal atom selected from the group consisting of Ti, Zr, and Hf; and a ligand coordinated to the metal atom, wherein the ligand contains a ligand represented by the following general formula (1),

   HO-X-R^{A1} (1)

   wherein R^{A1} represents a monovalent organic group having 2 to 30 carbon atoms; X represents a single bond or a linear or branched divalent organic group having 2 to 10 carbon atoms; and either R^{A1} and X contains one or more structures represented by the following general formulae (a-1) to (a-3), 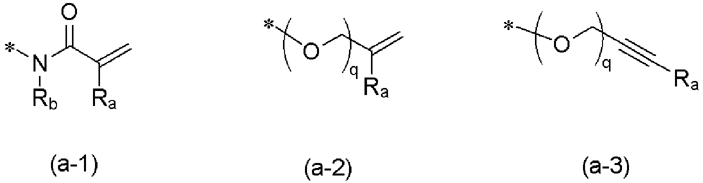
   wherein Rₐ represents a hydrogen atom or a monovalent organic group having 1 to 10 carbon atoms; R_{b} represents a hydrogen atom or a monovalent organic group having 1 to 10 carbon atoms; "q" represents 0 or 1; and "*" represents an attachment point.
[2] : The compound for forming a metal-containing film of the above [1], wherein R^{A1} in the general formula (1) includes any one or more of aromatic rings, alicyclic hydrocarbons, and heterocycles.
[3]: The compound for forming a metal-containing film of the above [1] or [2], wherein the ligand contains a ligand represented by the following general formula (2), 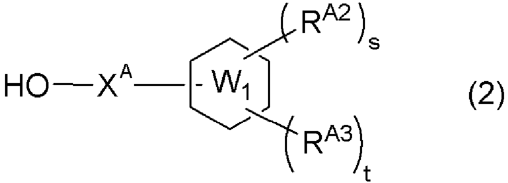 wherein W₁ is a divalent or trivalent organic group having 3 to 20 carbon atoms and including any one or more of aromatic rings, alicyclic hydrocarbons, and heterocycles; R^{A2} represents a hydrogen atom, a halogen atom, or a monovalent organic group having 1 to 10 carbon atoms; R^{A3} represents a monovalent organic group having 1 to 10 carbon atoms; X^{A} represents a single bond or a linear or branched divalent organic group having 1 to 10 carbon atoms; either of R^{A2} and X^{A} has one or more structures represented by the above general formulae (a-1) to (a-3); and "s" represents an integer of 1 to 6, "t" represents an integer from 0 to 5, and t+s is an integer from 1 to 6.
[4]: The compound for forming a metal-containing film of the above [3], wherein W₁ in the general formula (2) represents any one of the following structures. 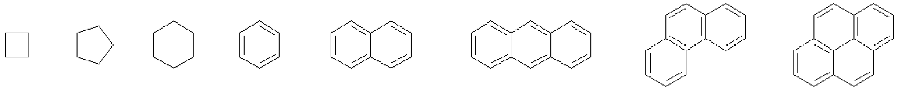 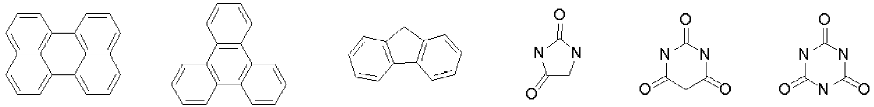
[5]: The compound for forming a metal-containing film of the above [3] or [4], wherein the compound for forming a metal-containing film is a reaction product of a reaction between: a metal compound represented by the following general formula (3) or a metal-containing compound containing any of a hydrolysate, condensate, or hydrolysis condensate of the metal compound represented by the following general formula (3); and a compound represented by the general formula (1) or (2),

   LₐMX^{B}_{b} (3)

   wherein, M represents any of Ti, Zr, and Hf; L represents a monodentate ligand having 0 to 30 carbon atoms or a polydentate ligand having 0 to 30 carbon atoms; X^{B} represents a hydrolyzable group selected from the group consisting of a halogen atom, an alkoxy group, a carboxylate group, an acyloxy group, and -NR^{a'}R^{b'}; R^{a'} and R^{b'} each independently represent a hydrogen atom or a monovalent organic group having 1 to 20 carbon atoms; and "a" and "b" represent integers from 0 to 4, satisfying a+b=4.
[6]: The compound for forming a metal-containing film of the above [5], wherein the general formula (3) has a structure represented by the following general formula (4),

   M(OR^{1A})₄ (4)

   wherein M is any of Ti, Zr, and Hf and R^{1A} represents a monovalent organic group having 1 to 20 carbon atoms.
[7] : The compound for forming a metal-containing film of any one of the above [1] to [6], further comprising a ligand represented by the following general formula (5), 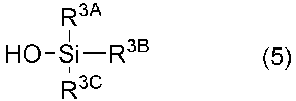
   wherein R^{3A}, R^{3B} and R^{3C} represent any organic group selected from the group consisting of: an organic group having 1 to 30 carbon atoms including a crosslinking group of a structure represented by any of the following general formulae (b-1) to (b-3); a substituted or unsubstituted alkyl group having 1 to 20 carbon atoms; and an aryl group having 6 to 20 carbon atoms, 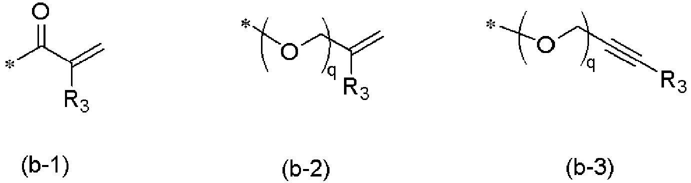
   wherein R₃ represents a hydrogen atom or a monovalent organic group having 1 to 10 carbon atoms; "q" represents 0 or 1; and "*" represents an attachment point.
[8] : A composition for forming a metal-containing film used for manufacturing a semiconductor, comprising: (A) the compound for forming a metal-containing film of any one of the above [1] to [7]; and (B) an organic solvent.
[9]: The composition for forming a metal-containing film of the above [8], further comprising one or more of (E) a crosslinking agent, (G) a surfactant, and (H) an acid generator.
[10]: The composition for forming a metal-containing film of the above [8] or [9], wherein the organic solvent (B) contains one or more organic solvents having a boiling point of 180°C or higher as (B1) a high-boiling-point solvent.
[11]: The composition for forming a metal-containing film of any one of the above [8] to [10], further comprising (BP) a flowability accelerator having any organic group represented by the following general formula (3') and an aromatic ring, 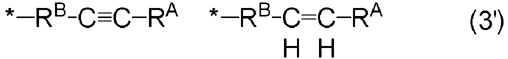 wherein "*" represents an attachment point to an oxygen atom; R^{B} represents a divalent organic group having 1 to 10 carbon atoms; and R^{A} represents a hydrogen atom or a monovalent organic group having 1 to 10 carbon atoms.
[12]: The composition for forming a metal-containing film of the above [11], wherein the flowability accelerator (BP) has at least one constituent unit represented by the following general formulae (BP-1), (BP-2), (BP-3), (BP-4), and (BP-5), 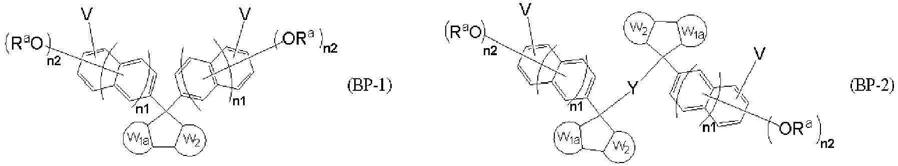
   wherein W₁ₐ and W₂ each independently represent a benzene ring or a naphthalene ring, and the hydrogen atoms in the benzene ring and naphthalene ring may be substituted with a hydrocarbon group having 1 to 6 carbon atoms; R^{a} each independently represents a group represented by the following formula (4'); Y represents a group represented by the following formula (5'); n1 each independently represents 0 or 1; n2 each independently represents 1 or 2; and V each independently represents a hydrogen atom or an attachment point, 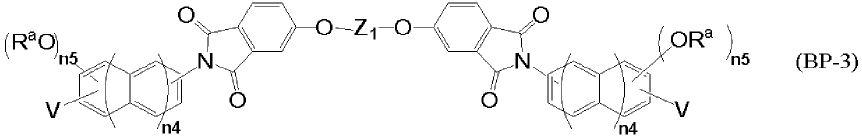
   wherein Z₁ represents a group represented by the following general formula (6); R^{a} each independently represents a group represented by the following formula (4'); n4 each independently represents 0 or 1; n5 each independently represents 1 or 2; and V each independently represents a hydrogen atom or an attachment point, 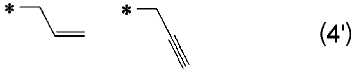
   wherein "*" represents an attachment point to an oxygen atom, 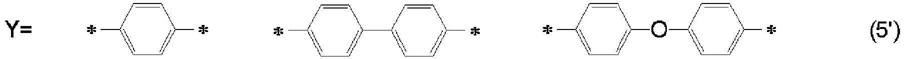
   wherein "*" represent an attachment point, 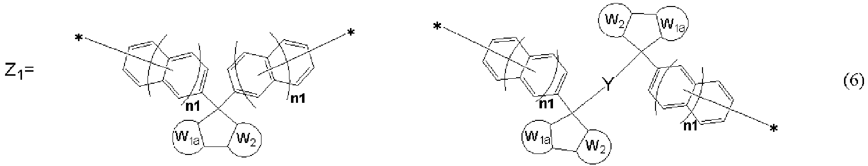
   wherein W₁ₐ, W₂, Y, and n1 represent as defined above, and "*" represent an attachment point, 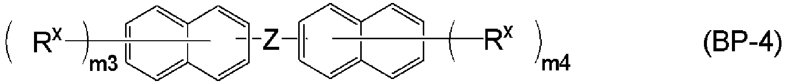
   wherein m3 and m4 each independently represent 1 or 2; Z represents a single bond or any of structures represented by the following general formula (7); and R^{x} represents any of structures represented by the following general formula (8), 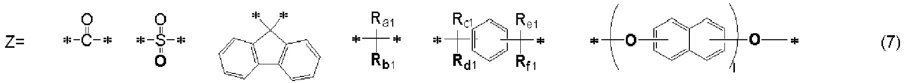
   wherein "*" represents an attachment point; "l" represents an integer of 0 to 3; Rₐ₁ to R_{f1} each independently represent a hydrogen atom, an alkyl group having 1 to 10 carbon atoms, a phenyl group, or a phenylethyl group, groups which may be substituted with fluorine; and Rₐ₁ and R_{b1} may be bonded each other to form a cyclic compound, 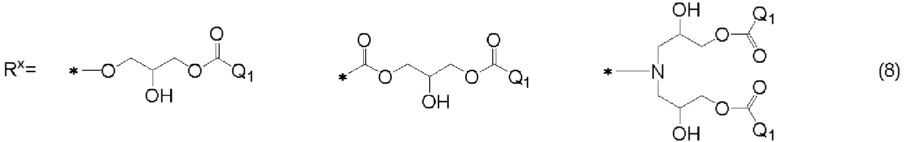
   wherein "*" represents an attachment point to an aromatic ring; Q₁ is a linear saturated hydrocarbon group having 1 to 30 carbon atoms or has a structure represented by the following general formula (9), 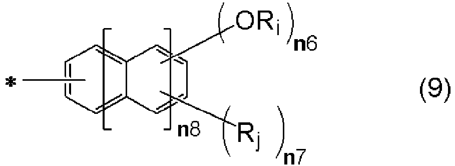
   wherein "*" represents an attachment point to a carbonyl group; Rᵢ represents a group represented by the formula (4'); Rⱼ represents a linear or branched hydrocarbon group having 1 to 10 carbon atoms, a halogen atom, a nitro group, an amino group, a nitrile group, an alkoxycarbonyl group having 2 to 10 carbon atoms, or an alkanoyloxy group having 1 to 10 carbon atoms; n6 and n7 each represent the number of substituents on the aromatic ring, and each represents an integer from 0 to 7, provided that n6+n7 is an integer from 0 to 7; and n8 represents an integer from 0 to 2, 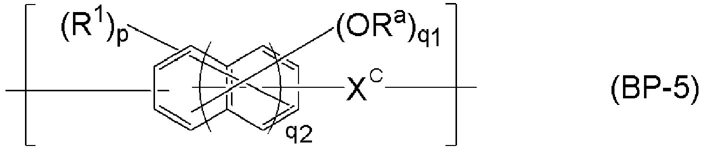
   wherein R¹ represents a saturated monovalent organic group having 1 to 30 carbon atoms or an unsaturated monovalent organic group having 2 to 30 carbon atoms; X^{C} represents a divalent organic group having 1 to 30 carbon atoms; R^{a} represents a group represented by the formula (4'); "p" represents an integer from 0 to 5, q1 represents an integer from 1 to 6, and p+q1 is an integer from 1 to 6; and q2 represents 0 or 1.
[13]: A patterning process for forming a pattern in a substrate to be processed, comprising the steps of:
   (I-1) applying the composition for forming a metal-containing film of any one of the above [8] to [12] onto a substrate to be processed, followed by heating to form a metal-containing film;
   (I-2) forming a resist upper layer film on the metal-containing film by using a photoresist material;
   (I-3) subjecting the resist upper layer film to pattern exposure and then development with a developer to form a pattern in the resist upper layer film;
   (I-4) transferring the pattern to the metal-containing film by dry etching while using the resist upper layer film having the formed pattern as a mask; and
   (I-5) processing the substrate to be processed while using the metal-containing film having the transferred pattern as a mask to form the pattern in the substrate to be processed.
[14]: A patterning process for forming a pattern in a substrate to be processed, comprising the steps of:
   (II-1) applying the composition for forming a metal-containing film of any one of the above [8] to [12] onto a substrate to be processed, followed by heating to form a metal-containing film;
   (II-2) forming a silicon-containing resist middle layer film on the metal-containing film;
   (II-3) forming a resist upper layer film on the silicon-containing resist middle layer film by using a photoresist material;
   (II-4) subjecting the resist upper layer film to pattern exposure and then development with a developer to form a pattern in the resist upper layer film;
   (II-5) transferring the pattern to the silicon-containing resist middle layer film by dry etching while using the resist upper layer film having the formed pattern as a mask;
   (II-6) transferring the pattern to the metal-containing film by dry etching while using the silicon-containing resist middle layer film having the transferred pattern as a mask; and
   (II-7) processing the substrate to be processed while using the metal-containing film having the transferred pattern as a mask to form the pattern in the substrate to be processed.
[15]: A patterning process for forming a pattern in a substrate to be processed, comprising the steps of:
   (III-1) applying the composition for forming a metal-containing film of any one of the above [8] to [12] onto a substrate to be processed, followed by heating to form a metal-containing film;
   (III-2) forming an inorganic hard mask middle layer film selected from the group consisting of a silicon oxide film, a silicon nitride film and a silicon oxynitride film on the metal-containing film;
   (III-3) forming an organic thin film on the inorganic hard mask middle layer film;
   (III-4) forming a resist upper layer film on the organic thin film by using a photoresist material;
   (III-5) subjecting the resist upper layer film to pattern exposure and then development with a developer to form a pattern in the resist upper layer film;
   (III-6) transferring the pattern to the organic thin film and the inorganic hard mask middle layer film by dry etching while using the resist upper layer film having the formed pattern as a mask;
   (III-7) transferring the pattern to the metal-containing film by dry etching while using the inorganic hard mask middle layer film having the transferred pattern as a mask; and
   (III-8) processing the substrate to be processed while using the metal-containing film having the transferred pattern as a mask to form the pattern in the substrate to be processed.
[16]: The patterning process for forming a pattern of the above [15], wherein the inorganic hard mask middle layer film is formed by a CVD method or an ALD method.
[17]: A patterning process for forming a pattern in a substrate to be processed, comprising the steps of:
   (IV-1) applying the composition for forming a metal-containing film of any one of the above [8] to [12] onto a substrate to be processed, followed by heating to form a metal-containing film;
   (IV-2) forming a resist underlayer film on the metal-containing film;
   (IV-3) forming a silicon-containing resist middle layer film or a combination of an inorganic hard mask middle layer film selected from the group consisting of a silicon oxide film, a silicon nitride film, and a silicon oxynitride film and an organic thin film on the resist underlayer film;
   (IV-4) forming a resist upper layer film on the silicon-containing resist middle layer film or the organic thin film by using a photoresist material;
   (IV-5) subjecting the resist upper layer film to pattern exposure and then development with a developer to form a pattern in the resist upper layer film;
   (IV-6) transferring the pattern to the silicon-containing resist middle layer film, or the organic thin film and the inorganic hard mask middle layer film by dry etching while using the resist upper layer film having the formed pattern as a mask;
   (IV-7) transferring the pattern to the resist underlayer film by dry etching while using the silicon-containing resist middle layer film having the transferred pattern or the inorganic hard mask middle layer film having the transferred pattern as a mask;
   (IV-8) transferring the pattern to the metal-containing film by dry etching while using the resist underlayer film having the transferred pattern as a mask; and
   (IV-9) processing the substrate to be processed while using the metal-containing film having the transferred pattern as a mask to form the pattern in the substrate to be processed.
[18]: A tone-reversal patterning process for forming a pattern in a substrate to be processed, comprising the steps of:
   (V-1) forming a resist underlayer film on a substrate to be processed;
   (V-2) forming a resist middle layer film or a combination of an inorganic hard mask middle layer film selected from the group consisting of a silicon oxide film, a silicon nitride film, and a silicon oxynitride film and an organic thin film on the resist underlayer film;
   (V-3) forming a resist upper layer film on the resist middle layer film or the combination of the inorganic hard mask middle layer film and the organic thin film by using a photoresist material;
   (V-4) subjecting the resist upper layer film to pattern exposure and then development with a developer to form a pattern in the resist upper layer film;
   (V-5) transferring the pattern to the resist middle layer film, or the organic thin film and the inorganic hard mask middle layer film by dry etching while using the resist upper layer film having the formed pattern as a mask;
   (V-6) transferring the pattern to the resist underlayer film by dry etching while using the resist middle layer film having the transferred pattern, or the inorganic hard mask middle layer film having the transferred pattern as a mask;
   (V-7) applying the composition for forming a metal-containing film of any one of the above [8] to [12] onto the resist underlayer film having the transferred pattern, followed by heating to cover the resist underlayer film with a metal-containing film, thereby filling a space between the resist underlayer film patterns with the metal-containing film;
   (V-8) etching back the metal-containing film covering the resist underlayer film having the transferred pattern by a chemical stripper or dry etching to expose an upper surface of the resist underlayer film having the formed pattern;
   (V-9) removing the resist middle layer film or the inorganic hard mask middle layer film remaining on the upper surface of the resist underlayer film by dry etching;
   (V-10) removing the resist underlayer film having the transformed pattern with its surface exposed by dry etching to form a reverse pattern of an original pattern in the metal-containing film; and
   (V-11) processing the substrate to be processed while using the metal-containing film having the formed reverse pattern as a mask to form the reverse pattern in the substrate to be processed.

It should be noted that the present invention is not limited to the above-described embodiments. The above embodiments are just examples, and any examples that have substantially the same feature and demonstrate the same functions and effects as those in the technical concept disclosed in claims of the present invention are included in the technical scope of the present invention.

## Claims

1. A compound for forming a metal-containing film to be contained in a composition for forming a metal-containing film, comprising: at least one metal atom selected from the group consisting of Ti, Zr, and Hf; and a ligand coordinated to the metal atom, wherein the ligand contains a ligand represented by the following general formula (1),
HO-X-R^{A1} (1)
wherein R^{A1} represents a monovalent organic group having 2 to 30 carbon atoms; X represents a single bond or a linear or branched divalent organic group having 2 to 10 carbon atoms; and either R^{A1} and X contains one or more structures represented by the following general formulae (a-1) to (a-3), 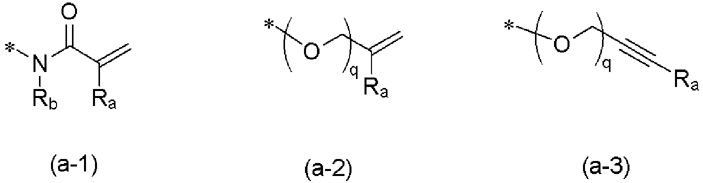
wherein Rₐ represents a hydrogen atom or a monovalent organic group having 1 to 10 carbon atoms; R_{b} represents a hydrogen atom or a monovalent organic group having 1 to 10 carbon atoms; "q" represents 0 or 1; and "*" represents an attachment point.

2. The compound for forming a metal-containing film according to claim 1, wherein R^{A1} in the general formula (1) includes any one or more of aromatic rings, alicyclic hydrocarbons, and heterocycles.

3. The compound for forming a metal-containing film according to claim 1 or 2, wherein the ligand contains a ligand represented by the following general formula (2), 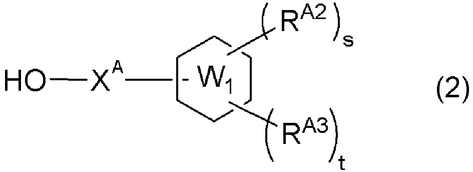 wherein W₁ is a divalent or trivalent organic group having 3 to 20 carbon atoms and including any one or more of aromatic rings, alicyclic hydrocarbons, and heterocycles; R^{A2} represents a hydrogen atom, a halogen atom, or a monovalent organic group having 1 to 10 carbon atoms; R^{A3} represents a monovalent organic group having 1 to 10 carbon atoms; X^{A} represents a single bond or a linear or branched divalent organic group having 1 to 10 carbon atoms; either of R^{A2} and X^{A} has one or more structures represented by the above general formulae (a-1) to (a-3); and "s" represents an integer of 1 to 6, "t" represents an integer from 0 to 5, and t+s is an integer from 1 to 6.

4. The compound for forming a metal-containing film according to claim 3, wherein W₁ in the general formula (2) represents any one of the following structures.

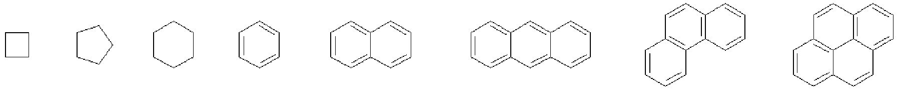

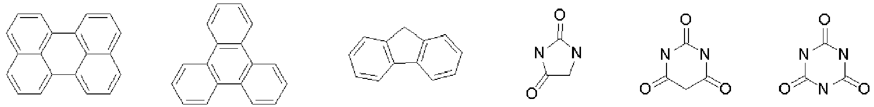

5. The compound for forming a metal-containing film according to claim 3 or 4, wherein the compound for forming a metal-containing film is a reaction product of a reaction between: a metal compound represented by the following general formula (3) or a metal-containing compound containing any of a hydrolysate, condensate, or hydrolysis condensate of the metal compound represented by the following general formula (3); and a compound represented by the general formula (1) or (2),
LₐMX^{B}_{b} (3)
wherein, M represents any of Ti, Zr, and Hf; L represents a monodentate ligand having 0 to 30 carbon atoms or a polydentate ligand having 0 to 30 carbon atoms; X^{B} represents a hydrolyzable group selected from the group consisting of a halogen atom, an alkoxy group, a carboxylate group, an acyloxy group, and -NR^{a'}R^{b'}; R^{a'} and R^{b'} each independently represent a hydrogen atom or a monovalent organic group having 1 to 20 carbon atoms; and "a" and "b" represent integers from 0 to 4, satisfying a+b=4.

6. The compound for forming a metal-containing film according to claim 5, wherein the general formula (3) has a structure represented by the following general formula (4),
M (OR^{1A}) ₄ (4)
wherein M is any of Ti, Zr, and Hf and R^{1A} represents a monovalent organic group having 1 to 20 carbon atoms.

7. The compound for forming a metal-containing film according to any one of claims 1 to 6, further comprising a ligand represented by the following general formula (5), 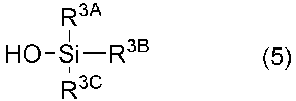
wherein R^{3A}, R^{3B} and R^{3C} represent any organic group selected from the group consisting of: an organic group having 1 to 30 carbon atoms including a crosslinking group of a structure represented by any of the following general formulae (b-1) to (b-3); a substituted or unsubstituted alkyl group having 1 to 20 carbon atoms; and an aryl group having 6 to 20 carbon atoms, 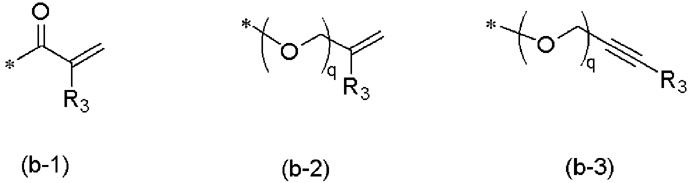
wherein R₃ represents a hydrogen atom or a monovalent organic group having 1 to 10 carbon atoms; "q" represents 0 or 1; and "*" represents an attachment point.

8. A composition for forming a metal-containing film used for manufacturing a semiconductor, comprising: (A) the compound for forming a metal-containing film according to any one of claims 1 to 7; and (B) an organic solvent.

9. The composition for forming a metal-containing film according to claim 8, further comprising one or more of (E) a crosslinking agent, (G) a surfactant, and (H) an acid generator.

10. The composition for forming a metal-containing film according to claim 8 or 9, wherein the organic solvent (B) contains one or more organic solvents having a boiling point of 180°C or higher as (B1) a high-boiling-point solvent.

11. The composition for forming a metal-containing film according to any one of claims 8 to 10, further comprising (BP) a flowability accelerator having any organic group represented by the following general formula (3') and an aromatic ring, 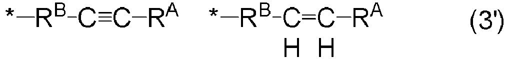 wherein "*" represents an attachment point to an oxygen atom; R^{B} represents a divalent organic group having 1 to 10 carbon atoms; and R^{A} represents a hydrogen atom or a monovalent organic group having 1 to 10 carbon atoms.

12. The composition for forming a metal-containing film according to claim 11, wherein the flowability accelerator (BP) has at least one constituent unit represented by the following general formulae (BP-1), (BP-2), (BP-3), (BP-4), and (BP-5), 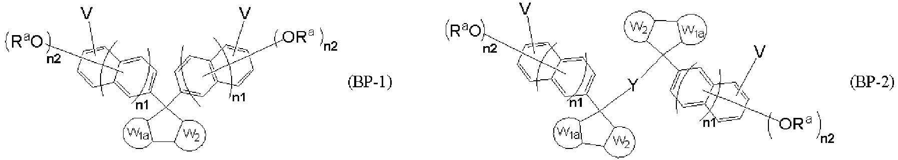
wherein W₁ₐ and W₂ each independently represent a benzene ring or a naphthalene ring, and the hydrogen atoms in the benzene ring and naphthalene ring may be substituted with a hydrocarbon group having 1 to 6 carbon atoms; R^{a} each independently represents a group represented by the following formula (4'); Y represents a group represented by the following formula (5'); n1 each independently represents 0 or 1; n2 each independently represents 1 or 2; and V each independently represents a hydrogen atom or an attachment point, 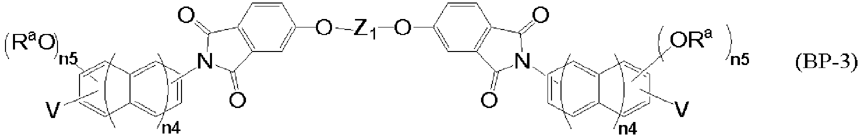
wherein Z₁ represents a group represented by the following general formula (6); R^{a} each independently represents a group represented by the following formula (4'); n4 each independently represents 0 or 1; n5 each independently represents 1 or 2; and V each independently represents a hydrogen atom or an attachment point, 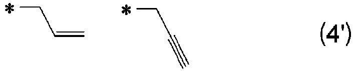
wherein "*" represents an attachment point to an oxygen atom, 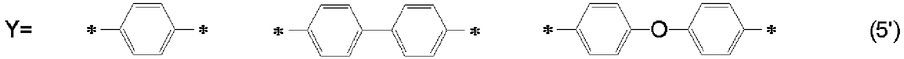
wherein "*" represent an attachment point, 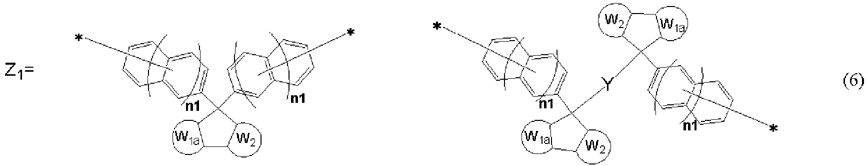
wherein W₁ₐ, W₂, Y, and n1 represent as defined above, and "*" represent an attachment point, 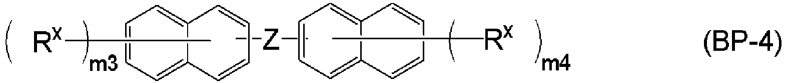
wherein m3 and m4 each independently represent 1 or 2; Z represents a single bond or any of structures represented by the following general formula (7); and R^{x} represents any of structures represented by the following general formula (8), 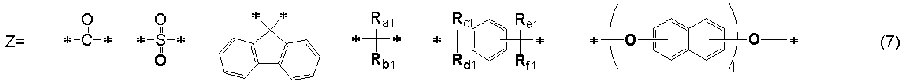
wherein "*" represents an attachment point; "l" represents an integer of 0 to 3; Rₐ₁ to R_{f1} each independently represent a hydrogen atom, an alkyl group having 1 to 10 carbon atoms, a phenyl group, or a phenylethyl group, groups which may be substituted with fluorine; and Rₐ₁ and R_{b1} may be bonded each other to form a cyclic compound, 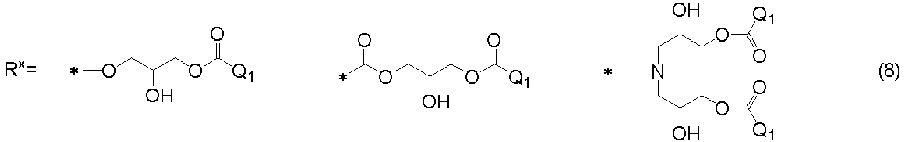
wherein "*" represents an attachment point to an aromatic ring; Q₁ is a linear saturated hydrocarbon group having 1 to 30 carbon atoms or has a structure represented by the following general formula (9), 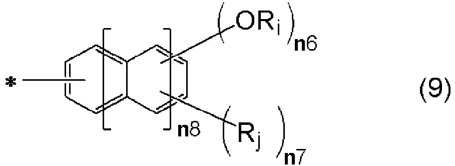
wherein "*" represents an attachment point to a carbonyl group; Rᵢ represents a group represented by the formula (4'); Rⱼ represents a linear or branched hydrocarbon group having 1 to 10 carbon atoms, a halogen atom, a nitro group, an amino group, a nitrile group, an alkoxycarbonyl group having 2 to 10 carbon atoms, or an alkanoyloxy group having 1 to 10 carbon atoms; n6 and n7 each represent the number of substituents on the aromatic ring, and each represents an integer from 0 to 7, provided that n6+n7 is an integer from 0 to 7; and n8 represents an integer from 0 to 2, 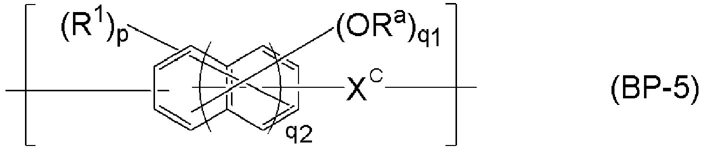
wherein R¹ represents a saturated monovalent organic group having 1 to 30 carbon atoms or an unsaturated monovalent organic group having 2 to 30 carbon atoms; X^{C} represents a divalent organic group having 1 to 30 carbon atoms; R^{a} represents a group represented by the formula (4'); "p" represents an integer from 0 to 5, q1 represents an integer from 1 to 6, and p+q1 is an integer from 1 to 6; and q2 represents 0 or 1.

13. A patterning process for forming a pattern in a substrate to be processed, comprising the steps of:
(a)
(I-1) applying the composition for forming a metal-containing film according to any one of claims 8 to 12 onto a substrate to be processed, followed by heating to form a metal-containing film;
(I-2) forming a resist upper layer film on the metal-containing film by using a photoresist material;
(I-3) subjecting the resist upper layer film to pattern exposure and then development with a developer to form a pattern in the resist upper layer film;
(I-4) transferring the pattern to the metal-containing film by dry etching while using the resist upper layer film having the formed pattern as a mask; and
(I-5) processing the substrate to be processed while using the metal-containing film having the transferred pattern as a mask to form the pattern in the substrate to be processed, or comprising the steps of:
(b)
(II-1) applying the composition for forming a metal-containing film according to any one of claims 8 to 12 onto a substrate to be processed, followed by heating to form a metal-containing film;
(II-2) forming a silicon-containing resist middle layer film on the metal-containing film;
(II-3) forming a resist upper layer film on the silicon-containing resist middle layer film by using a photoresist material;
(II-4) subjecting the resist upper layer film to pattern exposure and then development with a developer to form a pattern in the resist upper layer film;
(II-5) transferring the pattern to the silicon-containing resist middle layer film by dry etching while using the resist upper layer film having the formed pattern as a mask;
(II-6) transferring the pattern to the metal-containing film by dry etching while using the silicon-containing resist middle layer film having the transferred pattern as a mask; and
(II-7) processing the substrate to be processed while using the metal-containing film having the transferred pattern as a mask to form the pattern in the substrate to be processed, or comprising the steps of:
(c)
(III-1) applying the composition for forming a metal-containing film according to any one of claims 8 to 12 onto a substrate to be processed, followed by heating to form a metal-containing film;
(III-2) forming an inorganic hard mask middle layer film selected from the group consisting of a silicon oxide film, a silicon nitride film and a silicon oxynitride film on the metal-containing film;
(III-3) forming an organic thin film on the inorganic hard mask middle layer film;
(III-4) forming a resist upper layer film on the organic thin film by using a photoresist material;
(III-5) subjecting the resist upper layer film to pattern exposure and then development with a developer to form a pattern in the resist upper layer film;
(III-6) transferring the pattern to the organic thin film and the inorganic hard mask middle layer film by dry etching while using the resist upper layer film having the formed pattern as a mask;
(III-7) transferring the pattern to the metal-containing film by dry etching while using the inorganic hard mask middle layer film having the transferred pattern as a mask; and
(III-8) processing the substrate to be processed while using the metal-containing film having the transferred pattern as a mask to form the pattern in the substrate to be processed, or comprising the steps of:
(d)
(IV-1) applying the composition for forming a metal-containing film according to any one of claims 8 to 12 onto a substrate to be processed, followed by heating to form a metal-containing film;
(IV-2) forming a resist underlayer film on the metal-containing film;
(IV-3) forming a silicon-containing resist middle layer film or a combination of an inorganic hard mask middle layer film selected from the group consisting of a silicon oxide film, a silicon nitride film, and a silicon oxynitride film and an organic thin film on the resist underlayer film;
(IV-4) forming a resist upper layer film on the silicon-containing resist middle layer film or the organic thin film by using a photoresist material;
(IV-5) subjecting the resist upper layer film to pattern exposure and then development with a developer to form a pattern in the resist upper layer film;
(IV-6) transferring the pattern to the silicon-containing resist middle layer film, or the organic thin film and the inorganic hard mask middle layer film by dry etching while using the resist upper layer film having the formed pattern as a mask;
(IV-7) transferring the pattern to the resist underlayer film by dry etching while using the silicon-containing resist middle layer film having the transferred pattern or the inorganic hard mask middle layer film having the transferred pattern as a mask;
(IV-8) transferring the pattern to the metal-containing film by dry etching while using the resist underlayer film having the transferred pattern as a mask; and
(IV-9) processing the substrate to be processed while using the metal-containing film having the transferred pattern as a mask to form the pattern in the substrate to be processed.

14. The patterning process for forming a pattern according to claim 13, wherein, in step (III-2), the inorganic hard mask middle layer film is formed by a CVD method or an ALD method.

15. A tone-reversal patterning process for forming a pattern in a substrate to be processed, comprising the steps of:
(V-1) forming a resist underlayer film on a substrate to be processed;
(V-2) forming a resist middle layer film or a combination of an inorganic hard mask middle layer film selected from the group consisting of a silicon oxide film, a silicon nitride film, and a silicon oxynitride film and an organic thin film on the resist underlayer film;
(V-3) forming a resist upper layer film on the resist middle layer film or the combination of the inorganic hard mask middle layer film and the organic thin film by using a photoresist material;
(V-4) subjecting the resist upper layer film to pattern exposure and then development with a developer to form a pattern in the resist upper layer film;
(V-5) transferring the pattern to the resist middle layer film, or the organic thin film and the inorganic hard mask middle layer film by dry etching while using the resist upper layer film having the formed pattern as a mask;
(V-6) transferring the pattern to the resist underlayer film by dry etching while using the resist middle layer film having the transferred pattern, or the inorganic hard mask middle layer film having the transferred pattern as a mask;
(V-7) applying the composition for forming a metal-containing film according to any one of claims 8 to 12 onto the resist underlayer film having the transferred pattern, followed by heating to cover the resist underlayer film with a metal-containing film, thereby filling a space between the resist underlayer film patterns with the metal-containing film;
(V-8) etching back the metal-containing film covering the resist underlayer film having the transferred pattern by a chemical stripper or dry etching to expose an upper surface of the resist underlayer film having the formed pattern;
(V-9) removing the resist middle layer film or the inorganic hard mask middle layer film remaining on the upper surface of the resist underlayer film by dry etching;
(V-10) removing the resist underlayer film having the transformed pattern with its surface exposed by dry etching to form a reverse pattern of an original pattern in the metal-containing film; and
(V-11) processing the substrate to be processed while using the metal-containing film having the formed reverse pattern as a mask to form the reverse pattern in the substrate to be processed.
